# EUROPÄISCHE PATENTSCHRIFT

(11) **EP 3 411 455 B1**
(45) Veröffentlichungstag und Bekanntmachung des Hinweises auf die Patenterteilung: **21.10.2020**
(21) Anmeldenummer: 17700472.8
(22) Anmeldetag: 17.01.2017
(51) Int. Cl.: C09K 11/06, C07D 209/96, H01L 51/00, C07D 333/76, C07D 403/04, C07D 251/24, C07D 209/82, C07D 213/16, C07D 307/91, H01L 51/50, H05B 33/10, H05B 33/14

(54) **MATERIALIEN FÜR ELEKTRONISCHE VORRICHTUNGEN**
MATERIALS FOR ELECTRONIC DEVICES
MATÉRIAUX POUR DISPOSITIFS ÉLECTRONIQUES

(30) Priorität: 05.02.2016 EP 16154514
(43) Veröffentlichungstag der Anmeldung: 12.12.2018
(73) Patentinhaber: Merck Patent GmbH, 64293 Darmstadt (DE)
(72) Erfinder: VOGES, Frank, 67098 Bad Duerkheim (DE); MUJICA-FERNAUD, Teresa, 64283 Darmstadt (DE); MONTENEGRO, Elvira, 69469 Weinheim (DE); ANÉMIAN, Rémi Manouk, Seoul 657-169 (KR)
(86) Internationale Anmeldenummer: PCT/EP2017/000042
(87) Internationale Veröffentlichungsnummer: WO 2017/133829

(56) Entgegenhaltungen:
- EP-A1- 2 978 040
- WO-A1-2014/129846
- WO-A1-2014/171779
- WO-A1-2015/012618

## Beschreibung

Die vorliegenden Anmeldung betrifft ein Spirobifluoren-Derivat einer im Folgenden definierten Formel (I), welches sich zur Verwendung in elektronischen Vorrichtungen, insbesondere organischen Elektrolumineszenzvorrichtungen (OLEDs) eignet.

Unter elektronischen Vorrichtungen im Sinne dieser Anmeldung werden sogenannte organische elektronische Vorrichtungen verstanden (organic electronic devices), welche organische Halbleitermaterialien als Funktionsmaterialien enthalten. Insbesondere werden darunter OLEDs verstanden.

Der Aufbau von OLEDs, in denen organische Verbindungen als funktionelle Materialien eingesetzt werden, ist im Stand der Technik allgemein bekannt. Allgemein werden unter der Bezeichnung OLEDs elektronische Vorrichtungen verstanden, welche eine oder mehrere Schichten enthaltend organische Verbindungen aufweisen und unter Anlegen von elektrischer Spannung Licht emittieren.

Bei elektronischen Vorrichtungen, insbesondere OLEDs, besteht großes Interesse an der Verbesserung der Leistungsdaten, insbesondere Lebensdauer, Effizienz und Betriebsspannung. In diesen Punkten konnte noch keine vollständig zufriedenstellende Lösung gefunden werden.

Einen großen Einfluss auf die Leistungsdaten von elektronischen Vorrichtungen haben Schichten mit lochtransportierender Funktion, wie beispielsweise lochinjizierende Schichten, Lochtransportschichten, Elektronenblockierschichten und auch emittierende Schichten. Zur Verwendung in diesen Schichten werden kontinuierlich neue Materialien mit lochtransportierenden Eigenschaften gesucht.

Es ist im Stand der Technik bekannt, Spirobifluoren-Derivate zu verwenden, welche in einer Position gewählt aus Positionen 3 und 4 am Spirobifluoren mit einer Aminogruppe substituiert sind. Wahlweise kann diese Aminogruppe über eine Spacergruppe mit der Spirobifluoren-Gruppe verbunden sein.

In EP 2 978 040 A1 sind Spirofuorene- Verbindungen offenbart, die am Spirofluoren mit mehreren Diarylamino-Aryl-gruppen substituiert sind.

Im Rahmen von Untersuchungen zu neuartigen Materialien zur Verwendung in OLEDs wurde nun überraschend gefunden, dass sich Verbindungen, die sich von den oben genannten Verbindungen dadurch unterscheiden, dass sie speziell eine meta- oder ortho-verknüpfte Phenylengruppe zwischen dem Spirobifluoren und dem Stickstoffatom der Aminogruppe aufweisen, hervorragend zur Verwendung in OLEDs eignen, insbesondere als Materialien mit lochtransportierender Funktion.

Insbesondere sind sie den oben genannten Verbindungen bezüglich ihrer Leistungsdaten bei der Verwendung in OLEDs überlegen, ganz besonders bei der Lebensdauer, der Betriebsspannung und der Quanteneffizienz der OLEDs. Die gefundenen neuen Verbindungen weisen weiterhin eine oder mehrere Eigenschaften gewählt aus sehr guten lochleitenden Eigenschaften, sehr guten elektronenblockierenden Eigenschaften, hoher Glasübergangstemperatur, hoher Oxidationsstabilität, guter Löslichkeit und hoher Temperaturstabilität auf.

Gegenstand der vorliegenden Anmeldung ist damit eine Verbindung gemäß Formel (I) 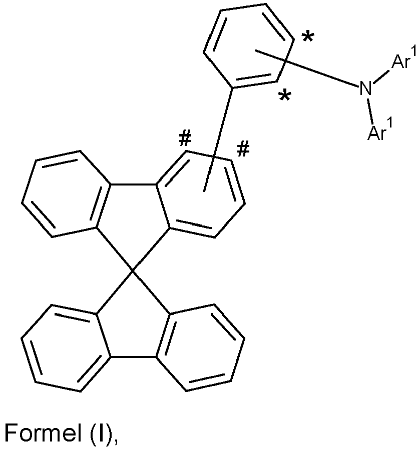 wobei die Verbindung an jeder der freien Positionen an der Spirobifluoren-Einheit jeweils mit einem Rest R¹ substituiert sein kann, und an jeder der freien Positionen an der Phenylen-Einheit jeweils mit einem Rest R² substituiert sein kann, und wobei für die auftretenden Variablen gilt:
- R¹: ist bei jedem Auftreten gleich oder verschieden gewählt aus H, D, F, C(=O)R⁴, CN, Si(R⁴)₃, N(R⁴)₂, P(=O)(R⁴)₂, OR⁴, S(=O)R⁴, S(=O)₂R⁴, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, wobei der Begriff "aromatisches Rinsystem" nicht Triarylamin umfasst, und heteroaromatischen Ringsystemen mit 5 bis 40 aromatischen Ring-atomen; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁴ substituiert sein können; und wobei eine oder mehrere CH₂-Gruppen in den genannten Alkyl-, Alkoxy-, Alkenyl- und Alkinylgruppen durch -R⁴C=CR⁴-, -C=C-, Si(R⁴)², C=O, C=NR⁴, -C(=O)O-, -C(=O)NR⁴-, NR⁴, P(=O)(R⁴), -O-, -S-, SO oder SO₂ ersetzt sein können;
- R²: ist bei jedem Auftreten gleich oder verschieden gewählt aus H, D, F, C(=O)R⁴, CN, Si(R⁴)3, P(=O)(R⁴)₂, OR⁴, S(=O)R⁴, S(=O)₂R⁴, gerad-kettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und hetero-aromatischen Ringsystemen mit 5 bis 40 aromatischen Ringatomen; wobei zwei oder mehr Reste R² miteinander verknüpft sein können und einen Ring bilden können; wobei die genannten Alkyl-, Alkoxy-, Alkenyl-und Alkinylgruppen und die genannten aromatischen Ringsysteme und heteroaromatischen Ringsysteme jeweils mit einem oder mehreren Resten R⁴ substituiert sein können; und wobei eine oder mehrere CH₂-Gruppen in den genannten Alkyl-, Alkoxy-, Alkenyl- und Alkinylgruppen durch -R⁴C=CR⁴-, -C=C-, Si(R⁴)², C=O, C=NR⁴, -C(=O)O-, -C(=O)NR⁴-, NR⁴, P(=O)(R⁴), -O-, -S-, SO oder SO₂ ersetzt sein können;
- Ar¹: ist bei jedem Auftreten gleich oder verschieden gewählt aus aromatischen Ringsystemen mit 6 bis 20 aromatischen Ringatomen, die optional mit einem oder mehreren Resten R³ substituiert sind, und aus heteroaromatischen Ringsystemen mit 5 bis 30 aromatischen Ringatomen, die optional mit einem oder mehreren Resten R³ substituiert sind;
- R³: ist bei jedem Auftreten gleich oder verschieden gewählt aus H, D, F, C(=O)R⁴, CN, Si(R⁴)3, N(R⁴)₂, P(=O)(R⁴)₂, OR⁴, S(=O)R⁴, S(=O)₂R⁴, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ring-atomen; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁴ substituiert sein können; und wobei eine oder mehrere CH₂-Gruppen in den genannten Alkyl-, Alkoxy-, Alkenyl- und Alkinylgruppen durch -R⁴C=CR⁴-, -C=C-, Si(R⁴)², C=O, C=NR⁴, -C(=O)O-, -C(=O)NR⁴-, NR⁴, P(=O)(R⁴), -O-, -S-, SO oder SO₂ ersetzt sein können;
- R⁴: ist bei jedem Auftreten gleich oder verschieden gewählt aus H, D, F, C(=O)R⁵, CN, Si(R⁵)₃, P(=O)(R⁵)₂, OR⁵, S(=O)R⁵, S(=O)₂R⁵, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ring-atomen; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁵ substituiert sein können; und wobei eine oder mehrere CH₂-Gruppen in den genannten Alkyl-, Alkoxy-, Alkenyl- und Alkinylgruppen durch -R⁵C=CR⁵-, -C=C-, Si(R⁵)₂, C=O, C=NR⁵, -C(=O)O-, -C(=O)NR⁵-, NR⁵, P(=O)(R⁵), -O-, -S-, SO oder SO₂ ersetzt sein können;
- R⁵: ist bei jedem Auftreten gleich oder verschieden gewählt aus H, D, F, CN, Alkyl- oder Alkoxygruppen mit 1 bis 20 C-Atomen, Alkenyl- oder Alkinylgruppen mit 2 bis 20 C-Atomen, aromatischen Ringsystemen mit 6 bis 40 aromatischen Ringatomen und heteroaromatischen Ringsystemen mit 5 bis 40 aromatischen Ringatomen; und wobei die genannten Alkyl-, Alkoxy-, Alkenyl- und Alkinylgruppen, aromatischen Ringsysteme und heteroaromatischen Ringsysteme mit F oder CN substituiert sein können;
wobei der Benzolring an einer der mit dem Zeichen # markierten Positionen an die Spirobifluorengruppe gebunden ist; und
wobei die Gruppe N(Ar¹)₂ an einer der mit dem Zeichen * markierten Positionen an den Benzolring gebunden ist.

Es gelten im Rahmen der vorliegenden Anmeldung die folgenden Definitionen:
Eine Arylgruppe im Sinne dieser Erfindung enthält 6 bis 40 aromatische Ringatome, von denen keines ein Heteroatom darstellt. Unter einer Arylgruppe im Sinne dieser Erfindung wird entweder ein einfacher aromatischer Cyclus, also Benzol, oder ein kondensierter aromatischer Polycyclus, beispielsweise Naphthalin, Phenanthren oder Anthracen, verstanden. Ein kondensierter aromatischer Polycyclus besteht im Sinne der vorliegenden Anmeldung aus zwei oder mehr miteinander kondensierten einfachen aromatischen Cyclen. Unter Kondensation zwischen Cyclen ist dabei zu verstehen, dass die Cyclen mindestens eine Kante miteinander teilen.

Eine Heteroarylgruppe im Sinne dieser Erfindung enthält 5 bis 40 aromatische Ringatome, von denen mindestens eines ein Heteroatom darstellt. Die Heteroatome der Heteroarylgruppe sind bevorzugt ausgewählt aus N, O und S. Unter einer Heteroarylgruppe im Sinne dieser Erfindung wird entweder ein einfacher heteroaromatischer Cyclus, beispielsweise Pyridin, Pyrimidin oder Thiophen, oder ein kondensierter heteroaromatischer Polycyclus, beispielsweise Chinolin oder Carbazol, verstanden. Ein kondensierter heteroaromatischer Polycyclus besteht im Sinne der vorliegenden Anmeldung aus zwei oder mehr miteinander kondensierten einfachen heteroaromatischen Thiophen, oder ein kondensierter heteroaromatischer Polycyclus, beispielsweise Chinolin oder Carbazol, verstanden. Ein kondensierter heteroaromatischer Polycyclus besteht im Sinne der vorliegenden Anmeldung aus zwei oder mehr miteinander kondensierten einfachen heteroaromatischen Cyclen. Unter Kondensation zwischen Cyclen ist dabei zu verstehen, dass die Cyclen mindestens eine Kante miteinander teilen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Triphenylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ein aromatisches Ringsystem im Sinne dieser Erfindung enthält 6 bis 40 C-Atome im Ringsystem und umfasst keine Heteroatome als aromatische Ringatome. Ein aromatisches Ringsystem im Sinne dieser Erfindung enthält daher keine Heteroarylgruppen. Unter einem aromatischen Ringsystem im Sinne dieser Erfindung soll ein System verstanden werden, das nicht notwendigerweise nur Arylgruppen enthält, sondern in dem auch mehrere Arylgruppen durch eine Einfachbindung oder durch eine nicht-aromatische Einheit, wie beispielsweise ein oder mehrere wahlweise substituierte C-, Si-, N-, O- oder S-Atome, verbunden sein können. Dabei umfasst die nicht-aromatische Einheit bevorzugt weniger als 10 % der von H verschiedenen Atome, bezogen auf die Gesamtzahl der von H verschiedenen Atome des Systems. So sollen beispielsweise auch Systeme wie 9,9'-Spirobifluoren, 9,9'-Diarylfluoren, Triarylamin, Diarylether und Stilben als aromatische Ringsysteme im Sinne dieser Erfindung verstanden werden, und ebenso Systeme, in denen zwei oder mehr Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Arylgruppen über Einfachbindungen miteinander verknüpft sind, als aromatische Ringsysteme im Sinne dieser Erfindung verstanden, wie beispielsweise Systeme wie Biphenyl und Terphenyl.

Ein heteroaromatisches Ringsystem im Sinne dieser Erfindung enthält 5 bis 40 aromatische Ringatome, von denen mindestens eines ein Heteroatom darstellt. Die Heteroatome des heteroaromatischen Ringsystems sind bevorzugt ausgewählt aus N, O und/oder S. Ein heteroaromatisches Ringsystem entspricht der oben genannten Definition eines aromatischen Ringsystems, weist jedoch mindestens ein Heteroatom als eines der aromatischen Ringatome auf. Es unterscheidet sich dadurch von einem aromatischen Ringsystem im Sinne der Definition der vorliegenden Anmeldung, welches gemäß dieser Definition kein Heteroatom als aromatisches Ringatom enthalten kann.

Unter einem aromatischen Ringsystem mit 6 bis 40 aromatischen Ringatomen oder einem heteroaromatischen Ringsystem mit 5 bis 40 aromatischen Ringatomen, werden insbesondere Gruppen verstanden, die abgeleitet sind von den oben unter Arylgruppen und Heteroarylgruppen genannten Gruppen sowie von Biphenyl, Terphenyl, Quaterphenyl, Fluoren, Spirobifluoren, Dihydrophenanthren, Dihydropyren, Tetrahydropyren, Indenofluoren, Truxen, Isotruxen, Spirotruxen, Spiroisotruxen, Indenocarbazol, oder von Kombinationen dieser Gruppen.

Im Rahmen der vorliegenden Erfindung werden unter einer geradkettigen Alkylgruppe mit 1 bis 20 C-Atomen bzw. einer verzweigten oder cyclischen Alkylgruppe mit 3 bis 20 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neo-Pentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden.

Unter einer Alkoxy- oder Thioalkylgruppe mit 1 bis 20 C-Atomen, in der auch einzelne H-Atome oder CH₂-Gruppen durch die oben bei der Definition der Reste genannten Gruppen substituiert sein können, werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet.

Bevorzugte Ausführungsformen von Verbindungen der Formel (I) stellen Verbindungen gemäß einer der Formeln (I-1) bis (I-4) dar 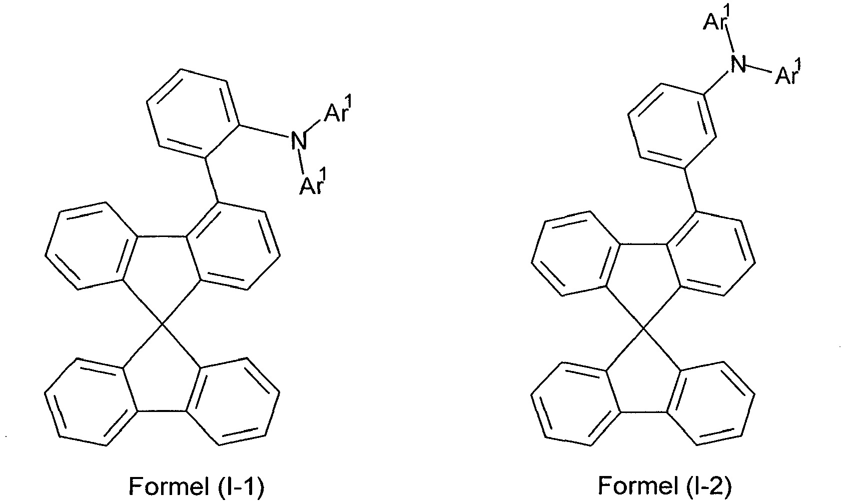 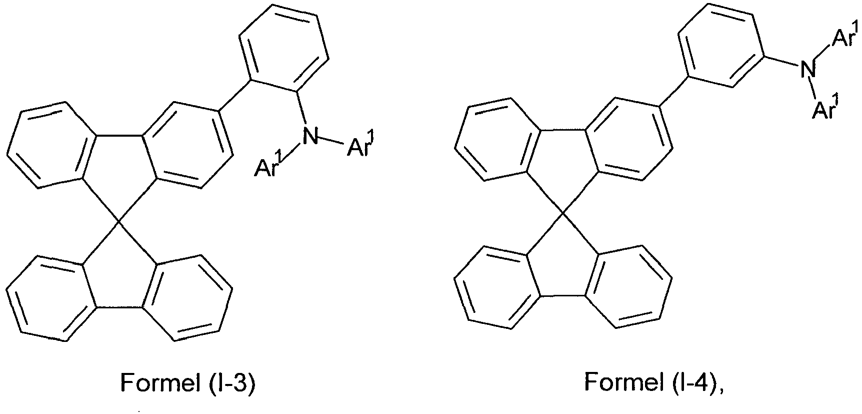 wobei die Formel an jeder der freien Positionen an der Spirobifluoren-Einheit jeweils mit einem Rest R¹ substituiert sein kann, und an jeder der freien Positionen an der Phenylen-Einheit jeweils mit einem Rest R² substituiert sein kann, und
wobei die auftretenden Gruppen wie oben definiert sind.

Unter den Verbindungen der Formel (I-1) bis (I-4) sind die Verbindungen der Formel (I-1) und (I-2) besonders bevorzugt.

Bevorzugt ist R¹ bei jedem Auftreten gleich oder verschieden gewählt aus H, D, F, CN, Si(R⁴)3, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, wobei der Begriff "aromatisches Ringsystem" nicht Triarylamin umfasst, und heteroaromatischen Ringsystemen mit 5 bis 40 aromatischen Ringatomen; wobei die genannten Alkylgruppen und die genannten aromatischen Ringsysteme und heteroaromatischen Ringsysteme jeweils mit einem oder mehreren Resten R⁴ substituiert sein können. Besonders bevorzugt ist R¹ bei jedem Auftreten gleich oder verschieden gewählt aus H, F, Methyl, Ethyl, Propyl, Butyl, tert-Butyl, Phenyl, Biphenyl, Terphenyl, Naphthyl, Carbazolyl, Benzofuranyl, Benzothiophenyl, Dibenzofuranyl, Dibenzothiophenyl, 9,9'-Dimethylfluorenyl und 9,9'-Diphenylfluorenyl. Ganz besonders bevorzugt ist R¹ gleich H.

Bevorzugt ist am Spirobifluoren-Grundgerüst kein Rest R¹ gebunden, genau ein Rest R¹ gebunden, der ungleich H ist, oder es sind genau zwei Reste R¹ gebunden, die ungleich H sind. Besonders bevorzugt ist am Spirobifluorenyl-Grundgerüst kein Rest R¹ gebunden.

Bevorzugt ist R² bei jedem Auftreten gleich oder verschieden gewählt aus H, D, F, CN, Si(R⁴)3, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen und die genannten aromatischen Ringsysteme und heteroaromatischen Ringsysteme jeweils mit einem oder mehreren Resten R⁴ substituiert sein können. Besonders bevorzugt ist R² bei jedem Auftreten gleich oder verschieden gewählt aus H, F, Methyl, Ethyl, Propyl, Butyl, tert-Butyl, Phenyl, Biphenyl, Terphenyl, Naphthyl, Carbazolyl, Benzofuranyl, Benzothiophenyl, Dibenzofuranyl, Dibenzothiophenyl, 9,9'-Dimethylfluorenyl und 9,9'-Diphenylfluorenyl. Ganz besonders bevorzugt ist R² gleich H.

Bevorzugt ist an die Phenylengruppe in Formel (I) kein Rest R² oder genau ein Rest R² gebunden, der ungleich H ist.

Bevorzugte Ausführungsformen der Formeln (I-1) bis (I-4) entsprechen daher den Formeln (I-1) bis (I-4), in denen jeweils keine Reste R¹ und R² an die entsprechenden freien Positionen gebunden sind, oder sie entsprechen den im Folgenden gezeigten substituierten Varianten 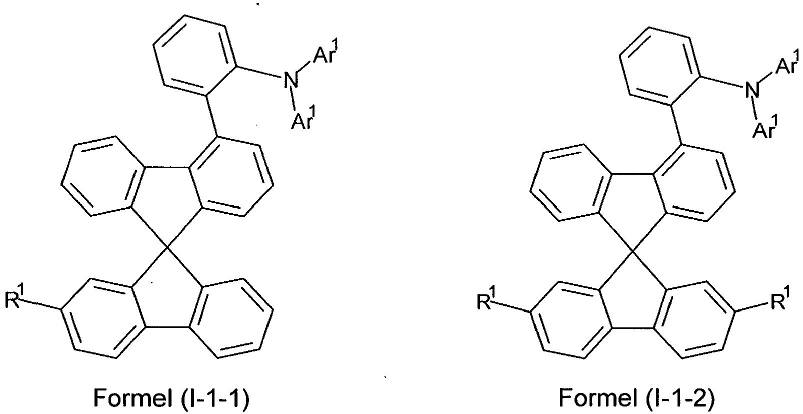 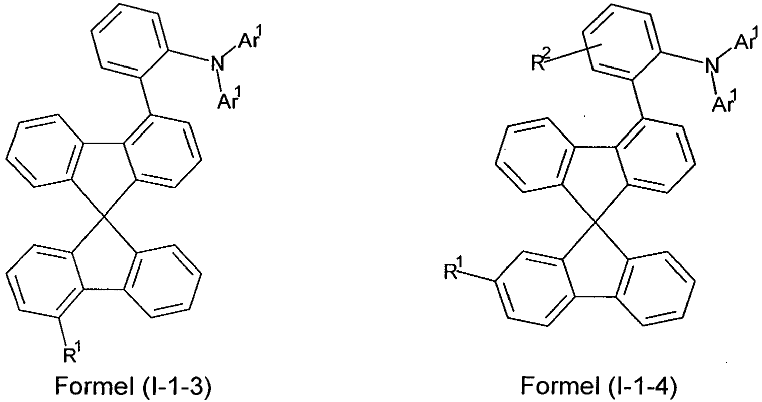 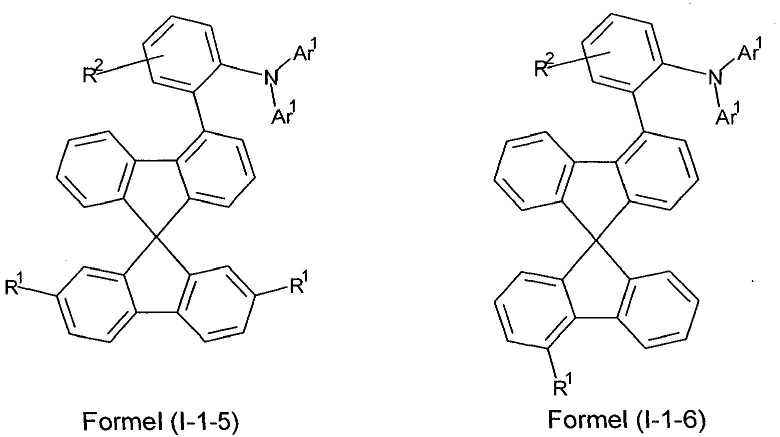 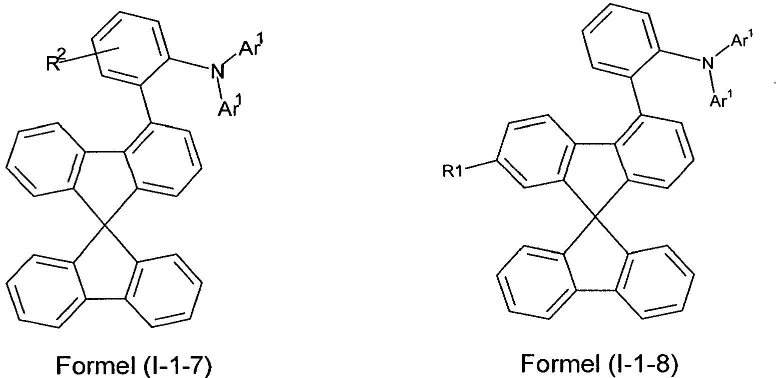 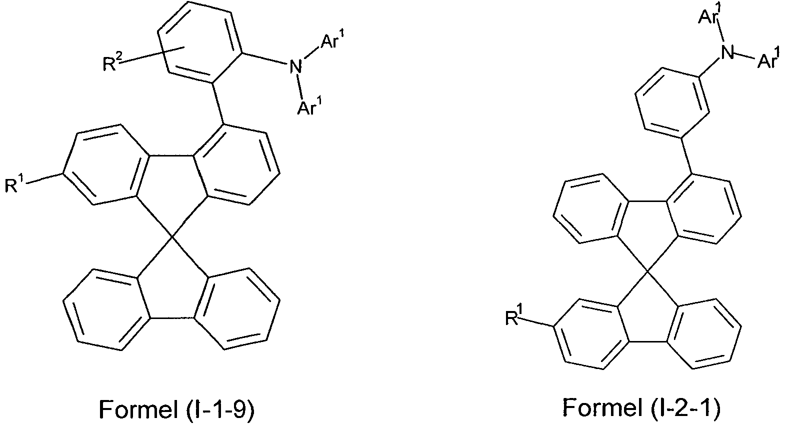 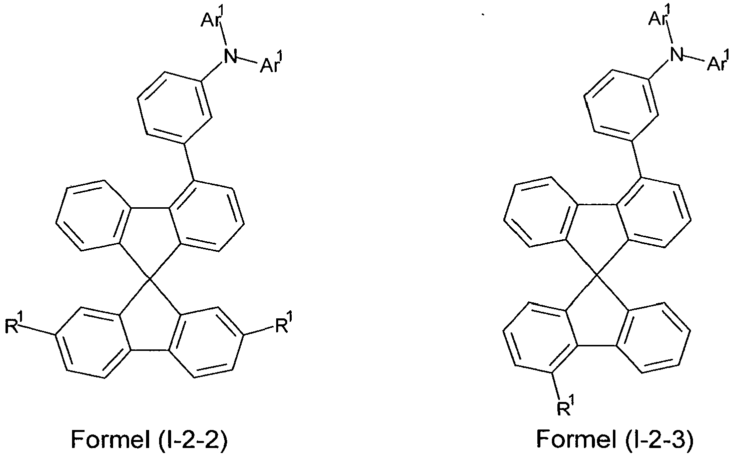 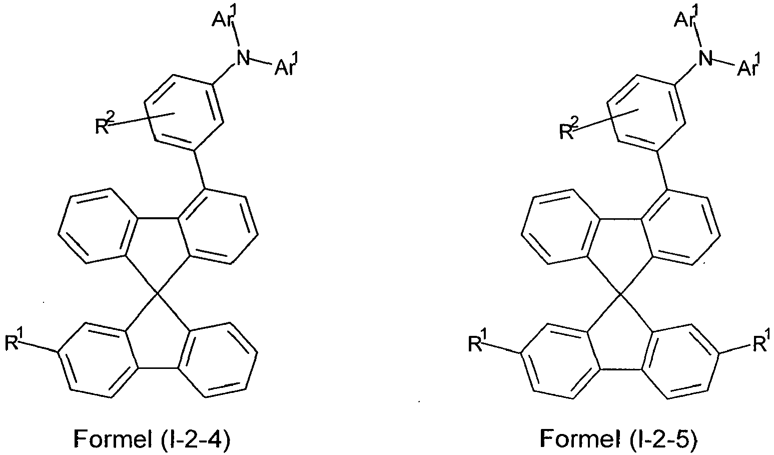 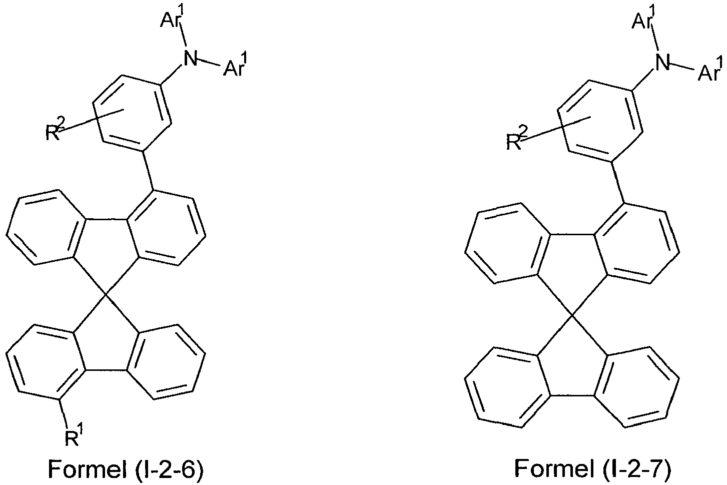 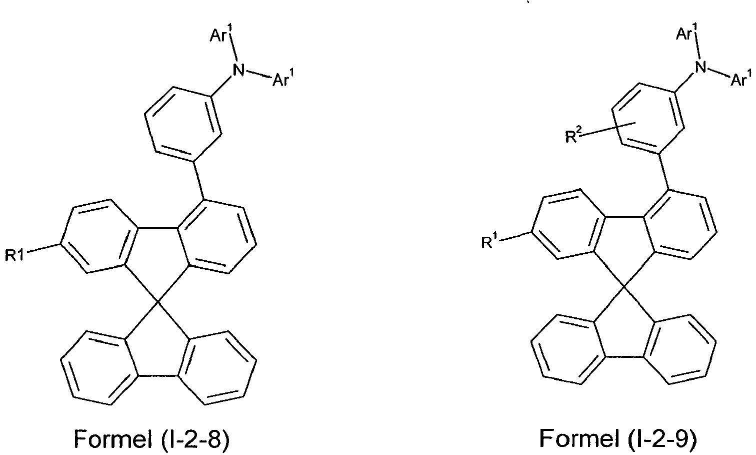 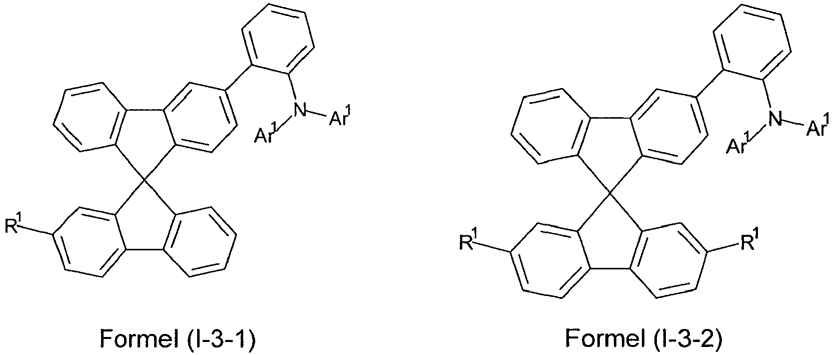 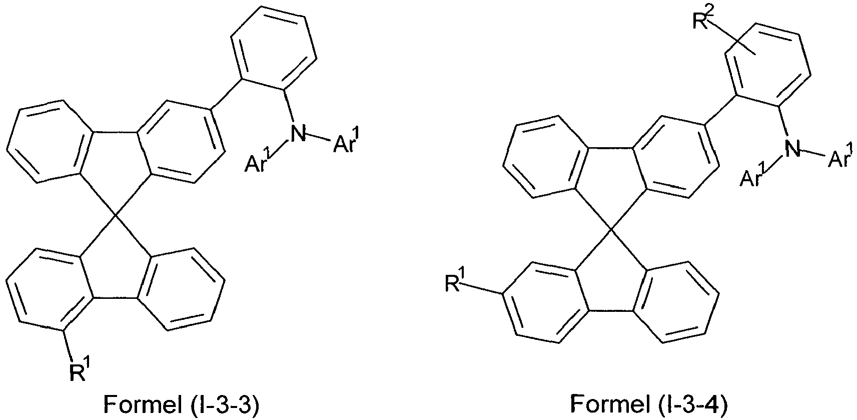 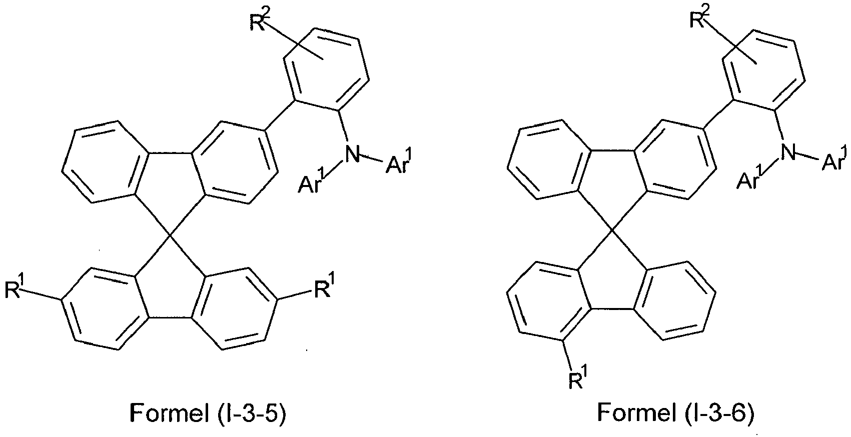 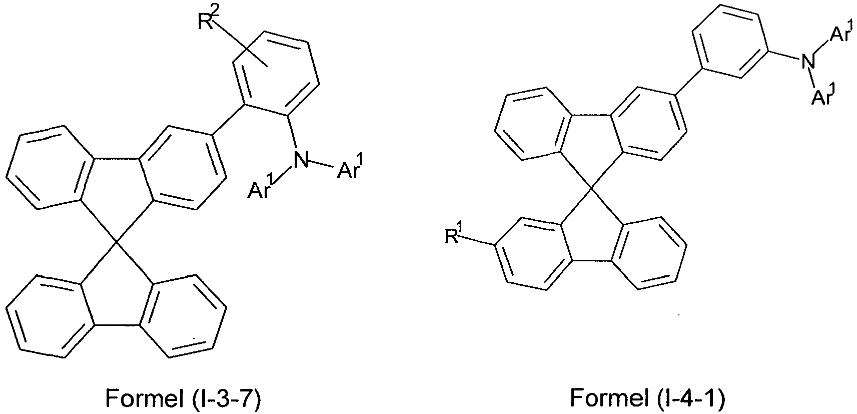 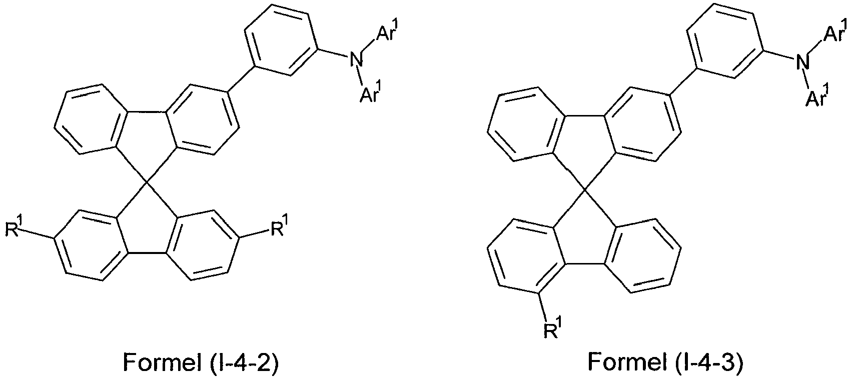 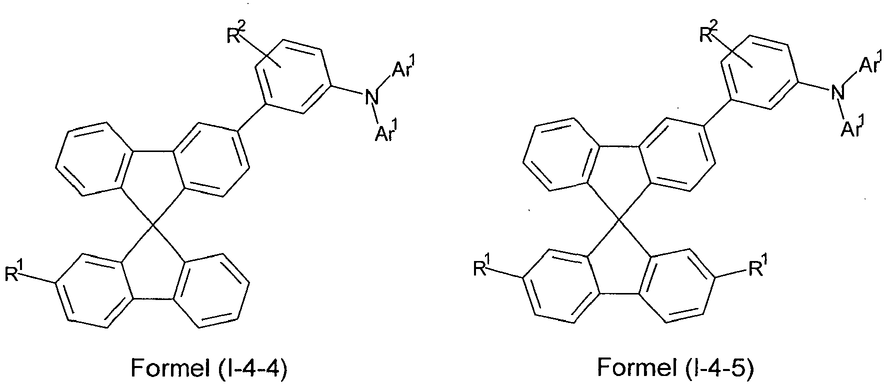 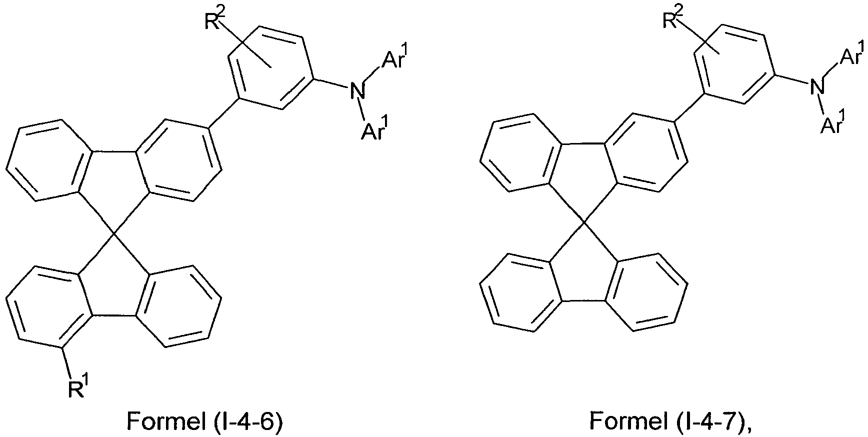 wobei die auftretenden Gruppen definiert sind wie oben, und wobei zusätzlich zu den eingezeichneten Resten R¹ und R² keine weiteren vorhanden sind.

Bevorzugt ist R³ bei jedem Auftreten gleich oder verschieden gewählt aus H, D, F, CN, Si(R⁴)₃, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen und die genannten aromatischen Ringsysteme und heteroaromatischen Ringsysteme jeweils mit einem oder mehreren Resten R⁴ substituiert sein können. Besonders bevorzugt ist R³ gleich H.

Bevorzugt ist R⁴ bei jedem Auftreten gleich oder verschieden gewählt aus H, D, F, CN, Si(R⁵)₃, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen und die genannten aromatischen Ringsysteme und heteroaromatischen Ringsysteme jeweils mit einem oder mehreren Resten R⁵ substituiert sein können. Besonders bevorzugt ist R⁴ gleich H.

Bevorzugt ist R⁵ bei jedem Auftreten gleich oder verschieden gewählt aus H, D, F, CN, Alkylgruppen mit 1 bis 20 C-Atomen, aromatischen Ringsystemen mit 6 bis 40 aromatischen Ringatomen und heteroaromatischen Ringsystemen mit 5 bis 40 aromatischen Ringatomen; wobei die genannten Alkylgruppen, aromatischen Ringsysteme und heteroaromatischen Ringsysteme mit F oder CN substituiert sein können.

Bevorzugt ist Ar¹, wenn es ein heteroaromatisches Ringsystem ist, bei jedem Auftreten gleich oder verschieden gewählt aus jeweils optional mit einem oder mehreren Resten R³ substituiertem Dibenzofuranyl, Dibenzothiophenyl, Benzofuranyl, Benzothiophenyl, benzo-annelliertem Dibenzofuranyl, benzo-annelliertem Dibenzothiophenyl, Indolyl, Chinolinyl, Pyridyl, Pyrimidyl, Pyrazinyl, Pyridazinyl, Triazinyl, Triazol, Oxazol, Oxadiazol, Benzoxazol, Benzothiazol, Phenanthrolyl und Azacarbazolyl.

Bevorzugt ist Ar¹, wenn es ein aromatisches Ringsystem ist, bei jedem Auftreten gleich oder verschieden gewählt aus aromatischen Ringsystemen mit 6 bis 12 aromatischen Ringatomen, die optional mit einem oder mehreren Resten R³ substituiert sind.

Besonders bevorzugte Gruppen Ar¹ sind jeweils optional mit einem oder mehreren Resten R³ substituiertes Phenyl, Biphenyl, Terphenyl, Quaterphenyl, Naphthyl, Fluorenyl, insbesondere 9,9'-Dimethylfluorenyl und 9,9'-Diphenylfluorenyl, Monobenzofluorenyl, Dibenzofluorenyl, Indenofluorenyl, Dibenzofuranyl, Dibenzothiophenyl, Benzofuranyl, Benzothiophenyl, benzo-annelliertem Dibenzofuranyl, benzo-annelliertem Dibenzothiophenyl, Indolyl, Chinolinyl, Pyridyl, Pyrimidyl, Pyrazinyl, Pyridazinyl und Triazinyl. Ganz besonders bevorzugt sind darunter mit einem oder mehreren Resten R³ substituiertes Phenyl, Biphenyl, Terphenyl, Quaterphenyl, Naphthyl, und Fluorenyl, insbesondere 9,9'-Dimethylfluorenyl und 9,9'-Diphenylfluorenyl.

Die Gruppen Ar¹ sind ganz besonders bevorzugt bei jedem Auftreten gleich oder verschieden gewählt aus Gruppen der folgenden Formeln:

| | | |
|---|---|---|
| 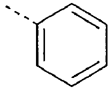 | 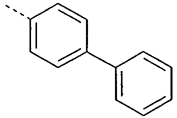 | 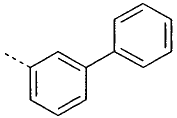 |
| Ar¹-1 | Ar¹-2 | Ar¹-3 |
| 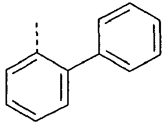 | 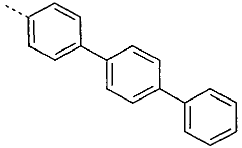 | 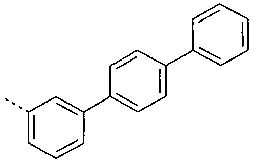 |
| Ar¹-4 | Ar¹-5 | Ar¹-6 |
| 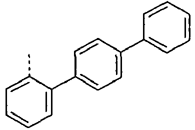 | 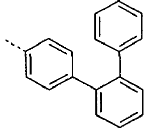 | 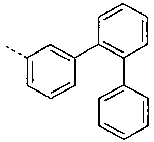 |
| Ar¹-7 | Ar¹-8 | Ar¹-9 |
| 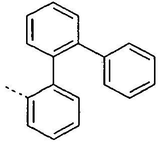 | 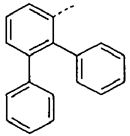 | 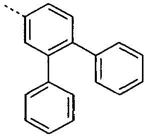 |
| Ar¹-10 | Ar¹-11 | Ar¹-12 |
| 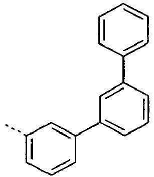 | 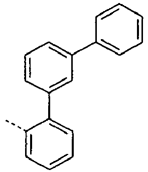 | 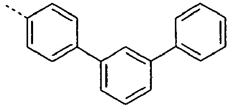 |
| Ar¹-13 | Ar¹-14 | Ar¹-15 |
| 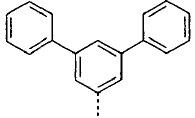 | 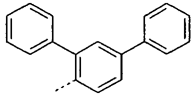 | 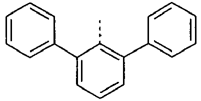 |
| Ar¹-16 | Ar¹-17 | Ar¹-18 |
| 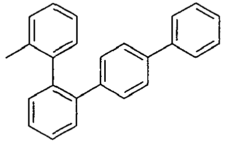 | 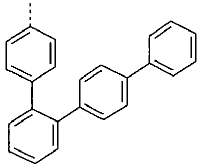 | 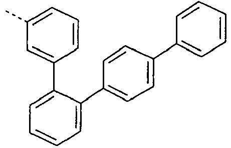 |
| Ar¹-19 | Ar¹-20 | Ar¹-21 |
| 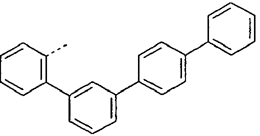 | 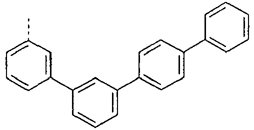 | 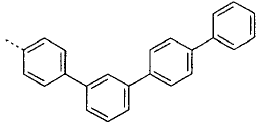 |
| Ar¹-22 | Ar¹-23 | Ar¹-24 |
| 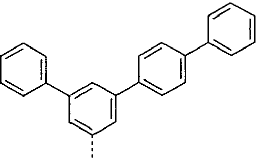 | 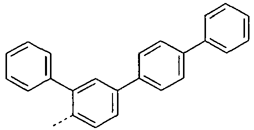 | 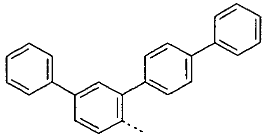 |
| Ar¹-25 | Ar¹-26 | Ar¹-27 |
| 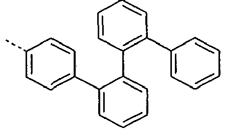 | 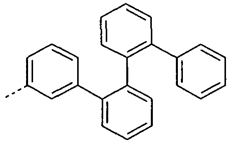 | 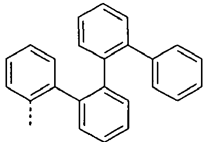 |
| Ar¹-28 | Ar¹-29 | Ar¹-30 |
| 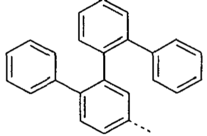 | 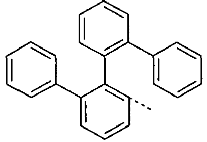 | 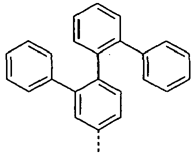 |
| Ar¹-31 | Ar¹-32 | Ar¹-33 |
| 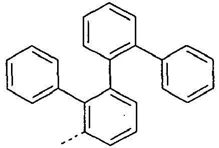 | 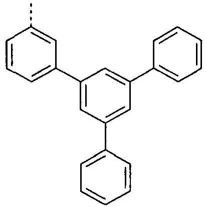 | 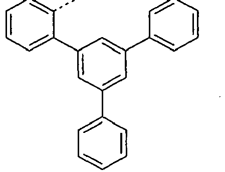 |
| Ar¹-34 | Ar¹-35 | Ar¹-36 |
| 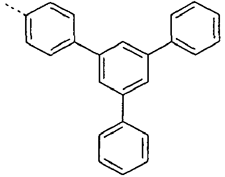 | 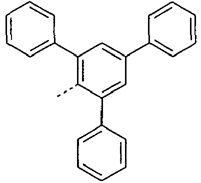 | 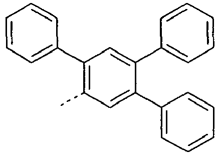 |
| Ar¹-37 | Ar¹-38 | Ar¹-39 |
| 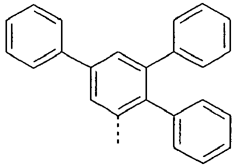 | 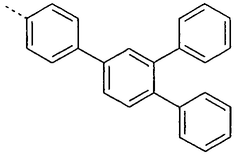 | 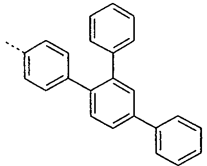 |
| Ar¹-40 | Ar¹-41 | Ar¹-42 |
| 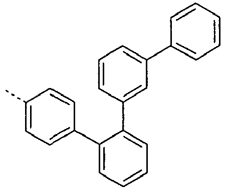 | 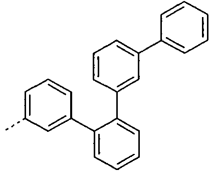 | 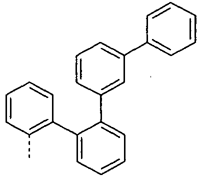 |
| Ar¹-43 | Ar¹-44 | Ar¹-45 |
| 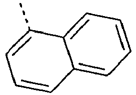 | 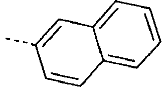 | 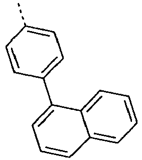 |
| Ar¹-46 | Ar¹-47 | Ar¹-48 |
| 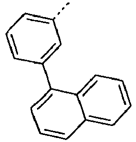 | 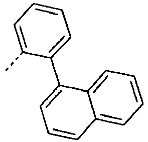 | 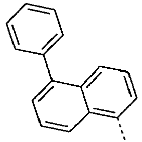 |
| Ar¹-49 | Ar¹-50 | Ar¹-51 |
| 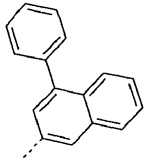 | 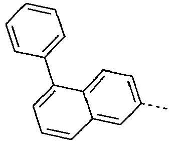 | 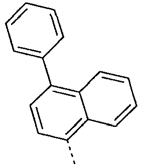 |
| Ar¹-52 | Ar¹-53 | Ar¹-54 |
| 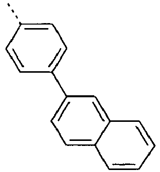 | 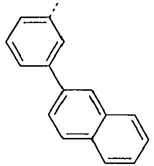 | 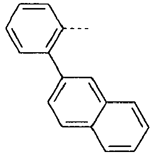 |
| Ar¹-55 | Ar¹-56 | Ar¹-57 |
| 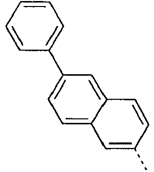 | | |
| Ar¹-58 | | |
| 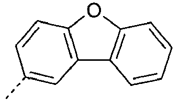 | 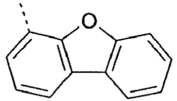 | 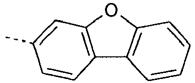 |
| Ar¹-59 | Ar¹-60 | Ar¹-61 |
| 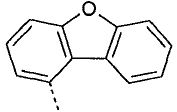 | 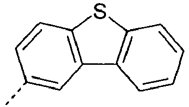 | 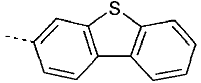 |
| Ar¹-62 | Ar¹-63 | Ar¹-64 |
| 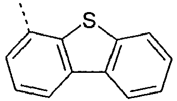 | 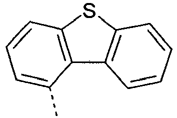 | 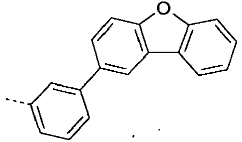 |
| Ar¹-65 | Ar¹-66 | Ar¹-67 |
| 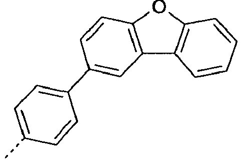 | 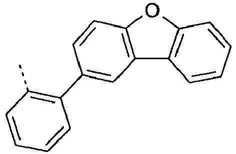 | 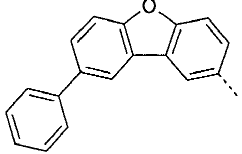 |
| Ar¹-68 | Ar¹-69 | Ar¹-70 |
| 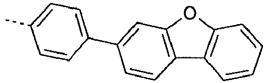 | 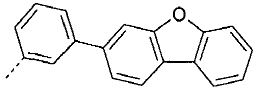 | 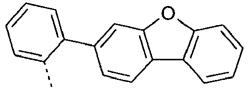 |
| Ar¹-71 | Ar¹-72 | Ar¹-73 |
| 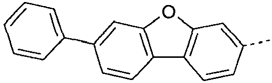 | 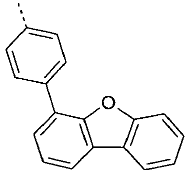 | 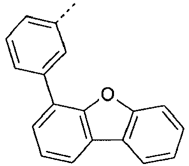 |
| Ar¹-74 | Ar¹-75 | Ar¹-76 |
| 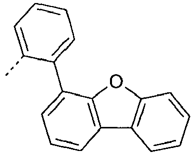 | 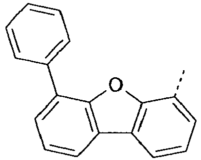 | 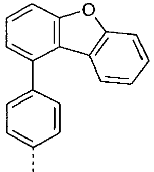 |
| Ar¹-77 | Ar¹-78 | Ar¹-79 |
| 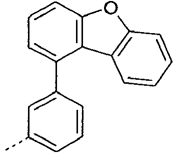 | 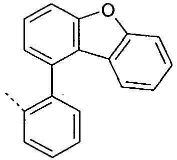 | 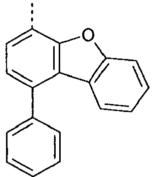 |
| Ar¹-80 | Ar¹-81 | Ar¹-82 |
| 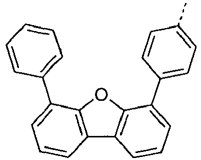 | 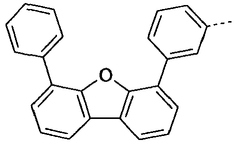 | 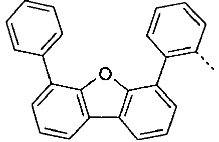 |
| Ar¹-83 | Ar¹-84 | Ar¹-85 |
| 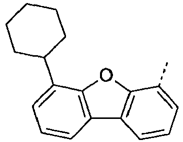 | 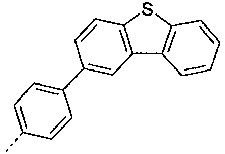 | 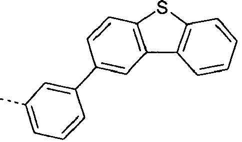 |
| Ar¹-86 | Ar¹-87 | Ar¹-88 |
| 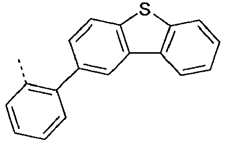 | 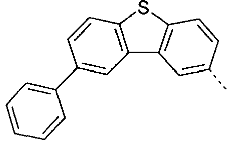 | 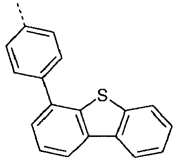 |
| Ar¹-89 | Ar¹-90 | Ar¹-91 |
| 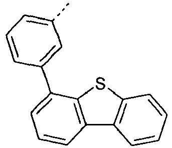 | 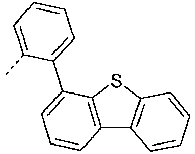 | 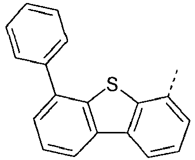 |
| Ar¹-92 | Ar¹-93 | Ar¹-94 |
| 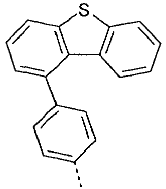 | 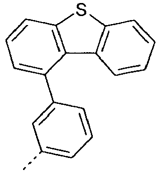 | 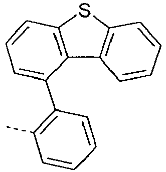 |
| Ar¹-95 | Ar¹-96 | Ar¹-97 |
| 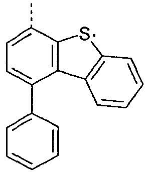 | 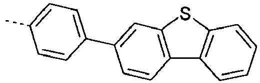 | 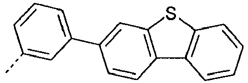 |
| Ar¹-98 | Ar¹-99 | Ar¹-100 |
| 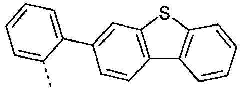 | 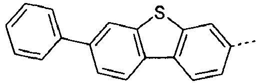 | 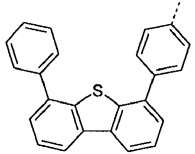 |
| Ar¹-101 | Ar¹-102 | Ar¹-103 |
| 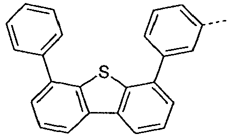 | 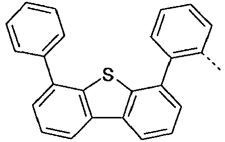 | 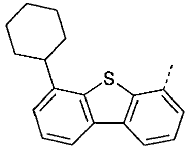 |
| Ar¹-104 | Ar¹-105 | Ar¹-106 |
| 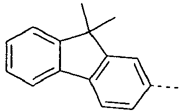 | 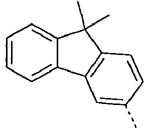 | 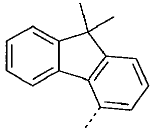 |
| Ar¹-107 | Ar¹-108 | Ar¹-109 |
| 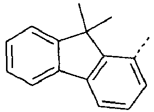 | 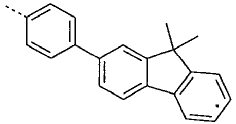 | 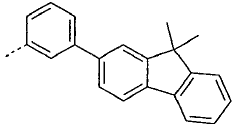 |
| Ar¹-110 | Ar¹-111 | Ar¹-112 |
| 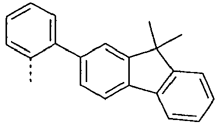 | 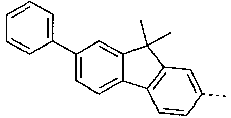 | 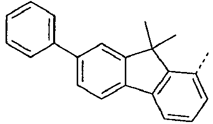 |
| Ar¹-113 | Ar¹-114 | Ar¹-115 |
| 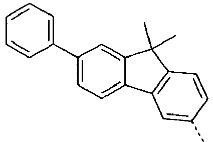 | 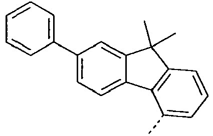 | 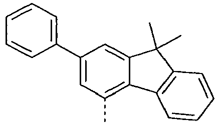 |
| Ar¹-116 | Ar¹-117 | Ar¹-118 |
| 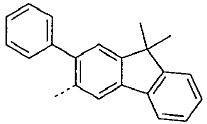 | 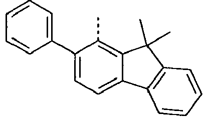 | 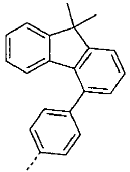 |
| Ar¹-119 | Ar¹-120 | Ar¹-121 |
| 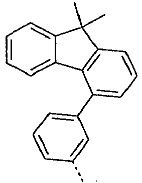 | 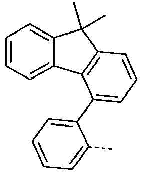 | 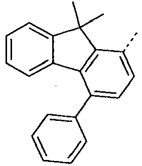 |
| Ar¹-122 | Ar¹-123 | Ar¹-124 |
| 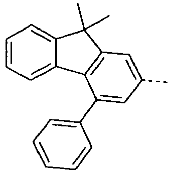 | 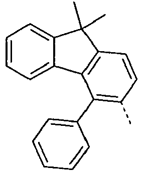 | 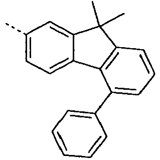 |
| Ar¹-125 | Ar¹-126 | Ar¹-127 |
| 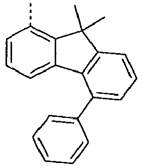 | 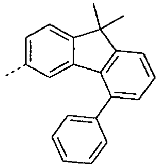 | 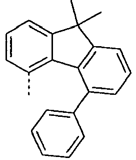 |
| Ar¹-128 | Ar¹-129 | Ar¹-130 |
| 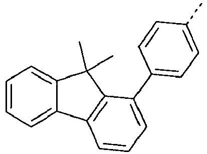 | 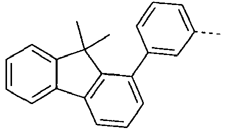 | 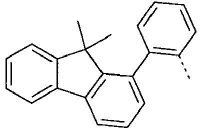 |
| Ar¹-131 | Ar¹-132 | Ar¹-133 |
| 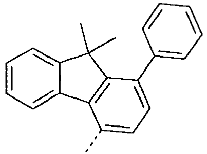 | 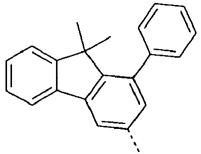 | 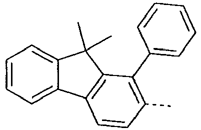 |
| Ar¹-134 | Ar¹-135 | Ar¹-136 |
| 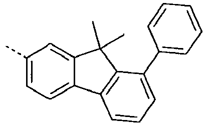 | 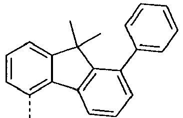 | 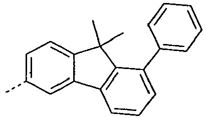 |
| Ar¹-137 | Ar¹-138 | Ar¹-139 |
| 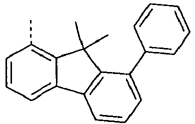 | 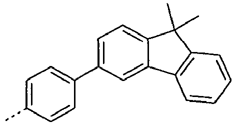 | 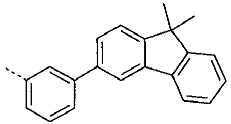 |
| Ar¹-140 | Ar¹-141 | Ar¹-142 |
| 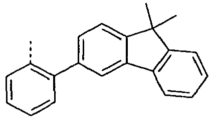 | 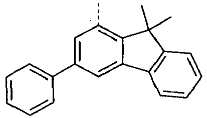 | 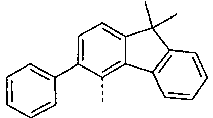 |
| Ar¹-143 | Ar¹-144 | Ar¹-145 |
| 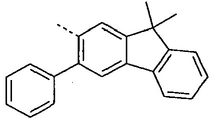 | 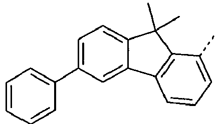 | 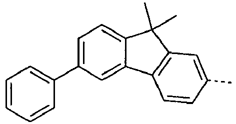 |
| Ar¹-146 | Ar¹-147 | Ar¹-148 |
| 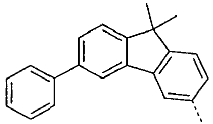 | 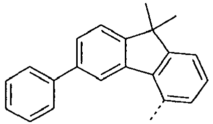 | 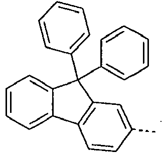 |
| Ar¹-149 | Ar¹-150 | Ar¹-151 |
| 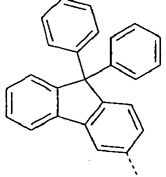 | 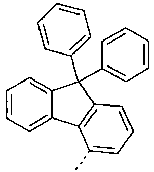 | 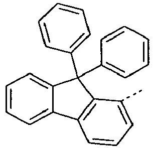 |
| Ar¹-152 | Ar¹-153 | Ar¹-154 |
| 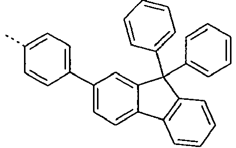 | 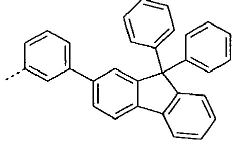 | 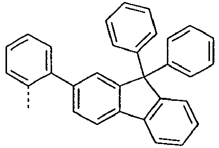 |
| Ar¹-155 | Ar¹-156 | Ar¹-157 |
| 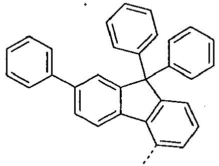 | 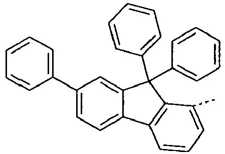 | 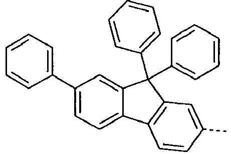 |
| Ar¹-158 | Ar¹-159 | Ar¹-160 |
| 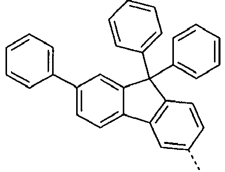 | 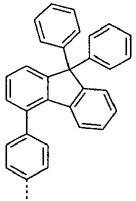 | 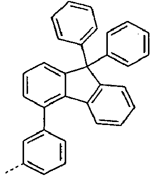 |
| Ar¹-161 | Ar¹-162 | Ar¹-163 |
| 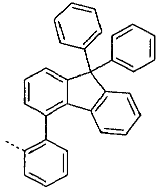 | 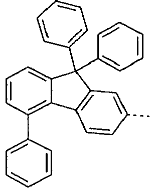 | 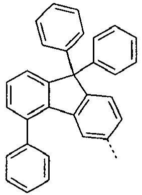 |
| Ar¹-164 | Ar¹-165 | Ar¹-166 |
| 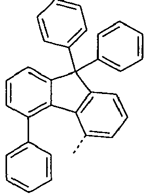 | 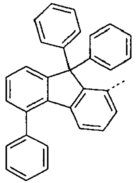 | 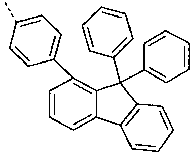 |
| Ar¹-167 | Ar¹-168 | Ar¹-169 |
| 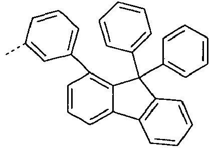 | 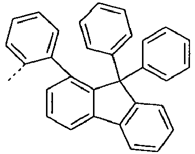 | 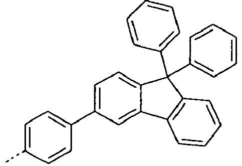 |
| Ar¹-169 | Ar¹-170 | Ar¹-171 |
| 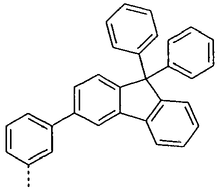 | 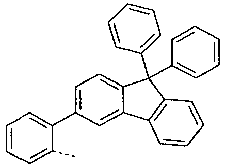 | 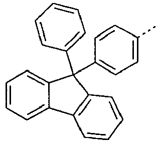 |
| Ar¹-172 | Ar¹-173 | Ar1-174 |
| 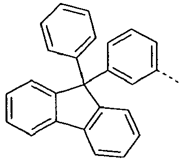 | 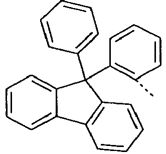 | 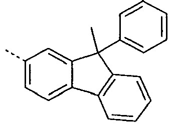 |
| Ar¹-175 | Ar¹-176 | Ar¹-177 |
| 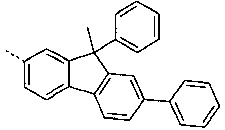 | 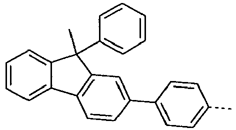 | 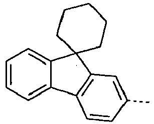 |
| Ar¹-178 | Ar¹-179 | Ar¹-180 |
| 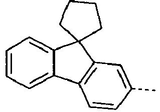 | 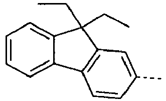 | 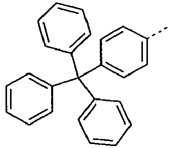 |
| Ar¹-181 | Ar¹-182 | Ar¹-183 |
| 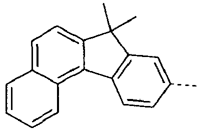 | 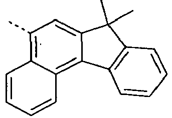 | 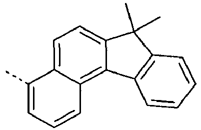 |
| Ar¹-184 | Ar¹-185 | Ar¹-186 |
| 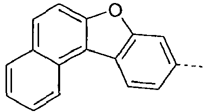 | 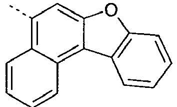 | |
| Ar¹-187 | Ar¹-188 | |
| 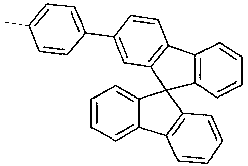 | 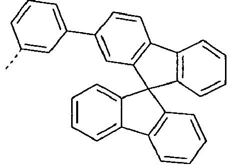 | 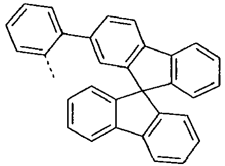 |
| Ar¹-189 | Ar¹-190 | Ar¹-191 |
| 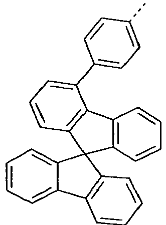 | 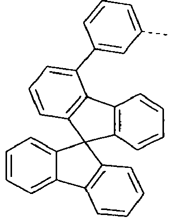 | 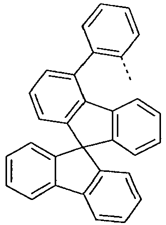 |
| Ar¹-192 | Ar¹-193 | Ar¹-194 |
| 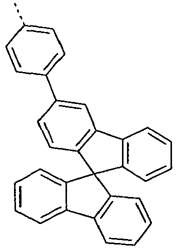 | 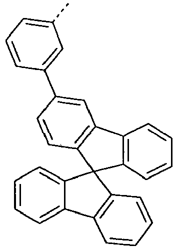 | 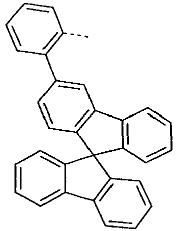 |
| Ar¹-195 | Ar¹-196 | Ar¹-197 |
| 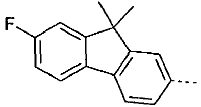 | 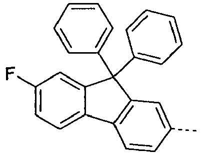 | 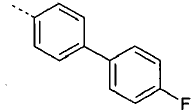 |
| Ar¹-198 | Ar¹-199 | Ar¹-200 |
| 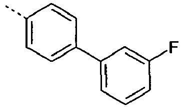 | 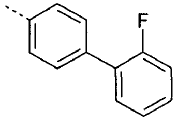 | 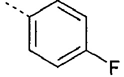 |
| Ar¹-201 | Ar¹-202 | Ar¹-203 |
| 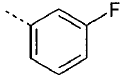 | 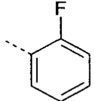 | 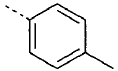 |
| Ar¹-204 | Ar¹-205 | Ar¹-206 |
| 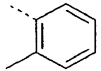 | 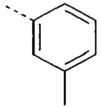 | 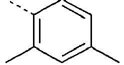 |
| Ar¹-207 | Ar¹-208 | Ar¹-209 |
| 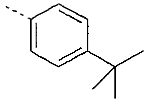 | 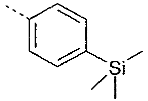 | 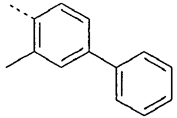 |
| Ar¹-210 | Ar¹-211 | Ar¹-212 |
| 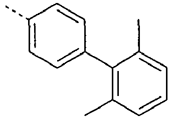 | 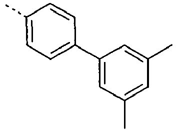 | 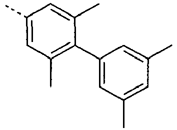 |
| Ar¹-213 | Ar¹-214 | Ar¹-215 |
| 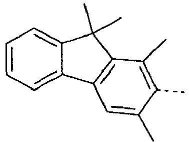 | 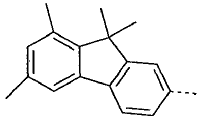 | 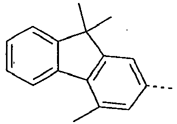 |
| Ar¹-216 | Ar¹-217 | Ar¹-218 |
| 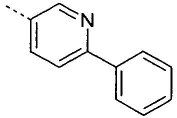 | 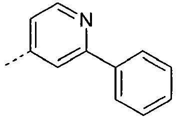 | 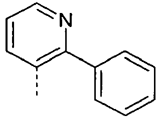 |
| Ar¹-219 | Ar¹-220 | Ar¹-221 |
| 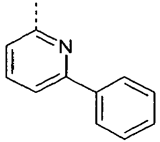 | 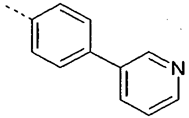 | 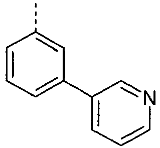 |
| Ar¹-222 | Ar¹-223 | Ar¹-224 |
| 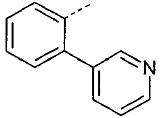 | 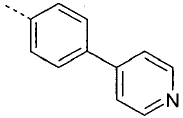 | 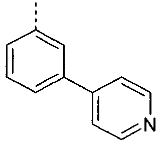 |
| Ar¹-225 | Ar¹-226 | Ar¹-227 |
| 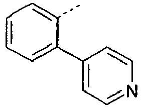 | 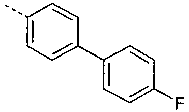 | 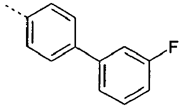 |
| Ar¹-228 | Ar¹-229 | Ar¹-230 |
| 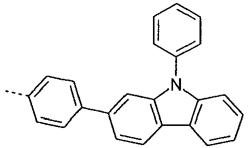 | 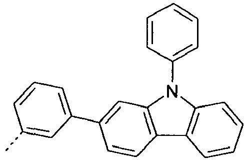 | 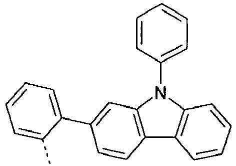 |
| Ar¹-231 | Ar¹-232 | Ar¹-233 |
| 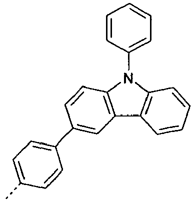 | 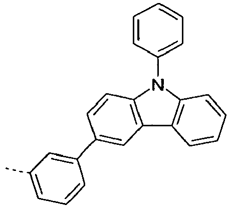 | 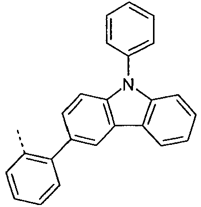 |
| Ar1-234 | Ar¹-235 | Ar¹-236 |
| 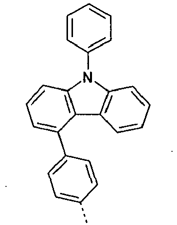 | 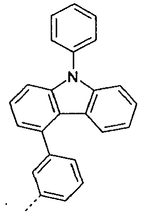 | 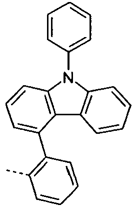 |
| Ar¹-237 | Ar¹-238 | Ar¹-239 |
| 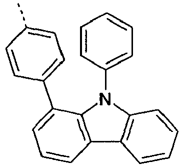 | 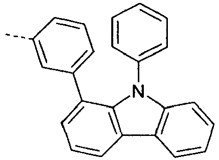 | 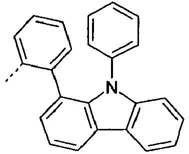 |
| Ar¹-240 | Ar¹-241 | Ar¹-242 |
| 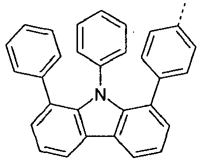 | 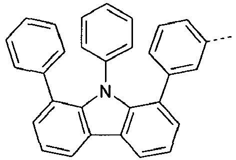 | 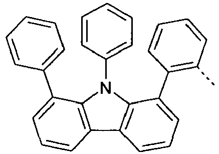 |
| Ar¹-243 | Ar¹-244 | Ar¹-245 |
| 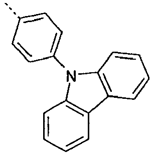 | 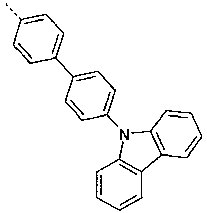 | 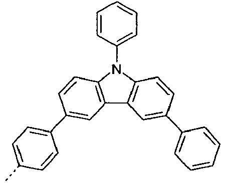 |
| Ar¹-246 | Ar¹-247 | Ar¹-248 |
| 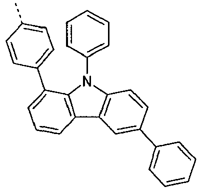 | 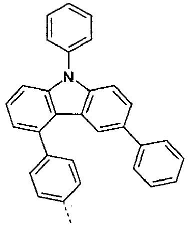 | 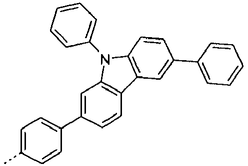 |
| Ar¹-249 | Ar¹-250 | Ar¹-251 |

wobei die Gruppen an allen freien Positionen jeweils mit Resten R³ substituiert sein können, welche wie oben definiert sind. Bevorzugt sind sie an allen freien Positionen unsubstituiert.

Es ist insbesondere bevorzugt, dass die bevorzugten Ausführungsformen der Grundstruktur der Formel (I) kombiniert mit den bevorzugten Ausführungsformen der Gruppen Ar¹, R¹, R², R³, R⁴ und R⁵ auftreten.

Bevorzugte Verbindungen gemäß Formel (I) entsprechen einer der folgenden Strukturen, wobei das Grundgerüst gemäß Formel (I-1) oder Formel (I-2) an den freien Positionen jeweils nicht mit Resten R¹ und R² substituiert ist, und wobei die Gruppen Ar¹ keine weiteren Substituenten als die in den entsprechenden Formeln gezeigten Substituenten aufweisen:

| Nr. | Grundgerüst gemäß | Eine der Gruppen Ar¹ gemäß | Andere der Gruppen Ar¹ gemäß | Nr. | Grundgerüst gemäß | Die eine der Gruppen Ar¹ ist gemäß | Die andere der Gruppen Ar¹ ist gemäß |
|---|---|---|---|---|---|---|---|
| 1 | Formel (I-1) | Ar¹-1 | Ar¹-1 | 981 | Formel (I-2) | Ar¹-1 | Ar¹-1 |
| 2 | Formel (I-1) | Ar¹-1 | Ar¹-2 | 982 | Formel (I-2) | Ar¹-1 | Ar¹-2 |
| 3 | Formel (I-1) | Ar¹-1 | Ar¹-3 | 983 | Formel (I-2) | Ar¹-1 | Ar¹-3 |
| 4 | Formel (I-1) | Ar¹-1 | Ar¹-4 | 984 | Formel (I-2) | Ar¹-1 | Ar¹-4 |
| 5 | Formel (I-1) | Ar¹-1 | Ar¹-59 | 985 | Formel (I-2) | Ar¹-1 | Ar¹-59 |
| 6 | Formel (I-1) | Ar¹-1 | Ar¹-60 | 986 | Formel (I-2) | Ar¹-1 | Ar¹-60 |
| 7 | Formel (I-1) | Ar¹-1 | Ar¹-63 | 987 | Formel (I-2) | Ar¹-1 | Ar¹-63 |
| 8 | Formel (I-1) | Ar¹-1 | Ar¹-65 | 988 | Formel (I-2) | Ar¹-1 | Ar¹-65 |
| 9 | Formel (I-1) | Ar¹-1 | Ar¹-68 | 989 | Formel (I-2) | Ar¹-1 | Ar¹-68 |
| 10 | Formel (I-1) | Ar¹-1 | Ar¹-75 | 990 | Formel (I-2) | Ar¹-1 | Ar¹-75 |
| 11 | Formel (I-1) | Ar¹-1 | Ar¹-87 | 991 | Formel (I-2) | Ar¹-1 | Ar¹-87 |
| 12 | Formel (I-1) | Ar¹-1 | Ar¹-91 | 992 | Formel (I-2) | Ar¹-1 | Ar¹-91 |
| 13 | Formel (I-1) | Ar¹-1 | Ar¹-107 | 993 | Formel (I-2) | Ar¹-1 | Ar¹-107 |
| 14 | Formel (I-1) | Ar¹-1 | Ar¹-109 | 994 | Formel (I-2) | Ar¹-1 | Ar¹-109 |
| 15 | Formel (I-1) | Ar¹-1 | Ar¹-111 | 995 | Formel (I-2) | Ar¹-1 | Ar¹-111 |
| 16 | Formel (I-1) | Ar¹-1 | Ar¹-114 | 996 | Formel (I-2) | Ar¹-1 | Ar¹-114 |
| 17 | Formel (I-1) | Ar¹-1 | Ar¹-121 | 997 | Formel (I-2) | Ar¹-1 | Ar¹-121 |
| 18 | Formel (I-1) | Ar¹-1 | Ar¹-151 | 998 | Formel (I-2) | Ar¹-1 | Ar¹-151 |
| 19 | Formel (I-1) | Ar¹-1 | Ar¹-153 | 999 | Formel (I-2) | Ar¹-1 | Ar¹-153 |
| 20 | Formel (I-1) | Ar¹-1 | Ar¹-162 | 1000 | Formel (I-2) | Ar¹-1 | Ar¹-162 |
| 21 | Formel (I-1) | Ar¹-2 | Ar¹-1 | 1001 | Formel (I-2) | Ar¹-2 | Ar¹-1 |
| 22 | Formel (I-1) | Ar¹-2 | Ar¹-2 | 1002 | Formel (I-2) | Ar¹-2 | Ar¹-2 |
| 23 | Formel (I-1) | Ar¹-2 | Ar¹-3 | 1003 | Formel (I-2) | Ar¹-2 | Ar¹-3 |
| 24 | Formel (I-1) | Ar¹-2 | Ar¹-4 | 1004 | Formel (I-2) | Ar¹-2 | Ar¹-4 |
| 25 | Formel (I-1) | Ar¹-2 | Ar¹-59 | 1005 | Formel (I-2) | Ar¹-2 | Ar¹-59 |
| 26 | Formel (I-1) | Ar¹-2 | Ar¹-60 | 1006 | Formel (I-2) | Ar¹-2 | Ar¹-60 |
| 27 | Formel (I-1) | Ar¹-2 | Ar¹-63 | 1007 | Formel (I-2) | Ar¹-2 | Ar¹-63 |
| 28 | Formel (I-1) | Ar¹-2 | Ar¹-65 | 1008 | Formel (I-2) | Ar¹-2 | Ar¹-65 |
| 29 | Formel (I-1) | Ar¹-2 | Ar¹-68 | 1009 | Formel (I-2) | Ar¹-2 | Ar¹-68 |
| 30 | Formel (I-1) | Ar¹-2 | Ar¹-75 | 1010 | Formel (I-2) | Ar¹-2 | Ar¹-75 |
| 31 | Formel (I-1) | Ar¹-2 | Ar¹-87 | 1011 | Formel (I-2) | Ar¹-2 | Ar¹-87 |
| 32 | Formel (I-1) | Ar¹-2 | Ar¹-91 | 1012 | Formel (I-2) | Ar¹-2 | Ar¹-91 |
| 33 | Formel (I-1) | Ar¹-2 | Ar¹-107 | 1013 | Formel (I-2) | Ar¹-2 | Ar¹-107 |
| 34 | Formel (I-1) | Ar¹-2 | Ar¹-109 | 1014 | Formel (I-2) | Ar¹-2 | Ar¹-109 |
| 35 | Formel (I-1) | Ar¹-2 | Ar¹-111 | 1015 | Formel (I-2) | Ar¹-2 | Ar¹-111 |
| 36 | Formel (I-1) | Ar¹-2 | Ar¹-114 | 1016 | Formel (I-2) | Ar¹-2 | Ar¹-114 |
| 37 | Formel (I-1) | Ar¹-2 | Ar¹-121 | 1017 | Formel (I-2) | Ar¹-2 | Ar¹-121 |
| 38 | Formel (I-1) | Ar¹-2 | Ar¹-151 | 1018 | Formel (I-2) | Ar¹-2 | Ar¹-151 |
| 39 | Formel (I-1) | Ar¹-2 | Ar¹-153 | 1019 | Formel (I-2) | Ar¹-2 | Ar¹-153 |
| 40 | Formel (I-1) | Ar¹-2 | Ar¹-162 | 1020 | Formel (I-2) | Ar¹-2 | Ar¹-162 |
| 41 | Formel (I-1) | Ar¹-3 | Ar¹-1 | 1021 | Formel (I-2) | Ar¹-3 | Ar¹-1 |
| 42 | Formel (I-1) | Ar¹-3 | Ar¹-2 | 1022 | Formel (I-2) | Ar¹-3 | Ar¹-2 |
| 43 | Formel (I-1) | Ar¹-3 | Ar¹-3 | 1023 | Formel (I-2) | Ar¹-3 | Ar¹-3 |
| 44 | Formel (I-1) | Ar¹-3 | Ar¹-4 | 1024 | Formel (I-2) | Ar¹-3 | Ar¹-4 |
| 45 | Formel (I-1) | Ar¹-3 | Ar¹-59 | 1025 | Formel (I-2) | Ar¹-3 | Ar¹-59 |
| 46 | Formel (I-1) | Ar¹-3 | Ar¹-60 | 1026 | Formel (I-2) | Ar¹-3 | Ar¹-60 |
| 47 | Formel (I-1) | Ar¹-3 | Ar¹-63 | 1027 | Formel (I-2) | Ar¹-3 | Ar¹-63 |
| 48 | Formel (I-1) | Ar¹-3 | Ar¹-65 | 1028 | Formel (I-2) | Ar¹-3 | Ar¹-65 |
| 49 | Formel (I-1) | Ar¹-3 | Ar¹-68 | 1029 | Formel (I-2) | Ar¹-3 | Ar¹-68 |
| 50 | Formel (I-1) | Ar¹-3 | Ar¹-75 | 1030 | Formel (I-2) | Ar¹-3 | Ar¹-75 |
| 51 | Formel (I-1) | Ar¹-3 | Ar¹-87 | 1031 | Formel (I-2) | Ar¹-3 | Ar¹-87 |
| 52 | Formel (I-1) | Ar¹-3 | Ar¹-91 | 1032 | Formel (I-2) | Ar¹-3 | Ar¹-91 |
| 53 | Formel (I-1) | Ar¹-3 | Ar¹-107 | 1033 | Formel (I-2) | Ar¹-3 | Ar¹-107 |
| 54 | Formel (I-1) | Ar¹-3 | Ar¹-109 | 1034 | Formel (I-2) | Ar¹-3 | Ar¹-109 |
| 55 | Formel (I-1) | Ar¹-3 | Ar¹-111 | 1035 | Formel (I-2) | Ar¹-3 | Ar¹-111 |
| 56 | Formel (I-1) | Ar¹-3 | Ar¹-114 | 1036 | Formel (I-2) | Ar¹-3 | Ar¹-114 |
| 57 | Formel (I-1) | Ar¹-3 | Ar¹-121 | 1037 | Formel (I-2) | Ar¹-3 | Ar¹-121 |
| 58 | Formel (I-1) | Ar¹-3 | Ar¹-151 | 1038 | Formel (I-2) | Ar¹-3 | Ar¹-151 |
| 59 | Formel (I-1) | Ar¹-3 | Ar¹-153 | 1039 | Formel (I-2) | Ar¹-3 | Ar¹-153 |
| 60 | Formel (I-1) | Ar¹-3 | Ar¹-162 | 1040 | Formel (I-2) | Ar¹-3 | Ar¹-162 |
| 61 | Formel (I-1) | Ar¹-4 | Ar¹-1 | 1041 | Formel (I-2) | Ar¹-4 | Ar¹-1 |
| 62 | Formel (I-1) | Ar¹-4 | Ar¹-2 | 1042 | Formel (I-2) | Ar¹-4 | Ar¹-2 |
| 63 | Formel (I-1) | Ar¹-4 | Ar¹-3 | 1043 | Formel (I-2 | Ar¹-4 | Ar¹-3 |
| 64 | Formel (I-1) | Ar¹-4 | Ar¹-4 | 1044 | Formel (I-2) | Ar¹-4 | Ar¹-4 |
| 65 | Formel (I-1) | Ar¹-4 | Ar¹-59 | 1045 | Formel (I-2) | Ar¹-4 | Ar¹-59 |
| 66 | Formel (I-1) | Ar¹-4 | Ar¹-60 | 1046 | Formel (I-2) | Ar¹-4 | Ar¹-60 |
| 67 | Formel (I-1) | Ar¹-4 | Ar¹-63 | 1047 | Formel (I-2) | Ar¹-4 | Ar¹-63 |
| 68 | Formel (I-1) | Ar¹-4 | Ar¹-65 | 1048 | Formel (I-2) | Ar¹-4 | Ar¹-65 |
| 69 | Formel (I-1) | Ar¹-4 | Ar¹-68 | 1049 | Formel (I-2) | Ar¹-4 | Ar¹-68 |
| 70 | Formel (I-1) | Ar¹-4 | Ar¹-75 | 1050 | Formel (I-2) | Ar¹-4 | Ar¹-75 |
| 71 | Formel (I-1) | Ar¹-4 | Ar¹-87 | 1051 | Formel (I-2) | Ar¹-4 | Ar¹-87 |
| 72 | Formel (I-1) | Ar¹-4 | Ar¹-91 | 1052 | Formel (I-2) | Ar¹-4 | Ar¹-91 |
| 73 | Formel (I-1) | Ar¹-4 | Ar¹-107 | 1053 | Formel (I-2) | Ar¹-4 | Ar¹-107 |
| 74 | Formel (I-1) | Ar¹-4 | Ar¹-109 | 1054 | Formel (I-2) | Ar¹-4 | Ar¹-109 |
| 75 | Formel (I-1) | Ar¹-4 | Ar¹-111 | 1055 | Formel (I-2) | Ar¹-4 | Ar¹-111 |
| 76 | Formel (I-1) | Ar¹-4 | Ar¹-114 | 1056 | Formel (I-2) | Ar¹-4 | Ar¹-114 |
| 77 | Formel (I-1) | Ar¹-4 | Ar¹-121 | 1057 | Formel (I-2) | Ar¹-4 | Ar¹-121 |
| 78 | Formel (I-1) | Ar¹-4 | Ar¹-151 | 1058 | Formel (I-2) | Ar¹-4 | Ar¹-151 |
| 79 | Formel (I-1) | Ar¹-4 | Ar¹-153 | 1059 | Formel (I-2) | Ar¹-4 | Ar¹-153 |
| 80 | Formel (I-1) | Ar¹-4 | Ar¹-162 | 1060 | Formel (I-2) | Ar¹-4 | Ar¹-162 |
| 81 | Formel (I-1) | Ar¹-5 | Ar¹-1 | 1061 | Formel (I-2) | Ar¹-5 | Ar¹-1 |
| 82 | Formel (I-1) | Ar¹-5 | Ar¹-2 | 1062 | Formel (I-2) | Ar¹-5 | Ar¹-2 |
| 83 | Formel (I-1) | Ar¹-5 | Ar¹-3 | 1063 | Formel (I-2) | Ar¹-5 | Ar¹-3 |
| 84 | Formel (I-1) | Ar¹-5 | Ar¹-4 | 1064 | Formel (I-2) | Ar¹-5 | Ar¹-4 |
| 85 | Formel (I-1) | Ar¹-5 | Ar¹-59 | 1065 | Formel (I-2) | Ar¹-5 | Ar¹-59 |
| 86 | Formel (I-1) | Ar¹-5 | Ar¹-60 | 1066 | Formel (I-2) | Ar¹-5 | Ar¹-60 |
| 87 | Formel (I-1) | Ar¹-5 | Ar¹-63 | 1067 | Formel (I-2) | Ar¹-5 | Ar¹-63 |
| 88 | Formel (I-1) | Ar¹-5 | Ar¹-65 | 1068 | Formel (I-2) | Ar¹-5 | Ar¹-65 |
| 89 | Formel (I-1) | Ar¹-5 | Ar¹-68 | 1069 | Formel (I-2) | Ar¹-5 | Ar¹-68 |
| 90 | Formel (I-1) | Ar¹-5 | Ar¹-75 | 1070 | Formel (I-2) | Ar¹-5 | Ar¹-75 |
| 91 | Formel (I-1) | Ar¹-5 | Ar¹-87 | 1071 | Formel (I-2) | Ar¹-5 | Ar¹-87 |
| 92 | Formel (I-1) | Ar¹-5 | Ar¹-91 | 1072 | Formel (I-2) | Ar¹-5 | Ar¹-91 |
| 93 | Formel (I-1) | Ar¹-5 | Ar¹-107 | 1073 | Formel (I-2) | Ar¹-5 | Ar¹-107 |
| 94 | Formel (I-1) | Ar¹-5 | Ar¹-109 | 1074 | Formel (I-2) | Ar¹-5 | Ar¹-109 |
| 95 | Formel (I-1) | Ar¹-5 | Ar¹-111 | 1075 | Formel (I-2) | Ar¹-5 | Ar¹-111 |
| 96 | Formel (I-1) | Ar¹-5 | Ar¹-114 | 1076 | Formel (I-2) | Ar¹-5 | Ar¹-114 |
| 97 | Formel (I-1) | Ar¹-5 | Ar¹-121 | 1077 | Formel (I-2) | Ar¹-5 | Ar¹-121 |
| 98 | Formel (I-1) | Ar¹-5 | Ar¹-151 | 1078 | Formel (I-2) | Ar¹-5 | Ar¹-151 |
| 99 | Formel (I-1) | Ar¹-5 | Ar¹-153 | 1079 | Formel (I-2) | Ar¹-5 | Ar¹-153 |
| 100 | Formel (I-1) | Ar¹-5 | Ar¹-162 | 1080 | Formel (I-2) | Ar¹-5 | Ar¹-162 |
| 101 | Formel (I-1) | Ar¹-59 | Ar¹-1 | 1081 | Formel (I-2) | Ar¹-59 | Ar¹-1 |
| 102 | Formel (I-1) | Ar¹-59 | Ar¹-2 | 1082 | Formel (I-2) | Ar¹-59 | Ar¹-2 |
| 103 | Formel (I-1) | Ar¹-59 | Ar¹-3 | 1083 | Formel (I-2) | Ar¹-59 | Ar¹-3 |
| 104 | Formel (I-1) | Ar¹-59 | Ar¹-4 | 1084 | Formel (I-2) | Ar¹-59 | Ar¹-4 |
| 105 | Formel (I-1) | Ar¹-59 | Ar¹-59 | 1085 | . Formel (I-2) | Ar¹-59 | Ar¹-59 |
| 106 | Formel (I-1) | Ar¹-59 | Ar¹-60 | 1086 | Formel (I-2) | Ar¹-59 | Ar¹-60 |
| 107 | Formel (I-1) | Ar¹-59 | Ar¹-63 | 1087 | Formel (I-2) | Ar¹-59 | Ar¹-63 |
| 108 | Formel (I-1) | Ar¹-59 | Ar¹-65 | 1088 | Formel (I-2) | Ar¹-59 | Ar¹-65 |
| 109 | Formel (I-1) | Ar¹-59 | Ar¹-68 | 1089 | Formel (I-2) | Ar¹-59 | Ar¹-68 |
| 110 | Formel (I-1) | Ar¹-59 | Ar¹-75 | 1090 | Formel (I-2) | Ar¹-59 | Ar¹-75 |
| 111 | Formel (I-1) | Ar¹-59 | Ar¹-87 | 1091 | Formel (I-2) | Ar¹-59 | Ar¹-87 |
| 112 | Formel (I-1) | Ar¹-59 | Ar¹-91 | 1092 | Formel (I-2) | Ar¹-59 | Ar¹-91 |
| 113 | Formel (I-1) | Ar¹-59 | Ar¹-107 | 1093 | Formel (I-2) | Ar¹-59 | Ar¹-107 |
| 114 | Formel (I-1) | Ar¹-59 | Ar¹-109 | 1094 | Formel (I-2) | Ar¹-59 | Ar¹-109 |
| 115 | Formel (I-1) | Ar¹-59 | Ar¹-111 | 1095 | Formel (I-2) | Ar¹-59 | Ar¹-111 |
| 116 | Formel (I-1) | Ar¹-59 | Ar¹-114 | 1096 | Formel (I-2) | Ar¹-59 | Ar¹-114 |
| 117 | Formel (I-1) | Ar¹-59 | Ar¹-121 | 1097 | Formel (I-2) | Ar¹-59 | Ar¹-121 |
| 118 | Formel (I-1) | Ar¹-59 | Ar¹-151 | 1098 | Formel (I-2) | Ar¹-59 | Ar¹-151 |
| 119 | Formel (I-1) | Ar¹-59 | Ar¹-153 | 1099 | Formel (I-2) | Ar¹-59 | Ar¹-153 |
| 120 | Formel (I-1) | Ar¹-59 | Ar¹-162 | 1100 | Formel (I-2) | Ar¹-59 | Ar¹-162 |
| 121 | Formel (I-1) | Ar¹-60 | Ar¹-1 | 1101 | Formel (I-2) | Ar¹-60 | Ar¹-1 |
| 122 | Formel (I-1) | Ar¹-60 | Ar¹-2 | 1102 | Formel (I-2) | Ar¹-60 | Ar¹-2 |
| 123 | Formel (I-1) | Ar¹-60 | Ar¹-3 | 1103 | Formel (I-2) | Ar¹-60 | Ar¹-3 |
| 124 | Formel (I-1) | Ar¹-60 | Ar¹-4 | 1104 | Formel (I-2) | Ar¹-60 | Ar¹-4 |
| 125 | Formel (I-1) | Ar¹-60 | Ar¹-59 | 1105 | Formel (I-2) | Ar¹-60 | Ar¹-59 |
| 126 | Formel (I-1) | Ar¹-60 | Ar¹-60 | 1106 | Formel (I-2) | Ar¹-60 | Ar¹-60 |
| 127 | Formel (I-1) | Ar¹-60 | Ar¹-63 | 1107 | Formel (I-2) | Ar¹-60 | Ar¹-63 |
| 128 | Formel (I-1) | Ar¹-60 | Ar¹-65 | 1108 | Formel (I-2) | Ar¹-60 | Ar¹-65 |
| 129 | Formel (I-1) | Ar¹-60 | Ar¹-68 | 1109 | Formel (I-2) | Ar¹-60 | Ar¹-68 |
| 130 | Formel (I-1) | Ar¹-60 | Ar¹-75 | 1110 | Formel (I-2) | Ar¹-60 | Ar¹-75 |
| 131 | Formel (I-1) | Ar¹-60 | Ar¹-87 | 1111 | Formel (I-2) | Ar¹-60 | Ar¹-87 |
| 132 | Formel (I-1) | Ar¹-60 | Ar¹-91 | 1112 | Formel (I-2) | Ar¹-60 | Ar¹-91 |
| 133 | Formel (I-1) | Ar¹-60 | Ar¹-107 | 1113 | Formel (I-2) | Ar¹-60 | Ar¹-107 |
| 134 | Formel (I-1) | Ar¹-60 | Ar¹-109 | 1114 | Formel (I-2) | Ar¹-60 | Ar¹-109 |
| 135 | Formel (I-1) | Ar¹-60 | Ar¹-111 | 1115 | Formel (I-2) | Ar¹-60 | Ar¹-111 |
| 136 | Formel (I-1) | Ar¹-60 | Ar¹-114 | 1116 | Formel (I-2) | Ar¹-60 | Ar¹-114 |
| 137 | Formel (I-1) | Ar¹-60 | Ar¹-121 | 1117 | Formel (I-2) | Ar¹-60 | Ar¹-121 |
| 138 | Formel (I-1) | Ar¹-60 | Ar¹-151 | 1118 | Formel (I-2) | Ar¹-60 | Ar¹-151 |
| 139 | Formel (I-1) | Ar¹-60 | Ar¹-153 | 1119 | Formel (I-2) | Ar¹-60 | Ar¹-153 |
| 140 | Formel (I-1) | Ar¹-60 | Ar¹-162 | 1120 | Formel (I-2) | Ar¹-60 | Ar¹-162 |
| 141 | Formel (I-1) | Ar¹-61 | Ar¹-1 | 1121 | Formel (I-2) | Ar¹-61 | Ar¹-1 |
| 142 | Formel (I-1) | Ar¹-61 | Ar¹-2 | 1122 | Formel (I-2) | Ar¹-61 | Ar¹-2 |
| 143 | Formel (I-1) | Ar¹-61 | Ar¹-3 | 1123 | Formel (I-2) | Ar¹-61 | Ar¹-3 |
| 144 | Formel (I-1) | Ar¹-61 | Ar¹-4 | 1124 | Formel (I-2) | Ar¹-61 | Ar¹-4 |
| 145 | Formel (I-1) | Ar¹-61 | Ar¹-59 | 1125 | Formel (I-2) | Ar¹-61 | Ar¹-59 |
| 146 | Formel (I-1) | Ar¹-61 | Ar¹-60 | 1126 | Formel (I-2) | Ar¹-61 | Ar¹-60 |
| 147 | Formel (I-1) | Ar¹-61 | Ar¹-63 | 1127 | Formel (I-2) | Ar¹-61 | Ar¹-63 |
| 148 | Formel (I-1) | Ar¹-61 | Ar¹-65 | 1128 | Formel (I-2) | Ar¹-61 | Ar¹-65 |
| 149 | Formel (I-1) | Ar¹-61 | Ar¹-68 | 1129 | Formel (I-2) | Ar¹-61 | Ar¹-68 |
| 150 | Formel (I-1) | Ar¹-61 | Ar¹-75 | 1130 | Formel (I-2) | Ar¹-61 | Ar¹-75 |
| 151 | Formel (I-1) | Ar¹-61 | Ar¹-87 | 1131 | Formel (I-2) | Ar¹-61 | Ar¹-87 |
| 152 | Formel (I-1) | Ar¹-61 | Ar¹-91 | 1132 | Formel (I-2) | Ar¹-61 | Ar¹-91 |
| 153 | Formel (I-1) | Ar¹-61 | Ar¹-107 | 1133 | Formel (I-2) | Ar¹-61 | Ar¹-107 |
| 154 | Formel (I-1) | Ar¹-61 | Ar¹-109 | 1134 | Formel (I-2) | Ar¹-61 | Ar¹-109 |
| 155 | Formel (I-1) | Ar¹-61 | Ar¹-111 | 1135 | Formel (I-2) | Ar¹-61 | Ar¹-111 |
| 156 | Formel (I-1) | Ar¹-61 | Ar¹-114 | 1136 | Formel (I-2) | Ar¹-61 | Ar¹-114 |
| 157 | Formel (I-1) | Ar¹-61 | Ar¹-121 | 1137 | Formel (I-2) | Ar¹-61 | Ar¹-121 |
| 158 | Formel (I-1) | Ar¹-61 | Ar¹-151 | 1138 | Formel (I-2) | Ar¹-61 | Ar¹-151 |
| 159 | Formel (I-1) | Ar¹-61 | Ar¹-153 | 1139 | Formel (I-2) | Ar¹-61 | Ar¹-153 |
| 160 | Formel (I-1) | Ar¹-61 | Ar¹-162 | 1140 | Formel (I-2) | Ar¹-61 | Ar¹-162 |
| 161 | Formel (I-1) | Ar¹-62 | Ar¹-1 | 1141 | Formel (I-2) | Ar¹-62 | Ar¹-1 |
| 162 | Formel (I-1) | Ar¹-62 | Ar¹-2 | 1142 | Formel (I-2) | Ar¹-62 | Ar¹-2 |
| 163 | Formel (I-1) | Ar¹-62 | Ar¹-3 | 1143 | Formel (I-2) | Ar¹-62 | Ar¹-3 |
| 164 | Formel (I-1) | Ar¹-62 | Ar¹-4 | 1144 | Formel (I-2) | Ar¹-62 | Ar¹-4 |
| 165 | Formel (I-1) | Ar¹-62 | Ar¹-59 | 1145 | Formel (I-2) | Ar¹-62 | Ar¹-59 |
| 166 | Formel (I-1) | Ar¹-62 | Ar¹-60 | 1146 | Formel (I-2) | Ar¹-62 | Ar¹-60 |
| 167 | Formel (I-1) | Ar¹-62 | Ar¹-63 | 1147 | Formel (I-2) | Ar¹-62 | Ar¹-63 |
| 168 | Formel (I-1) | Ar¹-62 | Ar¹-65 | 1148 | Formel (I-2) | Ar¹-62 | Ar¹-65 |
| 169 | Formel (I-1) | Ar¹-62 | Ar¹-68 | 1149 | Formel (I-2) | Ar¹-62 | Ar¹-68 |
| 170 | Formel (I-1) | Ar¹-62 | Ar¹-75 | 1150 | Formel (I-2) | Ar¹-62 | Ar¹-75 |
| 171 | Formel (I-1) | Ar¹-62 | Ar¹-87 | 1151 | Formel (I-2) | Ar¹-62 | Ar¹-87 |
| 172 | Formel (I-1) | Ar¹-62 | Ar¹-91 | 1152 | Formel (I-2) | Ar¹-62 | Ar¹-91 |
| 173 | Formel (I-1) | Ar¹-62 | Ar¹-107 | 1153 | Formel (I-2) | Ar¹-62 | Ar¹-107 |
| 174 | Formel (I-1) | Ar¹-62 | Ar¹-109 | 1154 | Formel (I-2) | Ar¹-62 | Ar¹-109 |
| 175 | Formel (I-1) | Ar¹-62 | Ar¹-111 | 1155 | Formel (I-2) | Ar¹-62 | Ar¹-111 |
| 176 | Formel (I-1) | Ar¹-62 | Ar¹-114 | 1156 | Formel (I-2) | Ar¹-62 | Ar¹-114 |
| 177 | Formel (I-1) | Ar¹-62 | Ar¹-121 | 1157 | Formel (I-2) | Ar¹-62 | Ar¹-121 |
| 178 | Formel (I-1) | Ar¹-62 | Ar¹-151 | 1158 | Formel (I-2) | Ar¹-62 | Ar¹-151 |
| 179 | Formel (I-1) | Ar¹-62 | Ar¹-153 | 1159 | Formel (I-2) | Ar¹-62 | Ar¹-153 |
| 180 | Formel (I-1) | Ar¹-62 | Ar¹-162 | 1160 | Formel (I-2) | Ar¹-62 | Ar¹-162 |
| 181 | Formel (I-1) | Ar¹-63 | Ar¹-1 | 1161 | Formel (I-2) | Ar¹-63 | Ar¹-1 |
| 182 | Formel (I-1) | Ar¹-63 | Ar¹-2 | 1162 | Formel (I-2) | Ar¹-63 | Ar¹-2 |
| 183 | Formel (I-1) | Ar¹-63 | Ar¹-3 | 1163 | Formel (I-2 | Ar¹-63 | Ar¹-3 |
| 184 | Formel (I-1) | Ar¹-63 | Ar¹-4 | 1164 | Formel (I-2 | Ar¹-63 | Ar¹-4 |
| 185 | Formel (I-1) | Ar¹-63 | Ar¹-59 | 1165 | Formel (I-2) | Ar¹-63 | Ar¹-59 |
| 186 | Formel (I-1) | Ar¹-63 | Ar¹-60 | 1166 | Formel (I-2) | Ar¹-63 | Ar¹-60 |
| 187 | Formel (I-1) | Ar¹-63 | Ar¹-63 | 1167 | Formel (I-2) | . Ar¹-63 | Ar¹-63 |
| 188 | Formel (I-1) | Ar¹-63 | Ar¹-65 | 1168 | Formel (I-2) | Ar¹-63 | Ar¹-65 |
| 189 | Formel (I-1) | Ar¹-63 | Ar¹-68 | 1169 | Formel (I-2) | Ar¹-63 | Ar¹-68 |
| 190 | Formel (I-1) | Ar¹-63 | Ar¹-75 | 1170 | Formel (I-2) | Ar¹-63 | Ar¹-75 |
| 191 | Formel (I-1) | Ar¹-63 | Ar¹-87 | 1171 | Formel (I-2) | Ar¹-63 | Ar¹-87 |
| 192 | Formel (I-1) | Ar¹-63 | Ar¹-91 | 1172 | Formel (I-2) | Ar¹-63 | Ar¹-91 |
| 193 | Formel (I-1) | Ar¹-63 | Ar¹-107 | 1173 | Formel (I-2) | Ar¹-63 | Ar¹-107 |
| 194 | Formel (I-1) | Ar¹-63 | Ar¹-109 | 1174 | Formel (I-2) | Ar¹-63 | Ar¹-109 |
| 195 | Formel (I-1) | Ar¹-63 | Ar¹-111 | 1175 | Formel (I-2) | Ar¹-63 | Ar¹-111 |
| 196 | Formel (I-1) | Ar¹-63 | Ar¹-114 | 1176 | Formel (I-2) | Ar¹-63 | Ar¹-114 |
| 197 | Formel (I-1) | Ar¹-63 | Ar¹-121 | 1177 | Formel (I-2) | Ar¹-63 | Ar¹-121 |
| 198 | Formel (I-1) | Ar¹-63 | Ar¹-151 | 1178 | Formel (I-2) | Ar¹-63 | Ar¹-151 |
| 199 | Formel (I-1) | Ar¹-63 | Ar¹-153 | 1179 | Formel (I-2) | Ar¹-63 | Ar¹-153 |
| 200 | Formel (I-1) | Ar¹-63 | Ar¹-162 | 1180 | Formel (I-2) | Ar¹-63 | Ar¹-162 |
| 201 | Formel (I-1) | Ar¹-64 | Ar¹-1 | 1181 | Formel (I-2) | Ar¹-64 | Ar¹-1 |
| 202 | Formel (I-1) | Ar¹-64 | Ar¹-2 | 1182 | Formel (I-2) | Ar¹-64 | Ar¹-2 |
| 203 | Formel (I-1) | Ar¹-64 | Ar¹-3 | 1183 | Formel (I-2) | Ar¹-64 | Ar¹-3 |
| 204 | Formel (I-1) | Ar¹-64 | Ar¹-4 | 1184 | Formel (I-2) | Ar¹-64 | Ar¹-4 |
| 205 | Formel (I-1) | Ar¹-64 | Ar¹-59 | 1185 | Formel (I-2) | Ar¹-64 | Ar¹-59 |
| 206 | Formel (I-1) | Ar¹-64 | Ar¹-60 | 1186 | Formel (I-2) | Ar¹-64 | Ar¹-60 |
| 207 | Formel (I-1) | Ar¹-64 | Ar¹-63 | 1187 | Formel (I-2) | Ar¹-64 | Ar¹-63 |
| 208 | Formel (I-1) | Ar¹-64 | Ar¹-65 | 1188 | Formel (I-2) | Ar¹-64 | Ar¹-65 |
| 209 | Formel (I-1) | Ar¹-64 | Ar¹-68 | 1189 | Formel (I-2) | Ar¹-64 | Ar¹-68 |
| 210 | Formel (I-1) | Ar¹-64 | Ar¹-75 | 1190 | Formel (I-2) | Ar¹-64 | Ar¹-75 |
| 211 | Formel (I-1) | Ar¹-64 | Ar¹-87 | 1191 | Formel (I-2) | Ar¹-64 | Ar¹-87 |
| 212 | Formel (I-1) | Ar¹-64 | Ar¹-91 | 1192 | Formel (I-2) | Ar¹-64 | Ar¹-91 |
| 213 | Formel (I-1) | Ar¹-64 | Ar¹-107 | 1193 | Formel (I-2) | Ar¹-64 | Ar¹-107 |
| 214 | Formel (I-1) | Ar¹-64 | Ar¹-109 | 1194 | Formel (I-2) | Ar¹-64 | Ar¹-109 |
| 215 | Formel (I-1) | Ar¹-64 | Ar¹-111 | 1195 | Formel (I-2) | Ar¹-64 | Ar¹-111 |
| 216 | Formel (I-1) | Ar¹-64 | Ar¹-114 | 1196 | Formel (I-2) | Ar¹-64 | Ar¹-114 |
| 217 | Formel (I-1) | Ar¹-64 | Ar¹-121 | 1197 | Formel (I-2) | Ar¹-64 | Ar¹-121 |
| 218 | Formel (I-1) | Ar¹-64 | Ar¹-151 | 1198 | Formel (I-2) | Ar¹-64 | Ar¹-151 |
| 219 | Formel (I-1) | Ar¹-64 | Ar¹-153 | 1199 | Formel (I-2) | Ar¹-64 | Ar¹-153 |
| 220 | Formel (I-1) | Ar¹-64 | Ar¹-162 | 1200 | Formel (I-2) | Ar¹-64 | Ar¹-162 |
| 221 | Formel (I-1) | Ar¹-65 | Ar¹-1 | 1201 | Formel (I-2) | Ar¹-65 | Ar¹-1 |
| 222 | Formel (I-1) | Ar¹-65 | Ar¹-2 | 1202 | Formel (I-2) | Ar¹-65 | Ar¹-2 |
| 223 | Formel (I-1) | Ar¹-65 | Ar¹-3 | 1203 | Formel (I-2) | Ar¹-65 | Ar¹-3 |
| 224 | Formel (I-1) | Ar¹-65 | Ar¹-4 | 1204 | Formel (I-2) | Ar¹-65 | Ar¹-4 |
| 225 | Formel (I-1) | Ar¹-65 | Ar¹-59 | 1205 | Formel (I-2) | Ar¹-65 | Ar¹-59 |
| 226 | Formel (I-1) | Ar¹-65 | Ar¹-60 | 1206 | Formel (I-2) | Ar¹-65 | Ar¹-60 |
| 227 | Formel (I-1) | Ar¹-65 | Ar¹-63 | 1207 | Formel (I-2) | Ar¹-65 | Ar¹-63 |
| 228 | Formel (I-1) | Ar¹-65 | Ar¹-65 | 1208 | Formel (I-2) | Ar¹-65 | Ar¹-65 |
| 229 | Formel (I-1) | Ar¹-65 | Ar¹-68 | 1209 | Formel (I-2) | Ar¹-65 | Ar¹-68 |
| 230 | Formel (I-1) | Ar¹-65 | Ar¹-75 | 1210 | Formel (I-2) | Ar¹-65 | Ar¹-75 |
| 231 | Formel (I-1) | Ar¹-65 | Ar¹-87 | 1211 | Formel (I-2) | Ar¹-65 | Ar¹-87 |
| 232 | Formel (I-1) | Ar¹-65 | Ar¹-91 | 1212 | Formel (I-2) | Ar¹-65 | Ar¹-91 |
| 233 | Formel (I-1) | Ar¹-65 | Ar¹-107 | 1213 | Formel (I-2) | Ar¹-65 | Ar¹-107 |
| 234 | Formel (I-1) | Ar¹-65 | Ar¹-109 | 1214 | Formel (I-2) | Ar¹-65 | Ar¹-109 |
| 235 | Formel (I-1) | Ar¹-65 | Ar¹-111 | 1215 | Formel (I-2) | Ar¹-65 | Ar¹-111 |
| 236 | Formel (I-1) | Ar¹-65 | Ar¹-114 | 1216 | Formel (I-2) | Ar¹-65 | Ar¹-114 |
| 237 | Formel (I-1) | Ar¹-65 | Ar¹-121 | 1217 | Formel (I-2) | Ar¹-65 | Ar¹-121 |
| 238 | Formel (I-1) | Ar¹-65 | Ar¹-151 | 1218 | Formel (I-2) | Ar¹-65 | Ar¹-151 |
| 239 | Formel (I-1) | Ar¹-65 | Ar¹-153 | 1219 | Formel (I-2) | Ar¹-65 | Ar¹-153 |
| 240 | Formel (I-1) | Ar¹-65 | Ar¹-162 | 1220 | Formel (I-2) | Ar¹-65 | Ar¹-162 |
| 241 | Formel (I-1) | Ar¹-66 | Ar¹-1 | 1221 | Formel (I-2) | Ar¹-66 | Ar¹-1 |
| 242 | Formel (I-1) | Ar¹-66 | Ar¹-2 | 1222 | Formel (I-2) | Ar¹-66 | Ar¹-2 |
| 243 | Formel (I-1) | Ar¹-66 | Ar¹-3 | 1223 | Formel (I-2) | Ar¹-66 | Ar¹-3 |
| 244 | Formel (I-1) | Ar¹-66 | Ar¹-4 | 1224 | Formel (I-2) | Ar¹-66 | Ar¹-4 |
| 245 | Formel (I-1) | Ar¹-66 | Ar¹-59 | 1225 | Formel (I-2) | Ar¹-66 | Ar¹-59 |
| 246 | Formel (I-1) | Ar¹-66 | Ar¹-60 | 1226 | Formel (I-2) | Ar¹-66 | Ar¹-60 |
| 247 | Formel (I-1) | Ar¹-66 | Ar¹-63 | 1227 | Formel (I-2) | Ar¹-66 | Ar¹-63 |
| 248 | Formel (I-1) | Ar¹-66 | Ar¹-65 | 1228 | Formel (I-2) | Ar¹-66 | Ar¹-65 |
| 249 | Formel (I-1) | Ar¹-66 | Ar¹-68 | 1229 | Formel (I-2 | Ar¹-66 | Ar¹-68 |
| 250 | Formel (I-1) | Ar¹-66 | Ar¹-75 | 1230 | Formel (I-2) | Ar¹-66 | Ar¹-75 |
| 251 | Formel (I-1) | Ar¹-66 | Ar¹-87 | 1231 | Formel (I-2) | Ar¹-66 | Ar¹-87 |
| 252 | Formel (I-1) | Ar¹-66 | Ar¹-91 | 1232 | Formel (I-2) | Ar¹-66 | Ar¹-91 |
| 253 | Formel (I-1) | Ar¹-66 | Ar¹-107 | 1233 | Formel (I-2) | Ar¹-66 | Ar¹-107 |
| 254 | Formel (I-1) | Ar¹-66 | Ar¹-109 | 1234 | Formel (I-2 | Ar¹-66 | Ar¹-109 |
| 255 | Formel (I-1) | Ar¹-66 | Ar¹-111 | 1235 | Formel (I-2) | Ar¹-66 | Ar¹-111 |
| 256 | Formel (I-1) | Ar¹-66 | Ar¹-114 | 1236 | Formel (I-2) | Ar¹-66 | Ar¹-114 |
| 257 | Formel (I-1) | Ar¹-66 | Ar¹-121 | 1237 | Formel (I-2) | Ar¹-66 | Ar¹-121 |
| 258 | Formel (I-1) | Ar¹-66 | Ar¹-151 | 1238 | Formel (I-2 | Ar¹-66 | Ar¹-151 |
| 259 | Formel (I-1) | Ar¹-66 | Ar¹-153 | 1239 | Formel (I-2) | Ar¹-66 | Ar¹-153 |
| 260 | Formel (I-1) | Ar¹-66 | Ar¹-162 | 1240 | Formel (I-2) | Ar¹-66 | Ar¹-162 |
| 261 | Formel (I-1) | Ar¹-68 | Ar¹-1 | 1241 | Formel (I-2) | Ar¹-68 | Ar¹-1 |
| 262 | Formel (I-1) | Ar¹-68 | Ar¹-2 | 1242 | Formel (I-2) | Ar¹-68 | Ar¹-2 |
| 263 | Formel (I-1) | Ar¹-68 | Ar¹-3 | 1243 | Formel (I-2) | Ar¹-68 | Ar¹-3 |
| 264 | Formel (I-1) | Ar¹-68 | Ar¹-4 | 1244 | Formel (I-2) | Ar¹-68 | Ar¹-4 |
| 265 | Formel (I-1) | Ar¹-68 | Ar¹-59 | 1245 | Formel (I-2) | Ar¹-68 | Ar¹-59 |
| 266 | Formel (I-1) | Ar¹-68 | Ar¹-60 | 1246 | Formel (I-2) | Ar¹-68 | Ar¹-60 |
| 267 | Formel (I-1) | Ar¹-68 | Ar¹-63 | 1247 | Formel (I-2) | Ar¹-68 | Ar¹-63 |
| 268 | Formel (I-1) | Ar¹-68 | Ar¹-65 | 1248 | Formel (I-2) | Ar¹-68 | Ar¹-65 |
| 269 | Formel (I-1) | Ar¹-68 | Ar¹-68 | 1249 | Formel (I-2) | Ar¹-68 | Ar¹-68 |
| 270 | Formel (I-1) | Ar¹-68 | Ar¹-75 | 1250 | Formel (I-2) | Ar¹-68 | Ar¹-75 |
| 271 | Formel (I-1) | Ar¹-68 | Ar¹-87 | 1251 | Formel (I-2) | Ar¹-68 | Ar¹-87 |
| 272 | Formel (I-1) | Ar¹-68 | Ar¹-91 | 1252 | Formel (I-2) | Ar¹-68 | Ar¹-91 |
| 273 | Formel (I-1) | Ar¹-68 | Ar¹-107 | 1253 | Formel (I-2) | Ar¹-68 | Ar¹-107 |
| 274 | Formel (I-1) | Ar¹-68 | Ar¹-109 | 1254 | Formel (I-2) | Ar¹-68 | Ar¹-109 |
| 275 | Formel (I-1) | Ar¹-68 | Ar¹-111 | 1255 | Formel (I-2) | Ar¹-68 | Ar¹-111 |
| 276 | Formel (I-1) | Ar¹-68 | Ar¹-114 | 1256 | Formel (I-2) | Ar¹-68 | Ar¹-114 |
| 277 | Formel (I-1) | Ar¹-68 | Ar¹-121 | 1257 | Formel (I-2) | Ar¹-68 | Ar¹-121 |
| 278 | Formel (I-1) | Ar¹-68 | Ar¹-151 | 1258 | Formel (I-2) | Ar¹-68 | Ar¹-151 |
| 279 | Formel (I-1) | Ar¹-68 | Ar¹-153 | 1259 | Formel (I-2) | Ar¹-68 | Ar¹-153 |
| 280 | Formel (I-1) | Ar¹-68 | Ar¹-162 | 1260 | Formel (I-2) | Ar¹-68 | Ar¹-162 |
| 281 | Formel (I-1) | Ar¹-71 | Ar¹-1 | 1261 | Formel (I-2) | Ar¹-71 | Ar¹-1 |
| 282 | Formel (I-1) | Ar¹-71 | Ar¹-2 | 1262 | Formel (I-2) | Ar¹-71 | Ar¹-2 |
| 283 | Formel (I-1) | Ar¹-71 | Ar¹-3 | 1263 | Formel (I-2) | Ar¹-71 | Ar¹-3 |
| 284 | Formel (I-1) | Ar¹-71 | Ar¹-4 | 1264 | Formel (I-2) | Ar¹-71 | Ar¹-4 |
| 285 | Formel (I-1) | Ar¹-71 | Ar¹-59 | 1265 | Formel (I-2) | Ar¹-71 | Ar¹-59 |
| 286 | Formel (I-1) | Ar¹-71 | Ar¹-60 | 1266 | Formel (I-2) | Ar¹-71 | Ar¹-60 |
| 287 | Formel (I-1) | Ar¹-71 | Ar¹-63 | 1267 | Formel (I-2) | Ar¹-71 | Ar¹-63 |
| 288 | Formel (I-1) | Ar¹-71 | Ar¹-65 | 1268 | Formel (I-2) | Ar¹-71 | Ar¹-65 |
| 289 | Formel (I-1) | Ar¹-71 | Ar¹-68 | 1269 | Formel (I-2) | Ar¹-71 | Ar¹-68 |
| 290 | Formel (I-1) | Ar¹-71 | Ar¹-75 | 1270 | Formel (I-2) | Ar¹-71 | Ar¹-75 |
| 291 | Formel (I-1) | Ar¹-71 | Ar¹-87 | 1271 | Formel (I-2) | Ar¹-71 | Ar¹-87 |
| 292 | Formel (I-1) | Ar¹-71 | Ar¹-91 | 1272 | Formel (I-2) | Ar¹-71 | Ar¹-91 |
| 293 | Formel (I-1) | Ar¹-71 | Ar¹-107 | 1273 | Formel (I-2) | Ar¹-71 | Ar¹-107 |
| 294 | Formel (I-1) | Ar¹-71 | Ar¹-109 | 1274 | Formel (I-2) | Ar¹-71 | Ar¹-109 |
| 295 | Formel (I-1) | Ar¹-71 | Ar¹-111 | 1275 | Formel (I-2) | Ar¹-71 | Ar¹-111 |
| 296 | Formel (I-1) | Ar¹-71 | Ar¹-114 | 1276 | Formel (I-2) | Ar¹-71 | Ar¹-114 |
| 297 | Formel (I-1) | Ar¹-71 | Ar¹-121 | 1277 | Formel (I-2) | Ar¹-71 | Ar¹-121 |
| 298 | Formel (I-1) | Ar¹-71 | Ar¹-151 | 1278 | Formel (I-2) | Ar¹-71 | Ar¹-151 |
| 299 | Formel (I-1) | Ar¹-71 | Ar¹-153 | 1279 | Formel (I-2) | Ar¹-71 | Ar¹-153 |
| 300 | Formel (I-1) | Ar¹-71 | Ar¹-162 | 1280 | Formel (I-2) | Ar¹-71 | Ar¹-162 |
| 301 | Formel (I-1) | Ar¹-75 | Ar¹-1 | 1281 | Formel (I-2) | Ar¹-75 | Ar¹-1 |
| 302 | Formel (I-1) | Ar¹-75 | Ar¹-2 | 1282 | Formel (I-2) | Ar¹-75 | Ar¹-2 |
| 303 | Formel (I-1) | Ar¹-75 | Ar¹-3 | 1283 | Formel (I-2) | Ar¹-75 | Ar¹-3 |
| 304 | Formel (I-1) | Ar¹-75 | Ar¹-4 | 1284 | Formel (I-2) | Ar¹-75 | Ar¹-4 |
| 305 | Formel (I-1) | Ar¹-75 | Ar¹-59 | 1285 | Formel (I-2) | Ar¹-75 | Ar¹-59 |
| 306 | Formel (I-1) | Ar¹-75 | Ar¹-60 | 1286 | Formel (I-2) | Ar¹-75 | Ar¹-60 |
| 307 | Formel (I-1) | Ar¹-75 | Ar¹-63 | 1287 | Formel (I-2) | Ar¹-75 | Ar¹-63 |
| 308 | Formel (I-1) | Ar¹-75 | Ar¹-65 | 1288 | Formel (I-2) | Ar¹-75 | Ar¹-65 |
| 309 | Formel (I-1) | Ar¹-75 | Ar¹-68 | 1289 | Formel (I-2) | Ar¹-75 | Ar¹-68 |
| 310 | Formel (I-1) | Ar¹-75 | Ar¹-75 | 1290 | Formel (I-2) | Ar¹-75 | Ar¹-75 |
| 311 | Formel (I-1) | Ar¹-75 | Ar¹-87 | 1291 | Formel (I-2) | Ar¹-75 | Ar¹-87 |
| 312 | Formel (I-1) | Ar¹-75 | Ar¹-91 | 1292 | Formel (I-2) | Ar¹-75 | Ar¹-91 |
| 313 | Formel (I-1) | Ar¹-75 | Ar¹-107 | 1293 | Formel (I-2) | Ar¹-75 | Ar¹-107 |
| 314 | Formel (I-1) | Ar¹-75 | Ar¹-109 | 1294 | Formel (I-2) | Ar¹-75 | Ar¹-109 |
| 315 | Formel (I-1) | Ar¹-75 | Ar¹-111 | 1295 | Formel (I-2) | Ar¹-75 | Ar¹-111 |
| 316 | Formel (I-1) | Ar¹-75 | Ar¹-114 | 1296 | Formel (I-2) | Ar¹-75 | Ar¹-114 |
| 317 | Formel (I-1) | Ar¹-75 | Ar¹-121 | 1297 | Formel (I-2) | Ar¹-75 | Ar¹-121 |
| 318 | Formel (I-1) | Ar¹-75 | Ar¹-151 | 1298 | Formel (I-2) | Ar¹-75 | Ar¹-151 |
| 319 | Formel (I-1) | Ar¹-75 | Ar¹-153 | 1299 | Formel (I-2) | Ar¹-75 | Ar¹-153 |
| 320 | Formel (I-1) | Ar¹-75 | Ar¹-162 | 1300 | Formel (I-2) | Ar¹-75 | Ar¹-162 |
| 321 | Formel (I-1) | Ar¹-76 | Ar¹-1 | 1301 | Formel (I-2) | Ar¹-76 | Ar¹-1 |
| 322 | Formel (I-1) | Ar¹-76 | Ar¹-2 | 1302 | Formel (I-2) | Ar¹-76 | Ar¹-2 |
| 323 | Formel (I-1) | Ar¹-76 | Ar¹-3 | 1303 | Formel (I-2) | Ar¹-76 | Ar¹-3 |
| 324 | Formel (I-1) | Ar¹-76 | Ar¹-4 | 1304 | Formel (I-2) | Ar¹-76 | Ar¹-4 |
| 325 | Formel (I-1) | Ar¹-76 | Ar¹-59 | 1305 | Formel (I-2) | Ar¹-76 | Ar¹-59 |
| 326 | Formel (I-1) | Ar¹-76 | Ar¹-60 | 1306 | Formel (I-2) | Ar¹-76 | Ar¹-60 |
| 327 | Formel (I-1) | Ar¹-76 | Ar¹-63 | 1307 | Formel (I-2) | Ar¹-76 | Ar¹-63 |
| 328 | Formel (I-1) | Ar¹-76 | Ar¹-65 | 1308 | Formel (I-2) | Ar¹-76 | Ar¹-65 |
| 329 | Formel (I-1) | Ar¹-76 | Ar¹-68 | 1309 | Formel (I-2) | Ar¹-76 | Ar¹-68 |
| 330 | Formel (I-1) | Ar¹-76 | Ar¹-75 | 1310 | Formel (I-2) | Ar¹-76 | Ar¹-75 |
| 331 | Formel (I-1) | Ar¹-76 | Ar¹-87 | 1311 | Formel (I-2) | Ar¹-76 | Ar¹-87 |
| 332 | Formel (I-1) | Ar¹-76 | Ar¹-91 | 1312 | Formel (I-2) | Ar¹-76 | Ar¹-91 |
| 333 | Formel (I-1) | Ar¹-76 | Ar¹-107 | 1313 | Formel (I-2) | Ar¹-76 | Ar¹-107 |
| 334 | Formel (I-1) | Ar¹-76 | Ar¹-109 | 1314 | Formel (I-2) | Ar¹-76 | Ar¹-109 |
| 335 | Formel (I-1) | Ar¹-76 | Ar¹-111 | 1315 | Formel (I-2) | Ar¹-76 | Ar¹-111 |
| 336 | Formel (I-1) | Ar¹-76 | Ar¹-114 | 1316 | Formel (I-2) | Ar¹-76 | Ar¹-114 |
| 337 | Formel (I-1) | Ar¹-76 | Ar¹-121 | 1317 | Formel (I-2) | Ar¹-76 | Ar¹-121 |
| 338 | Formel (I-1) | Ar¹-76 | Ar¹-151 | 1318 | Formel (I-2) | Ar¹-76 | Ar¹-151 |
| 339 | Formel (I-1) | Ar¹-76 | Ar¹-153 | 1319 | Formel (I-2) | Ar¹-76 | Ar¹-153 |
| 340 | Formel (I-1) | Ar¹-76 | Ar¹-162 | 1320 | Formel (I-2) | Ar¹-76 | Ar¹-162 |
| 341 | Formel (I-1) | Ar¹-79 | Ar¹-1 | 1321 | Formel (I-2) | Ar¹-79 | Ar¹-1 |
| 342 | Formel (I-1) | Ar¹-79 | Ar¹-2 | 1322 | Formel (I-2) | Ar¹-79 | Ar¹-2 |
| 343 | Formel (I-1) | Ar¹-79 | Ar¹-3 | 1323 | Formel (I-2) | Ar¹-79 | Ar¹-3 |
| 344 | Formel (I-1) | Ar¹-79 | Ar¹-4 | 1324 | Formel (I-2) | Ar¹-79 | Ar¹-4 |
| 345 | Formel (I-1) | Ar¹-79 | Ar¹-59 | 1325 | Formel (I-2) | Ar¹-79 | Ar¹-59 |
| 346 | Formel (I-1) | Ar¹-79 | Ar¹-60 | 1326 | Formel (I-2) | Ar¹-79 | Ar¹-60 |
| 347 | Formel (I-1) | Ar¹-79 | Ar¹-63 | 1327 | Formel (I-2) | Ar¹-79 | Ar¹-63 |
| 348 | Formel (I-1) | Ar¹-79 | Ar¹-65 | 1328 | Formel (I-2) | Ar¹-79 | Ar¹-65 |
| 349 | Formel (I-1) | Ar¹-79 | Ar¹-68 | 1329 | Formel (I-2) | Ar¹-79 | Ar¹-68 |
| 350 | Formel (I-1) | Ar¹-79 | Ar¹-75 | 1330 | Formel (I-2) | Ar¹-79 | Ar¹-75 |
| 351 | Formel (I-1) | Ar¹-79 | Ar¹-87 | 1331 | Formel (I-2) | Ar¹-79 | Ar¹-87 |
| 352 | Formel (I-1) | Ar¹-79 | Ar¹-91 | 1332 | Formel (I-2) | Ar¹-79 | Ar¹-91 |
| 353 | Formel (I-1) | Ar¹-79 | Ar¹-107 | 1333 | Formel (I-2) | Ar¹-79 | Ar¹-107 |
| 354 | Formel (I-1) | Ar¹-79 | Ar¹-109 | 1334 | Formel (I-2) | Ar¹-79 | Ar¹-109 |
| 355 | Formel (I-1) | Ar¹-79 | Ar¹-111 | 1335 | Formel (I-2) | Ar¹-79 | Ar¹-111 |
| 356 | Formel (I-1) | Ar¹-79 | Ar¹-114 | 1336 | Formel (I-2) | Ar¹-79 | Ar¹-114 |
| 357 | Formel (I-1) | Ar¹-79 | Ar¹-121 | 1337 | Formel (I-2) | Ar¹-79 | Ar¹-121 |
| 358 | Formel (I-1) | Ar¹-79 | Ar¹-151 | 1338 | Formel (I-2) | Ar¹-79 | Ar¹-151 |
| 359 | Formel (I-1) | Ar¹-79 | Ar¹-153 | 1339 | Formel (I-2) | Ar¹-79 | Ar¹-153 |
| 360 | Formel (I-1) | Ar¹-79 | Ar¹-162 | 1340 | Formel (I-2) | Ar¹-79 | Ar¹-162 |
| 361 | Formel (I-1) | Ar¹-87 | Ar¹-1 | 1341 | Formel (I-2) | Ar¹-87 | Ar¹-1 |
| 362 | Formel (I-1) | Ar¹-87 | Ar¹-2 | 1342 | Formel (I-2) | Ar¹-87 | Ar¹-2 |
| 363 | Formel (I-1) | Ar¹-87 | Ar¹-3 | 1343 | Formel (I-2) | Ar¹-87 | Ar¹-3 |
| 364 | Formel (I-1) | Ar¹-87 | Ar¹-4 | 1344 | Formel (I-2) | Ar¹-87 | Ar¹-4 |
| 365 | Formel (I-1) | Ar¹-87 | Ar¹-59 | 1345 | Formel (I-2) | Ar¹-87 | Ar¹-59 |
| 366 | Formel (I-1) | Ar¹-87 | Ar¹-60 | 1346 | Formel (I-2) | Ar¹-87 | Ar¹-60 |
| 367 | Formel (I-1) | Ar¹-87 | Ar¹-63 | 1347 | Formel (I-2) | Ar¹-87 | Ar¹-63 |
| 368 | Formel (I-1) | Ar¹-87 | Ar¹-65 | 1348 | Formel (I-2) | Ar¹-87 | Ar¹-65 |
| 369 | Formel (I-1) | Ar¹-87 | Ar¹-68 | 1349 | Formel (I-2) | Ar¹-87 | Ar¹-68 |
| 370 | Formel (I-1) | Ar¹-87 | Ar¹-75 | 1350 | Formel (I-2) | Ar¹-87 | Ar¹-75 |
| 371 | Formel (I-1) | Ar¹-87 | Ar¹-87 | 1351 | Formel (I-2) | Ar¹-87 | Ar¹-87 |
| 372 | Formel (I-1) | Ar¹-87 | Ar¹-91 | 1352 | Formel (I-2) | Ar¹-87 | Ar¹-91 |
| 373 | Formel (I-1) | Ar¹-87 | Ar¹-107 | 1353 | Formel (I-2) | Ar¹-87 | Ar¹-107 |
| 374 | Formel (I-1) | Ar¹-87 | Ar¹-109 | 1354 | Formel (I-2) | Ar¹-87 | Ar¹-109 |
| 375 | Formel (I-1) | Ar¹-87 | Ar¹-111 | 1355 | Formel (I-2) | Ar¹-87 | Ar¹-111 |
| 376 | Formel (I-1) | Ar¹-87 | Ar¹-114 | 1356 | Formel (I-2) | Ar¹-87 | Ar¹-114 |
| 377 | Formel (I-1) | Ar¹-87 | Ar¹-121 | 1357 | Formel (I-2) | Ar¹-87 | Ar¹-121 |
| 378 | Formel (I-1) | Ar¹-87 | Ar¹-151 | 1358 | Formel (I-2) | Ar¹-87 | Ar¹-151 |
| 379 | Formel (I-1) | Ar¹-87 | Ar¹-153 | 1359 | Formel (I-2) | Ar¹-87 | Ar¹-153 |
| 380 | Formel (I-1) | Ar¹-87 | Ar¹-162 | 1360 | Formel (I-2) | Ar¹-87 | Ar¹-162 |
| 381 | Formel (I-1) | Ar¹-91 | Ar¹-1 | 1361 | Formel (I-2) | Ar¹-91 | Ar¹-1 |
| 382 | Formel (I-1) | Ar¹-91 | Ar¹-2 | 1362 | Formel (I-2) | Ar¹-91 | Ar¹-2 |
| 383 | Formel (I-1) | Ar¹-91 | Ar¹-3 | 1363 | Formel (I-2) | Ar¹-91 | Ar¹-3 |
| 384 | Formel (I-1) | Ar¹-91 | Ar¹-4 | 1364 | Formel (I-2) | Ar¹-91 | Ar¹-4 |
| 385 | Formel (I-1) | Ar¹-91 | Ar¹-59 | 1365 | Formel (I-2) | Ar¹-91 | Ar¹-59 |
| 386 | Formel (I-1) | Ar¹-91 | Ar¹-60 | 1366 | Formel (I-2) | Ar¹-91 | Ar¹-60 |
| 387 | Formel (I-1) | Ar¹-91 | Ar¹-63 | 1367 | Formel (I-2) | Ar¹-91 | Ar¹-63 |
| 388 | Formel (I-1) | Ar¹-91 | Ar¹-65 | 1368 | Formel (I-2) | Ar¹-91 | Ar¹-65 |
| 389 | Formel (I-1) | Ar¹-91 | Ar¹-68 | 1369 | Formel (I-2) | Ar¹-91 | Ar¹-68 |
| 390 | Formel (I-1) | Ar¹-91 | Ar¹-75 | 1370 | Formel (I-2) | Ar¹-91 | Ar¹-75 |
| 391 | Formel (I-1) | Ar¹-91 | Ar¹-87 | 1371 | Formel (I-2) | Ar¹-91 | Ar¹-87 |
| 392 | Formel (I-1) | Ar¹-91 | Ar¹-91 | 1372 | Formel (I-2) | Ar¹-91 | Ar¹-91 |
| 393 | Formel (I-1) | Ar¹-91 | Ar¹-107 | 1373 | Formel (I-2) | Ar¹-91 | Ar¹-107 |
| 394 | Formel (I-1) | Ar¹-91 | Ar¹-109 | 1374 | Formel (I-2) | Ar¹-91 | Ar¹-109 |
| 395 | Formel (I-1) | Ar¹-91 | Ar¹-111 | 1375 | Formel (I-2) | Ar¹-91 | Ar¹-111 |
| 396 | Formel (I-1) | Ar¹-91 | Ar¹-114 | 1376 | Formel (I-2) | Ar¹-91 | Ar¹-114 |
| 397 | Formel (I-1) | Ar¹-91 | Ar¹-121 | 1377 | Formel (I-2) | Ar¹-91 | Ar¹-121 |
| 398 | Formel (I-1) | Ar¹-91 | Ar¹-151 | 1378 | Formel (I-2) | Ar¹-91 | Ar¹-151 |
| 399 | Formel (I-1) | Ar¹-91 | Ar¹-153 | 1379 | Formel (I-2) | Ar¹-91 | Ar¹-153 |
| 400 | Formel (I-1) | Ar¹-91 | Ar¹-162 | 1380 | Formel (I-2) | Ar¹-91 | Ar¹-162 |
| 401 | Formel (I-1) | Ar¹-92 | Ar¹-1 | 1381 | Formel (I-2) | Ar¹-92 | Ar¹-1 |
| 402 | Formel (I-1) | Ar¹-92 | Ar¹-2 | 1382 | Formel (I-2) | Ar¹-92 | Ar¹-2 |
| 403 | Formel (I-1) | Ar¹-92 | Ar¹-3 | 1383 | Formel (I-2) | Ar¹-92 | Ar¹-3 |
| 404 | Formel (I-1) | Ar¹-92 | Ar¹-4 | 1384 | Formel (I-2) | Ar¹-92 | Ar¹-4 |
| 405 | Formel (I-1) | Ar¹-92 | Ar¹-59 | 1385 | Formel (I-2) | Ar¹-92 | Ar¹-59 |
| 406 | Formel (I-1) | Ar¹-92 | Ar¹-60 | 1386 | Formel (I-2) | Ar¹-92 | Ar¹-60 |
| 407 | Formel (I-1) | Ar¹-92 | Ar¹-63 | 1387 | Formel (I-2) | Ar¹-92 | Ar¹-63 |
| 408 | Formel (I-1) | Ar¹-92 | Ar¹-65 | 1388 | Formel (I-2) | Ar¹-92 | Ar¹-65 |
| 409 | Formel (I-1) | Ar¹-92 | Ar¹-68 | 1389 | Formel (I-2) | Ar¹-92 | Ar¹-68 |
| 410 | Formel (I-1) | Ar¹-92 | Ar¹-75 | 1390 | Formel (I-2) | Ar¹-92 | Ar¹-75 |
| 411 | Formel (I-1) | Ar¹-92 | Ar¹-87 | 1391 | Formel (I-2) | Ar¹-92 | Ar¹-87 |
| 412 | Formel (I-1) | Ar¹-92 | Ar¹-91 | 1392 | Formel (I-2) | Ar¹-92 | Ar¹-91 |
| 413 | Formel (I-1) | Ar¹-92 | Ar¹-107 | 1393 | Formel (I-2) | Ar¹-92 | Ar¹-107 |
| 414 | Formel (I-1) | Ar¹-92 | Ar¹-109 | 1394 | Formel (I-2) | Ar¹-92 | Ar¹-109 |
| 415 | Formel (I-1) | Ar¹-92 | Ar¹-111 | 1395 | Formel (I-2) | Ar¹-92 | Ar¹-111 |
| 416 | Formel (I-1) | Ar¹-92 | Ar¹-114 | 1396 | Formel (I-2) | Ar¹-92 | Ar¹-114 |
| 417 | Formel (I-1) | Ar¹-92 | Ar¹-121 | 1397 | Formel (I-2) | Ar¹-92 | Ar¹-121 |
| 418 | Formel (I-1) | Ar¹-92 | Ar¹-151 | 1398 | Formel (I-2) | Ar¹-92 | Ar¹-151 |
| 419 | Formel (I-1) | Ar¹-92 | Ar¹-153 | 1399 | Formel (I-2) | Ar¹-92 | Ar¹-153 |
| 420 | Formel (I-1) | Ar¹-92 | Ar¹-162 | 1400 | Formel (I-2) | Ar¹-92 | Ar¹-162 |
| 421 | Formel (I-1) | Ar¹-95 | Ar¹-1 | 1401 | Formel (I-2) | Ar¹-95 | Ar¹-1 |
| 422 | Formel (I-1) | Ar¹-95 | Ar¹-2 | 1402 | Formel (I-2) | Ar¹-95 | Ar¹-2 |
| 423 | Formel (I-1) | Ar¹-95 | Ar¹-3 | 1403 | Formel (I-2) | Ar¹-95 | Ar¹-3 |
| 424 | Formel (I-1) | Ar¹-95 | Ar¹-4 | 1404 | Formel (I-2) | Ar¹-95 | Ar¹-4 |
| 425 | Formel (I-1) | Ar¹-95 | Ar¹-59 | 1405 | Formel (I-2) | Ar¹-95 | Ar¹-59 |
| 426 | Formel (I-1) | Ar¹-95 | Ar¹-60 | 1406 | Formel (I-2) | Ar¹-95 | Ar¹-60 |
| 427 | Formel (I-1) | Ar¹-95 | Ar¹-63 | 1407 | Formel (I-2) | Ar¹-95 | Ar¹-63 |
| 428 | Formel (I-1) | Ar¹-95 | Ar¹-65 | 1408 | Formel (I-2) | Ar¹-95 | Ar¹-65 |
| 429 | Formel (I-1) | Ar¹-95 | Ar¹-68 | 1409 | Formel (I-2) | Ar¹-95 | Ar¹-68 |
| 430 | Formel (I-1) | Ar¹-95 | Ar¹-75 | 1410 | Formel (I-2) | Ar¹-95 | Ar¹-75 |
| 431 | Formel (I-1) | Ar¹-95 | Ar¹-87 | 1411 | Formel (I-2) | Ar¹-95 | Ar¹-87 |
| 432 | Formel (I-1) | Ar¹-95 | Ar¹-91 | 1412 | Formel (I-2) | Ar¹-95 | Ar¹-91 |
| 433 | Formel (I-1) | Ar¹-95 | Ar¹-107 | 1413 | Formel (I-2) | Ar¹-95 | Ar¹-107 |
| 434 | Formel (I-1) | Ar¹-95 | Ar¹-109 | 1414 | Formel (I-2) | Ar¹-95 | Ar¹-109 |
| 435 | Formel (I-1) | Ar¹-95 | Ar¹-111 | 1415 | Formel (I-2) | Ar¹-95 | Ar¹-111 |
| 436 | Formel (I-1) | Ar¹-95 | Ar¹-114 | 1416 | Formel (I-2) | Ar¹-95 | Ar¹-114 |
| 437 | Formel (I-1) | Ar¹-95 | Ar¹-121 | 1417 | Formel (I-2) | Ar¹-95 | Ar¹-121 |
| 438 | Formel (I-1) | Ar¹-95 | Ar¹-151 | 1418 | Formel (I-2) | Ar¹-95 | Ar¹-151 |
| 439 | Formel (I-1) | Ar¹-95 | Ar¹-153 | 1419 | Formel (I-2) | Ar¹-95 | Ar¹-153 |
| 440 | Formel (I-1) | Ar¹-95 | Ar¹-162 | 1420 | Formel (I-2) | Ar¹-95 | Ar¹-162 |
| 441 | Formel (I-1) | Ar¹-99 | Ar¹-1 | 1421 | Formel (I-2) | Ar¹-99 | Ar¹-1 |
| 442 | Formel (I-1) | Ar¹-99 | Ar¹-2 | 1422 | Formel (I-2) | Ar¹-99 | Ar¹-2 |
| 443 | Formel (I-1) | Ar¹-99 | Ar¹-3 | 1423 | Formel (I-2) | Ar¹-99 | Ar¹-3 |
| 444 | Formel (I-1) | Ar¹-99 | Ar¹-4 | 1424 | Formel (I-2) | Ar¹-99 | Ar¹-4 |
| 445 | Formel (I-1) | Ar¹-99 | Ar¹-59 | 1425 | Formel (I-2) | Ar¹-99 | Ar¹-59 |
| 446 | Formel (I-1) | Ar¹-99 | Ar¹-60 | 1426 | Formel (I-2) | Ar¹-99 | Ar¹-60 |
| 447 | Formel (I-1) | Ar¹-99 | Ar¹-63 | 1427 | Formel (I-2) | Ar¹-99 | Ar¹-63 |
| 448 | Formel (I-1) | Ar¹-99 | Ar¹-65 | 1428 | Formel (I-2) | Ar¹-99 | Ar¹-65 |
| 449 | Formel (I-1) | Ar¹-99 | Ar¹-68 | 1429 | Formel (I-2) | Ar¹-99 | Ar¹-68 |
| 450 | Formel (I-1) | Ar¹-99 | Ar¹-75 | 1430 | Formel (I-2) | Ar¹-99 | Ar¹-75 |
| 451 | Formel (I-1) | Ar¹-99 | Ar¹-87 | 1431 | Formel (I-2) | Ar¹-99 | Ar¹-87 |
| 452 | Formel (I-1) | Ar¹-99 | Ar¹-91 | 1432 | Formel (I-2) | Ar¹-99 | Ar¹-91 |
| 453 | Formel (I-1) | Ar¹-99 | Ar¹-107 | 1433 | Formel (I-2) | Ar¹-99 | Ar¹-107 |
| 454 | Formel (I-1) | Ar¹-99 | Ar¹-109 | 1434 | Formel (I-2) | Ar¹-99 | Ar¹-109 |
| 455 | Formel (I-1) | Ar¹-99 | Ar¹-111 | 1435 | Formel (I-2) | Ar¹-99 | Ar¹-111 |
| 456 | Formel (I-1) | Ar¹-99 | Ar¹-114 | 1436 | Formel (I-2) | Ar¹-99 | Ar¹-114 |
| 457 | Formel (I-1) | Ar¹-99 | Ar¹-121 | 1437 | Formel (I-2) | Ar¹-99 | Ar¹-121 |
| 458 | Formel (I-1) | Ar¹-99 | Ar¹-151 | 1438 | Formel (I-2) | Ar¹-99 | Ar¹-151 |
| 459 | Formel (I-1) | Ar¹-99 | Ar¹-153 | 1439 | Formel (I-2) | Ar¹-99 | Ar¹-153 |
| 460 | Formel (I-1) | Ar¹-99 | Ar¹-162 | 1440 | Formel (I-2) | Ar¹-99 | Ar¹-162 |
| 461 | Formel (I-1) | Ar¹-107 | Ar¹-1 | 1441 | Formel (I-2) | Ar¹-107 | Ar¹-1 |
| 462 | Formel (I-1) | Ar¹-107 | Ar¹-2 | 1442 | Formel (I-2) | Ar¹-107 | Ar¹-2 |
| 463 | Formel (I-1) | Ar¹-107 | Ar¹-3 | 1443 | Formel (I-2) | Ar¹-107 | Ar¹-3 |
| 464 | Formel (I-1) | Ar¹-107 | Ar¹-4 | 1444 | Formel (I-2) | Ar¹-107 | Ar¹-4 |
| 465 | Formel (I-1) | Ar¹-107 | Ar¹-59 | 1445 | Formel (I-2) | Ar¹-107 | Ar¹-59 |
| 466 | Formel (I-1) | Ar¹-107 | Ar¹-60 | 1446 | Formel (I-2) | Ar¹-107 | Ar¹-60 |
| 467 | Formel (I-1) | Ar¹-107 | Ar¹-63 | 1447 | Formel (I-2) | Ar¹-107 | Ar¹-63 |
| 468 | Formel (I-1) | Ar¹-107 | Ar¹-65 | 1448 | Formel (I-2) | Ar¹-107 | Ar¹-65 |
| 469 | Formel (I-1) | Ar¹-107 | Ar¹-68 | 1449 | Formel (I-2) | Ar¹-107 | Ar¹-68 |
| 470 | Formel (I-1) | Ar¹-107 | Ar¹-75 | 1450 | Formel (I-2) | Ar¹-107 | Ar¹-75 |
| 471 | Formel (I-1) | Ar¹-107 | Ar¹-87 | 1451 | Formel (I-2) | Ar¹-107 | Ar¹-87 |
| 472 | Formel (I-1) | Ar¹-107 | Ar¹-91 | 1452 | Formel (I-2) | Ar¹-107 | Ar¹-91 |
| 473 | Formel (I-1) | Ar¹-107 | Ar¹-107 | 1453 | Formel (I-2) | Ar¹-107 | Ar¹-107 |
| 474 | Formel (I-1) | Ar¹-107 | Ar¹-109 | 1454 | Formel (I-2) | Ar¹-107 | Ar¹-109 |
| 475 | Formel (I-1) | Ar¹-107 | Ar¹-111 | 1455 | Formel (I-2) | Ar¹-107 | Ar¹-111 |
| 476 | Formel (I-1) | Ar¹-107 | Ar¹-114 | 1456 | Formel (I-2) | Ar¹-107 | Ar¹-114 |
| 477 | Formel (I-1) | Ar¹-107 | Ar¹-121 | 1457 | Formel (I-2) | Ar¹-107 | Ar¹-121 |
| 478 | Formel (I-1) | Ar¹-107 | Ar¹-151 | 1458 | Formel (I-2) | Ar¹-107 | Ar¹-151 |
| 479 | Formel (I-1) | Ar¹-107 | Ar¹-153 | 1459 | Formel (I-2) | Ar¹-107 | Ar¹-153 |
| 480 | Formel (I-1) | Ar¹-107 | Ar¹-162 | 1460 | Formel (I-2) | Ar¹-107 | Ar¹-162 |
| 481 | Formel (I-1) | Ar¹-108 | Ar¹-1 | 1461 | Formel (I-2) | Ar¹-108 | Ar¹-1 |
| 482 | Formel (I-1) | Ar¹-108 | Ar¹-2 | 1462 | Formel (I-2) | Ar¹-108 | Ar¹-2 |
| 483 | Formel (I-1) | Ar¹-108 | Ar¹-3 | 1463 | Formel (I-2) | Ar¹-108 | Ar¹-3 |
| 484 | Formel (I-1) | Ar¹-108 | Ar¹-4 | 1464 | Formel (I-2) | Ar¹-108 | Ar¹-4 |
| 485 | Formel (I-1) | Ar¹-108 | Ar¹-59 | 1465 | Formel (I-2) | Ar¹-108 | Ar¹-59 |
| 486 | Formel (I-1) | Ar¹-108 | Ar¹-60 | 1466 | Formel (I-2) | Ar¹-108 | Ar¹-60 |
| 487 | Formel (I-1) | Ar¹-108 | Ar¹-63 | 1467 | Formel (I-2) | Ar¹-108 | Ar¹-63 |
| 488 | Formel (I-1) | Ar¹-108 | Ar¹-65 | 1468 | Formel (I-2) | Ar¹-108 | Ar¹-65 |
| 489 | Formel (I-1) | Ar¹-108 | Ar¹-68 | 1469 | Formel (I-2) | Ar¹-108 | Ar¹-68 |
| 490 | Formel (I-1) | Ar¹-108 | Ar¹-75 | 1470 | Formel (I-2) | Ar¹-108 | Ar¹-75 |
| 491 | Formel (I-1) | Ar¹-108 | Ar¹-87 | 1471 | Formel (I-2) | Ar¹-108 | Ar¹-87 |
| 492 | Formel (I-1) | Ar¹-108 | Ar¹-91 | 1472 | Formel (I-2) | Ar¹-108 | Ar¹-91 |
| 493 | Formel (I-1) | Ar¹-108 | Ar¹-107 | 1473 | Formel (I-2) | Ar¹-108 | Ar¹-107 |
| 494 | Formel (I-1) | Ar¹-108 | Ar¹-109 | 1474 | Formel (I-2) | Ar¹-108 | Ar¹-109 |
| 495 | Formel (I-1) | Ar¹-108 | Ar¹-111 | 1475 | Formel (I-2) | Ar¹-108 | Ar¹-111 |
| 496 | Formel (I-1) | Ar¹-108 | Ar¹-114 | 1476 | Formel (I-2) | Ar¹-108 | Ar¹-114 |
| 497 | Formel (I-1) | Ar¹-108 | Ar¹-121 | 1477 | Formel (I-2) | Ar¹-108 | Ar¹-121 |
| 498 | Formel (I-1) | Ar¹-108 | Ar¹-151 | 1478 | Formel (I-2) | Ar¹-108 | Ar¹-151 |
| 499 | Formel (I-1) | Ar¹-108 | Ar¹-153 | 1479 | Formel (I-2) | Ar¹-108 | Ar¹-153 |
| 500 | Formel (I-1) | Ar¹-108 | Ar¹-162 | 1480 | Formel (I-2) | Ar¹-108 | Ar¹-162 |
| 501 | Formel (I-1) | Ar¹-109 | Ar¹-1 | 1481 | Formel (I-2) | Ar¹-109 | Ar¹-1 |
| 502 | Formel (I-1) | Ar¹-109 | Ar¹-2 | 1482 | Formel (I-2) | Ar¹-109 | Ar¹-2 |
| 503 | Formel (I-1) | Ar¹-109 | Ar¹-3 | 1483 | Formel (I-2) | Ar¹-109 | Ar¹-3 |
| 504 | Formel (I-1) | Ar¹-109 | Ar¹-4 | 1484 | Formel (I-2) | Ar¹-109 | Ar¹-4 |
| 505 | Formel (I-1) | Ar¹-109 | Ar¹-59 | 1485 | Formel (I-2) | Ar¹-109 | Ar¹-59 |
| 506 | Formel (I-1) | Ar¹-109 | Ar¹-60 | 1486 | Formel (I-2) | Ar¹-109 | Ar¹-60 |
| 507 | Formel (I-1) | Ar¹-109 | Ar¹-63 | 1487 | Formel (I-2) | Ar¹-109 | Ar¹-63 |
| 508 | Formel (I-1) | Ar¹-109 | Ar¹-65 | 1488 | Formel (I-2) | Ar¹-109 | Ar¹-65 |
| 509 | Formel (I-1) | Ar¹-109 | Ar¹-68 | 1489 | Formel (I-2) | Ar¹-109 | Ar¹-68 |
| 510 | Formel (I-1) | Ar¹-109 | Ar¹-75 | 1490 | Formel (I-2) | Ar¹-109 | Ar¹-75 |
| 511 | Formel (I-1) | Ar¹-109 | Ar¹-87 | 1491 | Formel (I-2) | Ar¹-109 | Ar¹-87 |
| 512 | Formel (I-1) | Ar¹-109 | Ar¹-91 | 1492 | Formel (I-2) | Ar¹-109 | Ar¹-91 |
| 513 | Formel (I-1) | Ar¹-109 | Ar¹-107 | 1493 | Formel (I-2) | Ar¹-109 | Ar¹-107 |
| 514 | Formel (I-1) | Ar¹-109 | Ar¹-109 | 1494 | Formel (I-2) | Ar¹-109 | Ar¹-109 |
| 515 | Formel (I-1) | Ar¹-109 | Ar¹-111 | 1495 | Formel (I-2) | Ar¹-109 | Ar¹-111 |
| 516 | Formel (I-1) | Ar¹-109 | Ar¹-114 | 1496 | Formel (I-2) | Ar¹-109 | Ar¹-114 |
| 517 | Formel (I-1) | Ar¹-109 | Ar¹-121 | 1497 | Formel (I-2) | Ar¹-109 | Ar¹-121 |
| 518 | Formel (I-1) | Ar¹-109 | Ar¹-151 | 1498 | Formel (I-2) | Ar¹-109 | Ar¹-151 |
| 519 | Formel (I-1) | Ar¹-109 | Ar¹-153 | 1499 | Formel (I-2) | Ar¹-109 | Ar¹-153 |
| 520 | Formel (I-1) | Ar¹-109 | Ar¹-162 | 1500 | Formel (I-2) | Ar¹-109 | Ar¹-162 |
| 521 | Formel (I-1) | Ar¹-110 | Ar¹-1 | 1501 | Formel (I-2) | Ar¹-110 | Ar¹-1 |
| 522 | Formel (I-1) | Ar¹-110 | Ar¹-2 | 1502 | Formel (I-2) | Ar¹-110 | Ar¹-2 |
| 523 | Formel (I-1) | Ar¹-110 | Ar¹-3 | 1503 | Formel (I-2) | Ar¹-110 | Ar¹-3 |
| 524 | Formel (I-1) | Ar¹-110 | Ar¹-4 | 1504 | Formel (I-2) | Ar¹-110 | Ar¹-4 |
| 525 | Formel (I-1) | Ar¹-110 | Ar¹-59 | 1505 | Formel (I-2) | Ar¹-110 | Ar¹-59 |
| 526 | Formel (I-1) | Ar¹-110 | Ar¹-60 | 1506 | Formel (I-2) | Ar¹-110 | Ar¹-60 |
| 527 | Formel (I-1) | Ar¹-110 | Ar¹-63 | 1507 | Formel (I-2) | Ar¹-110 | Ar¹-63 |
| 528 | Formel (I-1) | Ar¹-110 | Ar¹-65 | 1508 | Formel (I-2) | Ar¹-110 | Ar¹-65 |
| 529 | Formel (I-1) | Ar¹-110 | Ar¹-68 | 1509 | Formel (I-2) | Ar¹-110 | Ar¹-68 |
| 530 | Formel (I-1) | Ar¹-110 | Ar¹-75 | 1510 | Formel (I-2) | Ar¹-110 | Ar¹-75 |
| 531 | Formel (I-1) | Ar¹-110 | Ar¹-87 | 1511 | Formel (I-2) | Ar¹-110 | Ar¹-87 |
| 532 | Formel (I-1) | Ar¹-110 | Ar¹-91 | 1512 | Formel (I-2) | Ar¹-110 | Ar¹-91 |
| 533 | Formel (I-1) | Ar¹-110 | Ar¹-107 | 1513 | Formel (I-2) | Ar¹-110 | Ar¹-107 |
| 534 | Formel (I-1) | Ar¹-110 | Ar¹-109 | 1514 | Formel (I-2) | Ar¹-110 | Ar¹-109 |
| 535 | Formel (I-1) | Ar¹-110 | Ar¹-111 | 1515 | Formel (I-2) | Ar¹-110 | Ar¹-111 |
| 536 | Formel (I-1) | Ar¹-110 | Ar¹-114 | 1516 | Formel (I-2) | Ar¹-110 | Ar¹-114 |
| 537 | Formel (I-1) | Ar¹-110 | Ar¹-121 | 1517 | Formel (I-2) | Ar¹-110 | Ar¹-121 |
| 538 | Formel (I-1) | Ar¹-110 | Ar¹-151 | 1518 | Formel (I-2) | Ar¹-110 | Ar¹-151 |
| 539 | Formel (I-1) | Ar¹-110 | Ar¹-153 | 1519 | Formel (I-2) | Ar¹-110 | Ar¹-153 |
| 540 | Formel (I-1) | Ar¹-110 | Ar¹-162 | 1520 | Formel (I-2) | Ar¹-110 | Ar¹-162 |
| 541 | Formel (I-1) | Ar¹-111 | Ar¹-1 | 1521 | Formel (I-2) | Ar¹-111 | Ar¹-1 |
| 542 | Formel (I-1) | Ar¹-111 | Ar¹-2 | 1522 | Formel (I-2) | Ar¹-111 | Ar¹-2 |
| 543 | Formel (I-1) | Ar¹-111 | Ar¹-3 | 1523 | Formel (I-2) | Ar¹-111 | Ar¹-3 |
| 544 | Formel (I-1) | Ar¹-111 | Ar¹-4 | 1524 | Formel (I-2) | Ar¹-111 | Ar¹-4 |
| 545 | Formel (I-1) | Ar¹-111 | Ar¹-59 | 1525 | Formel (I-2) | Ar¹-111 | Ar¹-59 |
| 546 | Formel (I-1) | Ar¹-111 | Ar¹-60 | 1526 | Formel (I-2) | Ar¹-111 | Ar¹-60 |
| 547 | Formel (I-1) | Ar¹-111 | Ar¹-63 | 1527 | Formel (I-2) | Ar¹-111 | Ar¹-63 |
| 548 | Formel (I-1) | Ar¹-111 | Ar¹-65 | 1528 | Formel (I-2) | Ar¹-111 | Ar¹-65 |
| 549 | Formel (I-1) | Ar¹-111 | Ar¹-68 | 1529 | Formel (I-2) | Ar¹-111 | Ar¹-68 |
| 550 | Formel (I-1) | Ar¹-111 | Ar¹-75 | 1530 | Formel (I-2) | Ar¹-111 | Ar¹-75 |
| 551 | Formel (I-1) | Ar¹-111 | Ar¹-87 | 1531 | Formel (I-2) | Ar¹-111 | Ar¹-87 |
| 552 | Formel (I-1) | Ar¹-111 | Ar¹-91 | 1532 | Formel (I-2) | Ar¹-111 | Ar¹-91 |
| 553 | Formel (I-1) | Ar¹-111 | Ar¹-107 | 1533 | Formel (I-2) | Ar¹-111 | Ar¹-107 |
| 554 | Formel (I-1) | Ar¹-111 | Ar¹-109 | 1534 | Formel (I-2) | Ar¹-111 | Ar¹-109 |
| 555 | Formel (I-1) | Ar¹-111 | Ar¹-111 | 1535 | Formel (I-2) | Ar¹-111 | Ar¹-111 |
| 556 | Formel (I-1) | Ar¹-111 | Ar¹-114 | 1536 | Formel (I-2) | Ar¹-111 | Ar¹-114 |
| 557 | Formel (I-1) | Ar¹-111 | Ar¹-121 | 1537 | Formel (I-2) | Ar¹-111 | Ar¹-121 |
| 558 | Formel (I-1) | Ar¹-111 | Ar¹-151 | 1538 | Formel (I-2) | Ar¹-111 | Ar¹-151 |
| 559 | Formel (I-1) | Ar¹-111 | Ar¹-153 | 1539 | Formel (I-2) | Ar¹-111 | Ar¹-153 |
| 560 | Formel (I-1) | Ar¹-111 | Ar¹-162 | 1540 | Formel (I-2) | Ar¹-111 | Ar¹-162 |
| 561 | Formel (I-1) | Ar¹-112 | Ar¹-1 | 1541 | Formel (I-2) | Ar¹-112 | Ar¹-1 |
| 562 | Formel (I-1) | Ar¹-112 | Ar¹-2 | 1542 | Formel (I-2) | Ar¹-112 | Ar¹-2 |
| 563 | Formel (I-1) | Ar¹-112 | Ar¹-3 | 1543 | Formel (I-2) | Ar¹-112 | Ar¹-3 |
| 564 | Formel (I-1) | Ar¹-112 | Ar¹-4 | 1544 | Formel (I-2) | Ar¹-112 | Ar¹-4 |
| 565 | Formel (I-1) | Ar¹-112 | Ar¹-59 | 1545 | Formel (I-2) | Ar¹-112 | Ar¹-59 |
| 566 | Formel (I-1) | Ar¹-112 | Ar¹-60 | 1546 | Formel (I-2) | Ar¹-112 | Ar¹-60 |
| 567 | Formel (I-1) | Ar¹-112 | Ar¹-63 | 1547 | Formel (I-2) | Ar¹-112 | Ar¹-63 |
| 568 | Formel (I-1) | Ar¹-112 | Ar¹-65 | 1548 | Formel (I-2) | Ar¹-112 | Ar¹-65 |
| 569 | Formel (I-1) | Ar¹-112 | Ar¹-68 | 1549 | Formel (I-2) | Ar¹-112 | Ar¹-68 |
| 570 | Formel (I-1) | Ar¹-112 | Ar¹-75 | 1550 | Formel (I-2) | Ar¹-112 | Ar¹-75 |
| 571 | Formel (I-1) | Ar¹-112 | Ar¹-87 | 1551 | Formel (I-2) | Ar¹-112 | Ar¹-87 |
| 572 | Formel (I-1) | Ar¹-112 | Ar¹-91 | 1552 | Formel (I-2) | Ar¹-112 | Ar¹-91 |
| 573 | Formel (I-1) | Ar¹-112 | Ar¹-107 | 1553 | Formel (I-2) | Ar¹-112 | Ar¹-107 |
| 574 | Formel (I-1) | Ar¹-112 | Ar¹-109 | 1554 | Formel (I-2) | Ar¹-112 | Ar¹-109 |
| 575 | Formel (I-1) | Ar¹-112 | Ar¹-111 | 1555 | Formel (I-2) | Ar¹-112 | Ar¹-111 |
| 576 | Formel (I-1) | Ar¹-112 | Ar¹-114 | 1556 | Formel (I-2) | Ar¹-112 | Ar¹-114 |
| 577 | Formel (I-1) | Ar¹-112 | Ar¹-121 | 1557 | Formel (I-2) | Ar¹-112 | Ar¹-121 |
| 578 | Formel (I-1) | Ar¹-112 | Ar¹-151 | 1558 | Formel (I-2) | Ar¹-112 | Ar¹-151 |
| 579 | Formel (I-1) | Ar¹-112 | Ar¹-153 | 1559 | Formel (I-2) | Ar¹-112 | Ar¹-153 |
| 580 | Formel (I-1) | Ar¹-112 | Ar¹-162 | 1560 | Formel (I-2) | Ar¹-112 | Ar¹-162 |
| 581 | Formel (I-1) | Ar¹-113 | Ar¹-1 | 1561 | Formel (I-2) | Ar¹-113 | Ar¹-1 |
| 582 | Formel (I-1) | Ar¹-113 | Ar¹-2 | 1562 | Formel (I-2) | Ar¹-113 | Ar¹-2 |
| 583 | Formel (I-1) | Ar¹-113 | Ar¹-3 | 1563 | Formel (I-2) | Ar¹-113 | Ar¹-3 |
| 584 | Formel (I-1) | Ar¹-113 | Ar¹-4 | 1564 | Formel (I-2) | Ar¹-113 | Ar¹-4 |
| 585 | Formel (I-1) | Ar¹-113 | Ar¹-59 | 1565 | Formel (I-2) | Ar¹-113 | Ar¹-59 |
| 586 | Formel (I-1) | Ar¹-113 | Ar¹-60 | 1566 | Formel (I-2) | Ar¹-113 | Ar¹-60 |
| 587 | Formel (I-1) | Ar¹-113 | Ar¹-63 | 1567 | Formel (I-2) | Ar¹-113 | Ar¹-63 |
| 588 | Formel (I-1) | Ar¹-113 | Ar¹-65 | 1568 | Formel (I-2) | Ar¹-113 | Ar¹-65 |
| 589 | Formel (I-1) | Ar¹-113 | Ar¹-68 | 1569 | Formel (I-2) | Ar¹-113 | Ar¹-68 |
| 590 | Formel (I-1) | Ar¹-113 | Ar¹-75 | 1570 | Formel (I-2) | Ar¹-113 | Ar¹-75 |
| 591 | Formel (I-1) | Ar¹-113 | Ar¹-87 | 1571 | Formel (I-2) | Ar¹-113 | Ar¹-87 |
| 592 | Formel (I-1) | Ar¹-113 | Ar¹-91 | 1572 | Formel (I-2) | Ar¹-113 | Ar¹-91 |
| 593 | Formel (I-1) | Ar¹-113 | Ar¹-107 | 1573 | Formel (I-2) | Ar¹-113 | Ar¹-107 |
| 594 | Formel (I-1) | Ar¹-113 | Ar¹-109 | 1574 | Formel (I-2) | Ar¹-113 | Ar¹-109 |
| 595 | Formel (I-1) | Ar¹-113 | Ar¹-111 | 1575 | Formel (I-2) | Ar¹-113 | Ar¹-111 |
| 596 | Formel (I-1) | Ar¹-113 | Ar¹-114 | 1576 | Formel (I-2) | Ar¹-113 | Ar¹-114 |
| 597 | Formel (I-1) | Ar¹-113 | Ar¹-121 | 1577 | Formel (I-2) | Ar¹-113 | Ar¹-121 |
| 598 | Formel (I-1) | Ar¹-113 | Ar¹-151 | 1578 | Formel (I-2) | Ar¹-113 | Ar¹-151 |
| 599 | Formel (I-1) | Ar¹-113 | Ar¹-153 | 1579 | Formel (I-2) | Ar¹-113 | Ar¹-153 |
| 600 | Formel (I-1) | Ar¹-113 | Ar¹-162 | 1580 | Formel (I-2) | Ar¹-113 | Ar¹-162 |
| 601 | Formel (I-1) | Ar¹-114 | Ar¹-1 | 1581 | Formel (I-2) | Ar¹-114 | Ar¹-1 |
| 602 | Formel (I-1) | Ar¹-114 | Ar¹-2 | 1582 | Formel (I-2) | Ar¹-114 | Ar¹-2 |
| 603 | Formel (I-1) | Ar¹-114 | Ar¹-3 | 1583 | Formel (I-2) | Ar¹-114 | Ar¹-3 |
| 604 | Formel (I-1) | Ar¹-114 | Ar¹-4 | 1584 | Formel (I-2) | Ar¹-114 | Ar¹-4 |
| 605 | Formel (I-1) | Ar¹-114 | Ar¹-59 | 1585 | Formel (I-2) | Ar¹-114 | Ar¹-59 |
| 606 | Formel (I-1) | Ar¹-114 | Ar¹-60 | 1586 | Formel (I-2) | Ar¹-114 | Ar¹-60 |
| 607 | Formel (I-1) | Ar¹-114 | Ar¹-63 | 1587 | Formel (I-2) | . Ar¹-114 | Ar¹-63 |
| 608 | Formel (I-1) | Ar¹-114 | Ar¹-65 | 1588 | Formel (I-2) | Ar¹-114 | Ar¹-65 |
| 609 | Formel (I-1) | Ar¹-114 | Ar¹-68 | 1589 | Formel (I-2) | Ar¹-114 | Ar¹-68 |
| 610 | Formel (I-1) | Ar¹-114 | Ar¹-75 | 1590 | Formel (I-2) | Ar¹-114 | Ar¹-75 |
| 611 | Formel (I-1) | Ar¹-114 | Ar¹-87 | 1591 | Formel (I-2) | Ar¹-114 | Ar¹-87 |
| 612 | Formel (I-1) | Ar¹-114 | Ar¹-91 | 1592 | Formel (I-2) | Ar¹-114 | Ar¹-91 |
| 613 | Formel (I-1) | Ar¹-114 | Ar¹-107 | 1593 | Formel (I-2) | Ar¹-114 | Ar¹-107 |
| 614 | Formel (I-1) | Ar¹-114 | Ar¹-109 | 1594 | Formel (I-2) | Ar¹-114 | Ar¹-109 |
| 615 | Formel (I-1) | Ar¹-114 | Ar¹-111 | 1595 | Formel (I-2) | Ar¹-114 | Ar¹-111 |
| 616 | Formel (I-1) | Ar¹-114 | Ar¹-114 | 1596 | Formel (I-2) | Ar¹-114 | Ar¹-114 |
| 617 | Formel (I-1) | Ar¹-114 | Ar¹-121 | 1597 | Formel (I-2) | Ar¹-114 | Ar¹-121 |
| 618 | Formel (I-1) | Ar¹-114 | Ar¹-151 | 1598 | Formel (I-2) | Ar¹-114 | Ar¹-151 |
| 619 | Formel (I-1) | Ar¹-114 | Ar¹-153 | 1599 | Formel (I-2) | Ar¹-114 | Ar¹-153 |
| 620 | Formel (I-1) | Ar¹-114 | Ar¹-162 | 1600 | Formel (I-2) | Ar¹-114 | Ar¹-162 |
| 621 | Formel (I-1) | Ar¹-117 | Ar¹-1 | 1601 | Formel (I-2) | Ar¹-117 | Ar¹-1 |
| 622 | Formel (I-1) | Ar¹-117 | Ar¹-2 | 1602 | Formel (I-2) | Ar¹-117 | Ar¹-2 |
| 623 | Formel (I-1) | Ar¹-117 | Ar¹-3 | 1603 | Formel (I-2) | Ar¹-117 | Ar¹-3 |
| 624 | Formel (I-1) | Ar¹-117 | Ar¹-4 | 1604 | Formel (I-2) | Ar¹-117 | Ar¹-4 |
| 625 | Formel (I-1) | Ar¹-117 | Ar¹-59 | 1605 | Formel (I-2) | Ar¹-117 | Ar¹-59 |
| 626 | Formel (I-1) | Ar¹-117 | Ar¹-60 | 1606 | Formel (I-2) | Ar¹-117 | Ar¹-60 |
| 627 | Formel (I-1) | Ar¹-117 | Ar¹-63 | 1607 | Formel (I-2) | Ar¹-117 | Ar¹-63 |
| 628 | Formel (I-1) | Ar¹-117 | Ar¹-65 | 1608 | Formel (I-2) | Ar¹-117 | Ar¹-65 |
| 629 | Formel (I-1) | Ar¹-117 | Ar¹-68 | 1609 | Formel (I-2) | Ar¹-117 | Ar¹-68 |
| 630 | Formel (I-1) | Ar¹-117 | Ar¹-75 | 1610 | Formel (I-2) | Ar¹-117 | Ar¹-75 |
| 631 | Formel (I-1) | Ar¹-117 | Ar¹-87 | 1611 | Formel (I-2) | Ar¹-117 | Ar¹-87 |
| 632 | Formel (I-1) | Ar¹-117 | Ar¹-91 | 1612 | Formel (I-2) | Ar¹-117 | Ar¹-91 |
| 633 | Formel (I-1) | Ar¹-117 | Ar¹-107 | 1613 | Formel (I-2) | Ar¹-117 | Ar¹-107 |
| 634 | Formel (I-1) | Ar¹-117 | Ar¹-109 | 1614 | Formel (I-2) | Ar¹-117 | Ar¹-109 |
| 635 | Formel (I-1) | Ar¹-117 | Ar¹-111 | 1615 | Formel (I-2) | Ar¹-117 | Ar¹-111 |
| 636 | Formel (I-1) | Ar¹-117 | Ar¹-114 | 1616 | Formel (I-2) | Ar¹-117 | Ar¹-114 |
| 637 | Formel (I-1) | Ar¹-117 | Ar¹-121 | 1617 | Formel (I-2) | Ar¹-117 | Ar¹-121 |
| 638 | Formel (I-1) | Ar¹-117 | Ar¹-151 | 1618 | Formel (I-2) | Ar¹-117 | Ar¹-151 |
| 639 | Formel (I-1) | Ar¹-117 | Ar¹-153 | 1619 | Formel (I-2) | Ar¹-117 | Ar¹-153 |
| 640 | Formel (I-1) | Ar¹-117 | Ar¹-162 | 1620 | Formel (I-2) | Ar¹-117 | Ar¹-162 |
| 641 | Formel (I-1) | Ar¹-121 | Ar¹-1 | 1621 | Formel (I-2) | Ar¹-121 | Ar¹-1 |
| 642 | Formel (I-1) | Ar¹-121 | Ar¹-2 | 1622 | Formel (I-2) | Ar¹-121 | Ar¹-2 |
| 643 | Formel (I-1) | Ar¹-121 | Ar¹-3 | 1623 | Formel (I-2) | Ar¹-121 | Ar¹-3 |
| 644 | Formel (I-1) | Ar¹-121 | Ar¹-4 | 1624 | Formel (I-2) | Ar¹-121 | Ar¹-4 |
| 645 | Formel (I-1) | Ar¹-121 | Ar¹-59 | 1625 | Formel (I-2) | Ar¹-121 | Ar¹-59 |
| 646 | Formel (I-1) | Ar¹-121 | Ar¹-60 | 1626 | Formel (I-2) | Ar¹-121 | Ar¹-60 |
| 647 | Formel (I-1) | Ar¹-121 | Ar¹-63 | 1627 | Formel (I-2) | Ar¹-121 | Ar¹-63 |
| 648 | Formel (I-1) | Ar¹-121 | Ar¹-65 | 1628 | Formel (I-2) | Ar¹-121 | Ar¹-65 |
| 649 | Formel (I-1) | Ar¹-121 | Ar¹-68 | 1629 | Formel (I-2) | Ar¹-121 | Ar¹-68 |
| 650 | Formel (I-1) | Ar¹-121 | Ar¹-75 | 1630 | Formel (I-2) | Ar¹-121 | Ar¹-75 |
| 651 | Formel (I-1) | Ar¹-121 | Ar¹-87 | 1631 | Formel (I-2) | Ar¹-121 | Ar¹-87 |
| 652 | Formel (I-1) | Ar¹-121 | Ar¹-91 | 1632 | Formel (I-2) | Ar¹-121 | Ar¹-91 |
| 653 | Formel (I-1) | Ar¹-121 | Ar¹-107 | 1633 | Formel (I-2) | Ar¹-121 | Ar¹-107 |
| 654 | Formel (I-1) | Ar¹-121 | Ar¹-109 | 1634 | Formel (I-2) | Ar¹-121 | Ar¹-109 |
| 655 | Formel (I-1) | Ar¹-121 | Ar¹-111 | 1635 | Formel (I-2) | Ar¹-121 | Ar¹-111 |
| 656 | Formel (I-1) | Ar¹-121 | Ar¹-114 | 1636 | Formel (I-2) | Ar¹-121 | Ar¹-114 |
| 657 | Formel (I-1) | Ar¹-121 | Ar¹-121 | 1637 | Formel (I-2) | Ar¹-121 | Ar¹-121 |
| 658 | Formel (I-1) | Ar¹-121 | Ar¹-151 | 1638 | Formel (I-2) | Ar¹-121 | Ar¹-151 |
| 659 | Formel (I-1) | Ar¹-121 | Ar¹-153 | 1639 | Formel (I-2) | Ar¹-121 | Ar¹-153 |
| 660 | Formel (I-1) | Ar¹-121 | Ar¹-162 | 1640 | Formel (I-2) | Ar¹-121 | Ar¹-162 |
| 661 | Formel (I-1) | Ar¹-127 | Ar¹-1 | 1641 | Formel (I-2) | Ar¹-127 | Ar¹-1 |
| 662 | Formel (I-1) | Ar¹-127 | Ar¹-2 | 1642 | Formel (I-2) | Ar¹-127 | Ar¹-2 |
| 663 | Formel (I-1) | Ar¹-127 | Ar¹-3 | 1643 | Formel (I-2) | Ar¹-127 | Ar¹-3 |
| 664 | Formel (I-1) | Ar¹-127 | Ar¹-4 | 1644 | Formel (I-2) | Ar¹-127 | Ar¹-4 |
| 665 | Formel (I-1) | Ar¹-127 | Ar¹-59 | 1645 | Formel (I-2) | Ar¹-127 | Ar¹-59 |
| 666 | Formel (I-1) | Ar¹-127 | Ar¹-60 | 1646 | Formel (I-2) | Ar¹-127 | Ar¹-60 |
| 667 | Formel (I-1) | Ar¹-127 | Ar¹-63 | 1647 | Formel (I-2) | Ar¹-127 | Ar¹-63 |
| 668 | Formel (I-1) | Ar¹-127 | Ar¹-65 | 1648 | Formel (I-2) | Ar¹-127 | Ar¹-65 |
| 669 | Formel (I-1) | Ar¹-127 | Ar¹-68 | 1649 | Formel (I-2) | Ar¹-127 | Ar¹-68 |
| 670 | Formel (I-1) | Ar¹-127 | Ar¹-75 | 1650 | Formel (I-2) | Ar¹-127 | Ar¹-75 |
| 671 | Formel (I-1) | Ar¹-127 | Ar¹-87 | 1651 | Formel (I-2) | Ar¹-127 | Ar¹-87 |
| 672 | Formel (I-1) | Ar¹-127 | Ar¹-91 | 1652 | Formel (I-2) | Ar¹-127 | Ar¹-91 |
| 673 | Formel (I-1) | Ar¹-127 | Ar¹-107 | 1653 | Formel (I-2) | Ar¹-127 | Ar¹-107 |
| 674 | Formel (I-1) | Ar¹-127 | Ar¹-109 | 1654 | Formel (I-2) | Ar¹-127 | Ar¹-109 |
| 675 | Formel (I-1) | Ar¹-127 | Ar¹-111 | 1655 | Formel (I-2) | Ar¹-127 | Ar¹-111 |
| 676 | Formel (I-1) | Ar¹-127 | Ar¹-114 | 1656 | Formel (I-2) | Ar¹-127 | Ar¹-114 |
| 677 | Formel (I-1) | Ar¹-127 | Ar¹-121 | 1657 | Formel (I-2) | Ar¹-127 | Ar¹-121 |
| 678 | Formel (I-1) | Ar¹-127 | Ar¹-151 | 1658 | Formel (I-2) | Ar¹-127 | Ar¹-151 |
| 679 | Formel (I-1) | Ar¹-127 | Ar¹-153 | 1659 | Formel (I-2) | Ar¹-127 | Ar¹-153 |
| 680 | Formel (I-1) | Ar¹-127 | Ar¹-162 | 1660 | Formel (I-2) | Ar¹-127 | Ar¹-162 |
| 681 | Formel (I-1) | Ar¹-151 | Ar¹-1 | 1661 | Formel (I-2) | Ar¹-151 | Ar¹-1 |
| 682 | Formel (I-1) | Ar¹-151 | Ar¹-2 | 1662 | Formel (I-2) | Ar¹-151 | Ar¹-2 |
| 683 | Formel (I-1) | Ar¹-151 | Ar¹-3 | 1663 | Formel (I-2) | Ar¹-151 | Ar¹-3 |
| 684 | Formel (I-1) | Ar¹-151 | Ar¹-4 | 1664 | Formel (I-2) | Ar¹-151 | Ar¹-4 |
| 685 | Formel (I-1) | Ar¹-151 | Ar¹-59 | 1665 | Formel (I-2) | Ar¹-151 | Ar¹-59 |
| 686 | Formel (I-1) | Ar¹-151 | Ar¹-60 | 1666 | Formel (I-2) | Ar¹-151 | Ar¹-60 |
| 687 | Formel (I-1) | Ar¹-151 | Ar¹-63 | 1667 | Formel (I-2) | Ar¹-151 | Ar¹-63 |
| 688 | Formel (I-1) | Ar¹-151 | Ar¹-65 | 1668 | Formel (I-2) | Ar¹-151 | Ar¹-65 |
| 689 | Formel (I-1) | Ar¹-151 | Ar¹-68 | 1669 | Formel (I-2) | Ar¹-151 | Ar¹-68 |
| 690 | Formel (I-1) | Ar¹-151 | Ar¹-75 | 1670 | Formel (I-2) | Ar¹-151 | Ar¹-75 |
| 691 | Formel (I-1) | Ar¹-151 | Ar¹-87 | 1671 | Formel (I-2) | Ar¹-151 | Ar¹-87 |
| 692 | Formel (I-1) | Ar¹-151 | Ar¹-91 | 1672 | Formel (I-2) | Ar¹-151 | Ar¹-91 |
| 693 | Formel (I-1) | Ar¹-151 | Ar¹-107 | 1673 | Formel (I-2) | Ar¹-151 | Ar¹-107 |
| 694 | Formel (I-1) | Ar¹-151 | Ar¹-109 | 1674 | Formel (I-2) | Ar¹-151 | Ar¹-109 |
| 695 | Formel (I-1) | Ar¹-151 | Ar¹-111 | 1675 | Formel (I-2) | Ar¹-151 | Ar¹-111 |
| 696 | Formel (I-1) | Ar¹-151 | Ar¹-114 | 1676 | Formel (I-2) | Ar¹-151 | Ar¹-114 |
| 697 | Formel (I-1) | Ar¹-151 | Ar¹-121 | 1677 | Formel (I-2) | Ar¹-151 | Ar¹-121 |
| 698 | Formel (I-1) | Ar¹-151 | Ar¹-151 | 1678 | Formel (I-2) | Ar¹-151 | Ar¹-151 |
| 699 | Formel (I-1) | Ar¹-151 | Ar¹-153 | 1679 | Formel (I-2) | Ar¹-151 | Ar¹-153 |
| 700 | Formel (I-1) | Ar¹-151 | Ar¹-162 | 1680 | Formel (I-2) | Ar¹-151 | Ar¹-162 |
| 701 | Formel (I-1) | Ar¹-153 | Ar¹-1 | 1681 | Formel (I-2) | Ar¹-153 | Ar¹-1 |
| 702 | Formel (I-1) | Ar¹-153 | Ar¹-2 | 1682 | Formel (I-2) | Ar¹-153 | Ar¹-2 |
| 703 | Formel (I-1) | Ar¹-153 | Ar¹-3 | 1683 | Formel (I-2) | Ar¹-153 | Ar¹-3 |
| 704 | Formel (I-1) | Ar¹-153 | Ar¹-4 | 1684 | Formel (I-2) | Ar¹-153 | Ar¹-4 |
| 705 | Formel (I-1) | Ar¹-153 | Ar¹-59 | 1685 | Formel (I-2) | Ar¹-153 | Ar¹-59 |
| 706 | Formel (I-1) | Ar¹-153 | Ar¹-60 | 1686 | Formel (I-2) | Ar¹-153 | Ar¹-60 |
| 707 | Formel (I-1) | Ar¹-153 | Ar¹-63 | 1687 | Formel (I-2) | Ar¹-153 | Ar¹-63 |
| 708 | Formel (I-1) | Ar¹-153 | Ar¹-65 | 1688 | Formel (I-2) | Ar¹-153 | Ar¹-65 |
| 709 | Formel (I-1) | Ar¹-153 | Ar¹-68 | 1689 | Formel (I-2) | Ar¹-153 | Ar¹-68 |
| 710 | Formel (I-1) | Ar¹-153 | Ar¹-75 | 1690 | Formel (I-2) | Ar¹-153 | Ar¹-75 |
| 711 | Formel (I-1) | Ar¹-153 | Ar¹-87 | 1691 | Formel (I-2) | Ar¹-153 | Ar¹-87 |
| 712 | Formel (I-1) | Ar¹-153 | Ar¹-91 | 1692 | Formel (I-2) | Ar¹-153 | Ar¹-91 |
| 713 | Formel (I-1) | Ar¹-153 | Ar¹-107 | 1693 | Formel (I-2) | Ar¹-153 | Ar¹-107 |
| 714 | Formel (I-1) | Ar¹-153 | Ar¹-109 | 1694 | Formel (I-2) | Ar¹-153 | Ar¹-109 |
| 715 | Formel (I-1) | Ar¹-153 | Ar¹-111 | 1695 | Formel (I-2) | Ar¹-153 | Ar¹-111 |
| 716 | Formel (I-1) | Ar¹-153 | Ar¹-114 | 1696 | Formel (I-2) | Ar¹-153 | Ar¹-114 |
| 717 | Formel (I-1) | Ar¹-153 | Ar¹-121 | 1697 | Formel (I-2) | Ar¹-153 | Ar¹-121 |
| 718 | Formel (I-1) | Ar¹-153 | Ar¹-151 | 1698 | Formel (I-2) | Ar¹-153 | Ar¹-151 |
| 719 | Formel (I-1) | Ar¹-153 | Ar¹-153 | 1699 | Formel (I-2) | Ar¹-153 | Ar¹-153 |
| 720 | Formel (I-1) | Ar¹-153 | Ar¹-162 | 1700 | Formel (I-2) | Ar¹-153 | Ar¹-162 |
| 721 | Formel (I-1) | Ar¹-155 | Ar¹-1 | 1701 | Formel (I-2) | Ar¹-155 | Ar¹-1 |
| 722 | Formel (I-1) | Ar¹-155 | Ar¹-2 | 1702 | Formel (I-2) | Ar¹-155 | Ar¹-2 |
| 723 | Formel (I-1) | Ar¹-155 | Ar¹-3 | 1703 | Formel (I-2) | Ar¹-155 | Ar¹-3 |
| 724 | Formel (I-1) | Ar¹-155 | Ar¹-4 | 1704 | Formel (I-2) | Ar¹-155 | Ar¹-4 |
| 725 | Formel (I-1) | Ar¹-155 | Ar¹-59 | 1705 | Formel (I-2) | Ar¹-155 | Ar¹-59 |
| 726 | Formel (I-1) | Ar¹-155 | Ar¹-60 | 1706 | Formel (I-2) | Ar¹-155 | Ar¹-60 |
| 727 | Formel (I-1) | Ar¹-155 | Ar¹-63 | 1707 | Formel (I-2) | Ar¹-155 | Ar¹-63 |
| 728 | Formel (I-1) | Ar¹-155 | Ar¹-65 | 1708 | Formel (I-2) | Ar¹-155 | Ar¹-65 |
| 729 | Formel (I-1) | Ar¹-155 | Ar¹-68 | 1709 | Formel (I-2) | Ar¹-155 | Ar¹-68 |
| 730 | Formel (I-1) | Ar¹-155 | Ar¹-75 | 1710 | Formel (I-2) | Ar¹-155 | Ar¹-75 |
| 731 | Formel (I-1) | Ar¹-155 | Ar¹-87 | 1711 | Formel (I-2) | Ar¹-155 | Ar¹-87 |
| 732 | Formel (I-1) | Ar¹-155 | Ar¹-91 | 1712 | Formel (I-2) | Ar¹-155 | Ar¹-91 |
| 733 | Formel (I-1) | Ar¹-155 | Ar¹-107 | 1713 | Formel (I-2) | Ar¹-155 | Ar¹-107 |
| 734 | Formel (I-1) | Ar¹-155 | Ar¹-109 | 1714 | Formel (I-2) | Ar¹-155 | Ar¹-109 |
| 735 | Formel (I-1) | Ar¹-155 | Ar¹-111 | 1715 | Formel (I-2) | Ar¹-155 | Ar¹-111 |
| 736 | Formel (I-1) | Ar¹-155 | Ar¹-114 | 1716 | Formel (I-2) | Ar¹-155 | Ar¹-114 |
| 737 | Formel (I-1) | Ar¹-155 | Ar¹-121 | 1717 | Formel (I-2) | Ar¹-155 | Ar¹-121 |
| 738 | Formel (I-1) | Ar¹-155 | Ar¹-151 | 1718 | Formel (I-2) | Ar¹-155 | Ar¹-151 |
| 739 | Formel (I-1) | Ar¹-155 | Ar¹-153 | 1719 | Formel (I-2) | Ar¹-155 | Ar¹-153 |
| 740 | Formel (I-1) | Ar¹-155 | Ar¹-162 | 1720 | Formel (I-2) | Ar¹-155 | Ar¹-162 |
| 741 | Formel (I-1) | Ar¹-156 | Ar¹-1 | 1721 | Formel (I-2) | Ar¹-156 | Ar¹-1 |
| 742 | Formel (I-1) | Ar¹-156 | Ar¹-2 | 1722 | Formel (I-2) | Ar¹-156 | Ar¹-2 |
| 743 | Formel (I-1) | Ar¹-156 | Ar¹-3 | 1723 | Formel (I-2) | Ar¹-156 | Ar¹-3 |
| 744 | Formel (I-1) | Ar¹-156 | Ar¹-4 | 1724 | Formel (I-2) | Ar¹-156 | Ar¹-4 |
| 745 | Formel (I-1) | Ar¹-156 | Ar¹-59 | 1725 | Formel (I-2) | Ar¹-156 | Ar¹-59 |
| 746 | Formel (I-1) | Ar¹-156 | Ar¹-60 | 1726 | Formel (I-2) | Ar¹-156 | Ar¹-60 |
| 747 | Formel (I-1) | Ar¹-156 | Ar¹-63 | 1727 | Formel (I-2) | Ar¹-156 | Ar¹-63 |
| 748 | Formel (I-1) | Ar¹-156 | Ar¹-65 | 1728 | Formel (I-2) | Ar¹-156 | Ar¹-65 |
| 749 | Formel (I-1) | Ar¹-156 | Ar¹-68 | 1729 | Formel (I-2) | Ar¹-156 | Ar¹-68 |
| 750 | Formel (I-1) | Ar¹-156 | Ar¹-75 | 1730 | Formel (I-2) | Ar¹-156 | Ar¹-75 |
| 751 | Formel (I-1) | Ar¹-156 | Ar¹-87 | 1731 | Formel (I-2) | Ar¹-156 | Ar¹-87 |
| 752 | Formel (I-1) | Ar¹-156 | Ar¹-91 | 1732 | Formel (I-2) | Ar¹-156 | Ar¹-91 |
| 753 | Formel (I-1) | Ar¹-156 | Ar¹-107 | 1733 | Formel (I-2) | Ar¹-156 | Ar¹-107 |
| 754 | Formel (I-1) | Ar¹-156 | Ar¹-109 | 1734 | Formel (I-2) | Ar¹-156 | Ar¹-109 |
| 755 | Formel (I-1) | Ar¹-156 | Ar¹-111 | 1735 | Formel (I-2) | Ar¹-156 | Ar¹-111 |
| 756 | Formel (I-1) | Ar¹-156 | Ar¹-114 | 1736 | Formel (I-2) | Ar¹-156 | Ar¹-114 |
| 757 | Formel (I-1) | Ar¹-156 | Ar¹-121 | 1737 | Formel (I-2) | Ar¹-156 | Ar¹-121 |
| 758 | Formel (I-1) | Ar¹-156 | Ar¹-151 | 1738 | Formel (I-2) | Ar¹-156 | Ar¹-151 |
| 759 | Formel (I-1) | Ar¹-156 | Ar¹-153 | 1739 | Formel (I-2) | Ar¹-156 | Ar¹-153 |
| 760 | Formel (I-1) | Ar¹-156 | Ar¹-162 | 1740 | Formel (I-2) | Ar¹-156 | Ar¹-162 |
| 761 | Formel (I-1) | Ar¹-157 | Ar¹-1 | 1741 | Formel (I-2) | Ar¹-157 | Ar¹-1 |
| 762 | Formel (I-1) | Ar¹-157 | Ar¹-2 | 1742 | Formel (I-2) | Ar¹-157 | Ar¹-2 |
| 763 | Formel (I-1) | Ar¹-157 | Ar¹-3 | 1743 | Formel (I-2) | Ar¹-157 | Ar¹-3 |
| 764 | Formel (I-1) | Ar¹-157 | Ar¹-4 | 1744 | Formel (I-2) | Ar¹-157 | Ar¹-4 |
| 765 | Formel (I-1) | Ar¹-157 | Ar¹-59 | 1745 | Formel (I-2) | Ar¹-157 | Ar¹-59 |
| 766 | Formel (I-1) | Ar¹-157 | Ar¹-60 | 1746 | Formel (I-2) | Ar¹-157 | Ar¹-60 |
| 767 | Formel (I-1) | Ar¹-157 | Ar¹-63 | 1747 | Formel (I-2) | Ar¹-157 | Ar¹-63 |
| 768 | Formel (I-1) | Ar¹-157 | Ar¹-65 | 1748 | Formel (I-2) | Ar¹-157 | Ar¹-65 |
| 769 | Formel (I-1) | Ar¹-157 | Ar¹-68 | 1749 | Formel (I-2) | Ar¹-157 | Ar¹-68 |
| 770 | Formel (I-1) | Ar¹-157 | Ar¹-75 | 1750 | Formel (I-2) | Ar¹-157 | Ar¹-75 |
| 771 | Formel (I-1) | Ar¹-157 | Ar¹-87 | 1751 | Formel (I-2) | Ar¹-157 | Ar¹-87 |
| 772 | Formel (I-1) | Ar¹-157 | Ar¹-91 | 1752 | Formel (I-2) | Ar¹-157 | Ar¹-91 |
| 773 | Formel (I-1) | Ar¹-157 | Ar¹-107 | 1753 | Formel (I-2) | Ar¹-157 | Ar¹-107 |
| 774 | Formel (I-1) | Ar¹-157 | Ar¹-109 | 1754 | Formel (I-2) | Ar¹-157 | Ar¹-109 |
| 775 | Formel (I-1) | Ar¹-157 | Ar¹-111 | 1755 | Formel (I-2) | Ar¹-157 | Ar¹-111 |
| 776 | Formel (I-1) | Ar¹-157 | Ar¹-114 | 1756 | Formel (I-2) | Ar¹-157 | Ar¹-114 |
| 777 | Formel (I-1) | Ar¹-157 | Ar¹-121 | 1757 | Formel (I-2) | Ar¹-157 | Ar¹-121 |
| 778 | Formel (I-1) | Ar¹-157 | Ar¹-151 | 1758 | Formel (I-2) | Ar¹-157 | Ar¹-151 |
| 779 | Formel (I-1) | Ar¹-157 | Ar¹-153 | 1759 | Formel (I-2) | Ar¹-157 | Ar¹-153 |
| 780 | Formel (I-1) | Ar¹-157 | Ar¹-162 | 1760 | Formel (I-2) | Ar¹-157 | Ar¹-162 |
| 781 | Formel (I-1) | Ar¹-158 | Ar¹-1 | 1761 | Formel (I-2) | Ar¹-158 | Ar¹-1 |
| 782 | Formel (I-1) | Ar¹-158 | Ar¹-2 | 1762 | Formel (I-2) | Ar¹-158 | Ar¹-2 |
| 783 | Formel (I-1) | Ar¹-158 | Ar¹-3 | 1763 | Formel (I-2) | Ar¹-158 | Ar¹-3 |
| 784 | Formel (I-1) | Ar¹-158 | Ar¹-4 | 1764 | Formel (I-2) | Ar¹-158 | Ar¹-4 |
| 785 | Formel (I-1) | Ar¹-158 | Ar¹-59 | 1765 | Formel (I-2) | Ar¹-158 | Ar¹-59 |
| 786 | Formel (I-1) | Ar¹-158 | Ar¹-60 | 1766 | Formel (I-2) | Ar¹-158 | Ar¹-60 |
| 787 | Formel (I-1) | Ar¹-158 | Ar¹-63 | 1767 | Formel (I-2) | Ar¹-158 | Ar¹-63 |
| 788 | Formel (I-1) | Ar¹-158 | Ar¹-65 | 1768 | Formel (I-2) | Ar¹-158 | Ar¹-65 |
| 789 | Formel (I-1) | Ar¹-158 | Ar¹-68 | 1769 | Formel (I-2) | Ar¹-158 | Ar¹-68 |
| 790 | Formel (I-1) | Ar¹-158 | Ar¹-75 | 1770 | Formel (I-2) | Ar¹-158 | Ar¹-75 |
| 791 | Formel (I-1) | Ar¹-158 | Ar¹-87 | 1771 | Formel (I-2) | Ar¹-158 | Ar¹-87 |
| 792 | Formel (I-1) | Ar¹-158 | Ar¹-91 | 1772 | Formel (I-2) | Ar¹-158 | Ar¹-91 |
| 793 | Formel (I-1) | Ar¹-158 | Ar¹-107 | 1773 | Formel (I-2) | Ar¹-158 | Ar¹-107 |
| 794 | Formel (I-1) | Ar¹-158 | Ar¹-109 | 1774 | Formel (I-2) | Ar¹-158 | Ar¹-109 |
| 795 | Formel (I-1) | Ar¹-158 | Ar¹-111 | 1775 | Formel (I-2) | Ar¹-158 | Ar¹-111 |
| 796 | Formel (I-1) | Ar¹-158 | Ar¹-114 | 1776 | Formel (I-2) | Ar¹-158 | Ar¹-114 |
| 797 | Formel (I-1) | Ar¹-158 | Ar¹-121 | 1777 | Formel (I-2) | Ar¹-158 | Ar¹-121 |
| 798 | Formel (I-1) | Ar¹-158 | Ar¹-151 | 1778 | Formel (I-2) | Ar¹-158 | Ar¹-151 |
| 799 | Formel (I-1) | Ar¹-158 | Ar¹-153 | 1779 | Formel (I-2) | Ar¹-158 | Ar¹-153 |
| 800 | Formel (I-1) | Ar¹-158 | Ar¹-162 | 1780 | Formel (I-2) | Ar¹-158 | Ar¹-162 |
| 801 | Formel (I-1) | Ar¹-160 | Ar¹-1 | 1781 | Formel (I-2) | Ar¹-160 | Ar¹-1 |
| 802 | Formel (I-1) | Ar¹-160 | Ar¹-2 | 1782 | Formel (I-2) | Ar¹-160 | Ar¹-2 |
| 803 | Formel (I-1) | Ar¹-160 | Ar¹-3 | 1783 | Formel (I-2) | Ar¹-160 | Ar¹-3 |
| 804 | Formel (I-1) | Ar¹-160 | Ar¹-4 | 1784 | Formel (I-2) | Ar¹-160 | Ar¹-4 |
| 805 | Formel (I-1) | Ar¹-160 | Ar¹-59 | 1785 | Formel (I-2) | Ar¹-160 | Ar¹-59 |
| 806 | Formel (I-1) | Ar¹-160 | Ar¹-60 | 1786 | Formel (I-2) | Ar¹-160 | Ar¹-60 |
| 807 | Formel (I-1) | Ar¹-160 | Ar¹-63 | 1787 | Formel (I-2) | Ar¹-160 | Ar¹-63 |
| 808 | Formel (I-1) | Ar¹-160 | Ar¹-65 | 1788 | Formel (I-2) | Ar¹-160 | Ar¹-65 |
| 809 | Formel (I-1) | Ar¹-160 | Ar¹-68 | 1789 | Formel (I-2) | Ar¹-160 | Ar¹-68 |
| 810 | Formel (I-1) | Ar¹-160 | Ar¹-75 | 1790 | Formel (I-2) | Ar¹-160 | Ar¹-75 |
| 811 | Formel (I-1) | Ar¹-160 | Ar¹-87 | 1791 | Formel (I-2) | Ar¹-160 | Ar¹-87 |
| 812 | Formel (I-1) | Ar¹-160 | Ar¹-91 | 1792 | Formel (I-2) | Ar¹-160 | Ar¹-91 |
| 813 | Formel (I-1) | Ar¹-160 | Ar¹-107 | 1793 | Formel (I-2) | Ar¹-160 | Ar¹-107 |
| 814 | Formel (I-1) | Ar¹-160 | Ar¹-109 | 1794 | Formel (I-2) | Ar¹-160 | Ar¹-109 |
| 815 | Formel (I-1) | Ar¹-160 | Ar¹-111 | 1795 | Formel (I-2) | Ar¹-160 | Ar¹-111 |
| 816 | Formel (I-1) | Ar¹-160 | Ar¹-114 | 1796 | Formel (I-2) | Ar¹-160 | Ar¹-114 |
| 817 | Formel (I-1) | Ar¹-160 | Ar¹-121 | 1797 | Formel (I-2) | Ar¹-160 | Ar¹-121 |
| 818 | Formel (I-1) | Ar¹-160 | Ar¹-151 | 1798 | Formel (I-2) | Ar¹-160 | Ar¹-151 |
| 819 | Formel (I-1) | Ar¹-160 | Ar¹-153 | 1799 | Formel (I-2) | Ar¹-160 | Ar¹-153 |
| 820 | Formel (I-1) | Ar¹-160 | Ar¹-162 | 1800 | Formel (I-2) | Ar¹-160 | Ar¹-162 |
| 821 | Formel (I-1) | Ar¹-162 | Ar¹-1 | 1801 | Formel (I-2) | Ar¹-162 | Ar¹-1 |
| 822 | Formel (I-1) | Ar¹-162 | Ar¹-2 | 1802 | Formel (I-2) | Ar¹-162 | Ar¹-2 |
| 823 | Formel (I-1) | Ar¹-162 | Ar¹-3 | 1803 | Formel (I-2) | Ar¹-162 | Ar¹-3 |
| 824 | Formel (I-1) | Ar¹-162 | Ar¹-4 | 1804 | Formel (I-2) | Ar¹-162 | Ar¹-4 |
| 825 | Formel (I-1) | Ar¹-162 | Ar¹-59 | 1805 | Formel (I-2) | Ar¹-162 | Ar¹-59 |
| 826 | Formel (I-1) | Ar¹-162 | Ar¹-60 | 1806 | Formel (I-2) | Ar¹-162 | Ar¹-60 |
| 827 | Formel (I-1) | Ar¹-162 | Ar¹-63 | 1807 | Formel (I-2) | Ar¹-162 | Ar¹-63 |
| 828 | Formel (I-1) | Ar¹-162 | Ar¹-65 | 1808 | Formel (I-2) | Ar¹-162 | Ar¹-65 |
| 829 | Formel (I-1) | Ar¹-162 | Ar¹-68 | 1809 | Formel (I-2) | Ar¹-162 | Ar¹-68 |
| 830 | Formel (I-1) | Ar¹-162 | Ar¹-75 | 1810 | Formel (I-2) | Ar¹-162 | Ar¹-75 |
| 831 | Formel (I-1) | Ar¹-162 | Ar¹-87 | 1811 | Formel (I-2) | Ar¹-162 | Ar¹-87 |
| 832 | Formel (I-1) | Ar¹-162 | Ar¹-91 | 1812 | Formel (I-2) | Ar¹-162 | Ar¹-91 |
| 833 | Formel (I-1) | Ar¹-162 | Ar¹-107 | 1813 | Formel (I-2) | Ar¹-162 | Ar¹-107 |
| 834 | Formel (I-1) | Ar¹-162 | Ar¹-109 | 1814 | Formel (I-2) | Ar¹-162 | Ar¹-109 |
| 835 | Formel (I-1) | Ar¹-162 | Ar¹-111 | 1815 | Formel (I-2) | Ar¹-162 | Ar¹-111 |
| 836 | Formel (I-1) | Ar¹-162 | Ar¹-114 | 1816 | Formel (I-2) | Ar¹-162 | Ar¹-114 |
| 837 | Formel (I-1) | Ar¹-162 | Ar¹-121 | 1817 | Formel (I-2) | Ar¹-162 | Ar¹-121 |
| 838 | Formel (I-1) | Ar¹-162 | Ar¹-151 | 1818 | Formel (I-2) | Ar¹-162 | Ar¹-151 |
| 839 | Formel (I-1) | Ar¹-162 | Ar¹-153 | 1819 | Formel (I-2) | Ar¹-162 | Ar¹-153 |
| 840 | Formel (I-1) | Ar¹-162 | Ar¹-162 | 1820 | Formel (I-2) | Ar¹-162 | Ar¹-162 |
| 841 | Formel (I-1) | Ar¹-163 | Ar¹-1 | 1821 | Formel (I-2) | Ar¹-163 | Ar¹-1 |
| 842 | Formel (I-1) | Ar¹-163 | Ar¹-2 | 1822 | Formel (I-2) | Ar¹-163 | Ar¹-2 |
| 843 | Formel (I-1) | Ar¹-163 | Ar¹-3 | 1823 | Formel (I-2) | Ar¹-163 | Ar¹-3 |
| 844 | Formel (I-1) | Ar¹-163 | Ar¹-4 | 1824 | Formel (I-2) | Ar¹-163 | Ar¹-4 |
| 845 | Formel (I-1) | Ar¹-163 | Ar¹-59 | 1825 | Formel (I-2) | Ar¹-163 | Ar¹-59 |
| 846 | Formel (I-1) | Ar¹-163 | Ar¹-60 | 1826 | Formel (I-2) | Ar¹-163 | Ar¹-60 |
| 847 | Formel (I-1) | Ar¹-163 | Ar¹-63 | 1827 | Formel (I-2) | Ar¹-163 | Ar¹-63 |
| 848 | Formel (I-1) | Ar¹-163 | Ar¹-65 | 1828 | Formel (I-2) | Ar¹-163 | Ar¹-65 |
| 849 | Formel (I-1) | Ar¹-163 | Ar¹-68 | 1829 | Formel (I-2) | Ar¹-163 | Ar¹-68 |
| 850 | Formel (I-1) | Ar¹-163 | Ar¹-75 | 1830 | Formel (I-2) | Ar¹-163 | Ar¹-75 |
| 851 | Formel (I-1) | Ar¹-163 | Ar¹-87 | 1831 | Formel (I-2) | Ar¹-163 | Ar¹-87 |
| 852 | Formel (I-1) | Ar¹-163 | Ar¹-91 | 1832 | Formel (I-2) | Ar¹-163 | Ar¹-91 |
| 853 | Formel (I-1) | Ar¹-163 | Ar¹-107 | 1833 | Formel (I-2) | Ar¹-163 | Ar¹-107 |
| 854 | Formel (I-1) | Ar¹-163 | Ar¹-109 | 1834 | Formel (I-2) | Ar¹-163 | Ar¹-109 |
| 855 | Formel (I-1) | Ar¹-163 | Ar¹-111 | 1835 | Formel (I-2) | Ar¹-163 | Ar¹-111 |
| 856 | Formel (I-1) | Ar¹-163 | Ar¹-114 | 1836 | Formel (I-2) | Ar¹-163 | Ar¹-114 |
| 857 | Formel (I-1) | Ar¹-163 | Ar¹-121 | 1837 | Formel (I-2) | Ar¹-163 | Ar¹-121 |
| 858 | Formel (I-1) | Ar¹-163 | Ar¹-151 | 1838 | Formel (I-2) | Ar¹-163 | Ar¹-151 |
| 859 | Formel (I-1) | Ar¹-163 | Ar¹-153 | 1839 | Formel (I-2) | Ar¹-163 | Ar¹-153 |
| 860 | Formel (I-1) | Ar¹-163 | Ar¹-162 | 1840 | Formel (I-2) | Ar¹-163 | Ar¹-162 |
| 861 | Formel (I-1) | Ar¹-164 | Ar¹-1 | 1841 | Formel (I-2) | Ar¹-164 | Ar¹-1 |
| 862 | Formel (I-1) | Ar¹-164 | Ar¹-2 | 1842 | Formel (I-2) | Ar¹-164 | Ar¹-2 |
| 863 | Formel (I-1) | Ar¹-164 | Ar¹-3 | 1843 | Formel (I-2) | Ar¹-164 | Ar¹-3 |
| 864 | Formel (I-1) | Ar¹-164 | Ar¹-4 | 1844 | Formel (I-2) | Ar¹-164 | Ar¹-4 |
| 865 | Formel (I-1) | Ar¹-164 | Ar¹-59 | 1845 | Formel (I-2) | Ar¹-164 | Ar¹-59 |
| 866 | Formel (I-1) | Ar¹-164 | Ar¹-60 | 1846 | Formel (I-2) | Ar¹-164 | Ar¹-60 |
| 867 | Formel (I-1) | Ar¹-164 | Ar¹-63 | 1847 | Formel (I-2) | Ar¹-164 | Ar¹-63 |
| 868 | Formel (I-1) | Ar¹-164 | Ar¹-65 | 1848 | Formel (I-2) | Ar¹-164 | Ar¹-65 |
| 869 | Formel (I-1) | Ar¹-164 | Ar¹-68 | 1849 | Formel (I-2) | Ar¹-164 | Ar¹-68 |
| 870 | Formel (I-1) | Ar¹-164 | Ar¹-75 | 1850 | Formel (I-2) | Ar¹-164 | Ar¹-75 |
| 871 | Formel (I-1) | Ar¹-164 | Ar¹-87 | 1851 | Formel (I-2) | Ar¹-164 | Ar¹-87 |
| 872 | Formel (I-1) | Ar¹-164 | Ar¹-91 | 1852 | Formel (I-2) | Ar¹-164 | Ar¹-91 |
| 873 | Formel (I-1) | Ar¹-164 | Ar¹-107 | 1853 | Formel (I-2) | Ar¹-164 | Ar¹-107 |
| 874 | Formel (I-1) | Ar¹-164 | Ar¹-109 | 1854 | Formel (I-2) | Ar¹-164 | Ar¹-109 |
| 875 | Formel (I-1) | Ar¹-164 | Ar¹-111 | 1855 | Formel (I-2) | Ar¹-164 | Ar¹-111 |
| 876 | Formel (I-1) | Ar¹-164 | Ar¹-114 | 1856 | Formel (I-2) | Ar¹-164 | Ar¹-114 |
| 877 | Formel (I-1) | Ar¹-164 | Ar¹-121 | 1857 | Formel (I-2) | Ar¹-164 | Ar¹-121 |
| 878 | Formel (I-1) | Ar¹-164 | Ar¹-151 | 1858 | Formel (I-2) | Ar¹-164 | Ar¹-151 |
| 879 | Formel (I-1) | Ar¹-164 | Ar¹-153 | 1859 | Formel (I-2) | Ar¹-164 | Ar¹-153 |
| 880 | Formel (I-1) | Ar¹-164 | Ar¹-162 | 1860 | Formel (I-2) | Ar¹-164 | Ar¹-162 |
| 881 | Formel (I-1) | Ar¹-165 | Ar¹-1 | 1861 | Formel (I-2) | Ar¹-165 | Ar¹-1 |
| 882 | Formel (I-1) | Ar¹-165 | Ar¹-2 | 1862 | Formel (I-2) | Ar¹-165 | Ar¹-2 |
| 883 | Formel (I-1) | Ar¹-165 | Ar¹-3 | 1863 | Formel (I-2) | Ar¹-165 | Ar¹-3 |
| 884 | Formel (I-1) | Ar¹-165 | Ar¹-4 | 1864 | Formel (I-2) | Ar¹-165 | Ar¹-4 |
| 885 | Formel (I-1) | Ar¹-165 | Ar¹-59 | 1865 | Formel (I-2) | Ar¹-165 | Ar¹-59 |
| 886 | Formel (I-1) | Ar¹-165 | Ar¹-60 | 1866 | Formel (I-2) | Ar¹-165 | Ar¹-60 |
| 887 | Formel (I-1) | Ar¹-165 | Ar¹-63 | 1867 | Formel (I-2) | Ar¹-165 | Ar¹-63 |
| 888 | Formel (I-1) | Ar¹-165 | Ar¹-65 | 1868 | Formel (I-2) | Ar¹-165 | Ar¹-65 |
| 889 | Formel (I-1) | Ar¹-165 | Ar¹-68 | 1869 | Formel (I-2) | Ar¹-165 | Ar¹-68 |
| 890 | Formel (I-1) | Ar¹-165 | Ar¹-75 | 1870 | Formel (I-2) | Ar¹-165 | Ar¹-75 |
| 891 | Formel (I-1) | Ar¹-165 | Ar¹-87 | 1871 | Formel (I-2) | Ar¹-165 | Ar¹-87 |
| 892 | Formel (I-1) | Ar¹-165 | Ar¹-91 | 1872 | Formel (I-2) | Ar¹-165 | Ar¹-91 |
| 893 | Formel (I-1) | Ar¹-165 | Ar¹-107 | 1873 | Formel (I-2) | Ar¹-165 | Ar¹-107 |
| 894 | Formel (I-1) | Ar¹-165 | Ar¹-109 | 1874 | Formel (I-2) | Ar¹-165 | Ar¹-109 |
| 895 | Formel (I-1) | Ar¹-165 | Ar¹-111 | 1875 | Formel (I-2) | Ar¹-165 | Ar¹-111 |
| 896 | Formel (I-1) | Ar¹-165 | Ar¹-114 | 1876 | Formel (I-2) | Ar¹-165 | Ar¹-114 |
| 897 | Formel (I-1) | Ar¹-165 | Ar¹-121 | 1877 | Formel (I-2) | Ar¹-165 | Ar¹-121 |
| 898 | Formel (I-1) | Ar¹-165 | Ar¹-151 | 1878 | Formel (I-2) | Ar¹-165 | Ar¹-151 |
| 899 | Formel (I-1) | Ar¹-165 | Ar¹-153 | 1879 | Formel (I-2) | Ar¹-165 | Ar¹-153 |
| 900 | Formel (I-1) | Ar¹-165 | Ar¹-162 | 1880 | Formel (I-2) | Ar¹-165 | Ar¹-162 |
| 901 | Formel (I-1) | Ar¹-189 | Ar¹-1 | 1881 | Formel (I-2) | Ar¹-189 | Ar¹-1 |
| 902 | Formel (I-1) | Ar¹-189 | Ar¹-2 | 1882 | Formel (I-2) | Ar¹-189 | Ar¹-2 |
| 903 | Formel (I-1) | Ar¹-189 | Ar¹-3 | 1883 | Formel (I-2) | Ar¹-189 | Ar¹-3 |
| 904 | Formel (I-1) | Ar¹-189 | Ar¹-4 | 1884 | Formel (I-2) | Ar¹-189 | Ar¹-4 |
| 905 | Formel (I-1) | Ar¹-189 | Ar¹-59 | 1885 | Formel (I-2) | Ar¹-189 | Ar¹-59 |
| 906 | Formel (I-1) | Ar¹-189 | Ar¹-60 | 1886 | Formel (I-2) | Ar¹-189 | Ar¹-60 |
| 907 | Formel (I-1) | Ar¹-189 | Ar¹-63 | 1887 | Formel (I-2) | Ar¹-189 | Ar¹-63 |
| 908 | Formel (I-1) | Ar¹-189 | Ar¹-65 | 1888 | Formel (I-2) | Ar¹-189 | Ar¹-65 |
| 909 | Formel (I-1) | Ar¹-189 | Ar¹-68 | 1889 | Formel (I-2) | Ar¹-189 | Ar¹-68 |
| 910 | Formel (I-1) | Ar¹-189 | Ar¹-75 | 1890 | Formel (I-2) | Ar¹-189 | Ar¹-75 |
| 911 | Formel (I-1) | Ar¹-189 | Ar¹-87 | 1891 | Formel (I-2) | Ar¹-189 | Ar¹-87 |
| 912 | Formel (I-1) | Ar¹-189 | Ar¹-91 | 1892 | Formel (I-2) | Ar¹-189 | Ar¹-91 |
| 913 | Formel (I-1) | Ar¹-189 | Ar¹-107 | 1893 | Formel (I-2) | Ar¹-189 | Ar¹-107 |
| 914 | Formel (I-1) | Ar¹-189 | Ar¹-109 | 1894 | Formel (I-2) | Ar¹-189 | Ar¹-109 |
| 915 | Formel (I-1) | Ar¹-189 | Ar¹-111 | 1895 | Formel (I-2) | Ar¹-189 | Ar¹-111 |
| 916 | Formel (I-1) | Ar¹-189 | Ar¹-114 | 1896 | Formel (I-2) | Ar¹-189 | Ar¹-114 |
| 917 | Formel (I-1) | Ar¹-189 | Ar¹-121 | 1897 | Formel (I-2 | Ar¹-189 | Ar¹-121 |
| 918 | Formel (I-1) | Ar¹-189 | Ar¹-151 | 1898 | Formel (I-2) | Ar¹-189 | Ar¹-151 |
| 919 | Formel (I-1) | Ar¹-189 | Ar¹-153 | 1899 | Formel (I-2) | Ar¹-189 | Ar¹-153 |
| 920 | Formel (I-1) | Ar¹-189 | Ar¹-162 | 1900 | Formel (I-2) | Ar¹-189 | Ar¹-162 |
| 921 | Formel (I-1) | Ar¹-192 | Ar¹-1 | 1901 | Formel (I-2) | Ar¹-192 | Ar¹-1 |
| 922 | Formel (I-1) | Ar¹-192 | Ar¹-2 | 1902 | Formel (I-2) | Ar¹-192 | Ar¹-2 |
| 923 | Formel (I-1) | Ar¹-192 | Ar¹-3 | 1903 | Formel (I-2) | Ar¹-192 | Ar¹-3 |
| 924 | Formel (I-1) | Ar¹-192 | Ar¹-4 | 1904 | Formel (I-2) | Ar¹-192 | Ar¹-4 |
| 925 | Formel (I-1) | Ar¹-192 | Ar¹-59 | 1905 | Formel (I-2) | Ar¹-192 | Ar¹-59 |
| 926 | Formel (I-1) | Ar¹-192 | Ar¹-60 | 1906 | Formel (I-2) | Ar¹-192 | Ar¹-60 |
| 927 | Formel (I-1) | Ar¹-192 | Ar¹-63 | 1907 | Formel (I-2) | Ar¹-192 | Ar¹-63 |
| 928 | Formel (I-1) | Ar¹-192 | Ar¹-65 | 1908 | Formel (I-2) | Ar¹-192 | Ar¹-65 |
| 929 | Formel (I-1) | Ar¹-192 | Ar¹-68 | 1909 | Formel (I-2) | Ar¹-192 | Ar¹-68 |
| 930 | Formel (I-1) | Ar¹-192 | Ar¹-75 | 1910 | Formel (I-2) | Ar¹-192 | Ar¹-75 |
| 931 | Formel (I-1) | Ar¹-192 | Ar¹-87 | 1911 | Formel (I-2) | Ar¹-192 | Ar¹-87 |
| 932 | Formel (I-1) | Ar¹-192 | Ar¹-91 | 1912 | Formel (I-2) | Ar¹-192 | Ar¹-91 |
| 933 | Formel (I-1) | Ar¹-192 | Ar¹-107 | 1913 | Formel (I-2) | Ar¹-192 | Ar¹-107 |
| 934 | Formel (I-1) | Ar¹-192 | Ar¹-109 | 1914 | Formel (I-2) | Ar¹-192 | Ar¹-109 |
| 935 | Formel (I-1) | Ar¹-192 | Ar¹-111 | 1915 | Formel (I-2) | Ar¹-192 | Ar¹-111 |
| 936 | Formel (I-1) | Ar¹-192 | Ar¹-114 | 1916 | Formel (I-2) | Ar¹-192 | Ar¹-114 |
| 937 | Formel (I-1) | Ar¹-192 | Ar¹-121 | 1917 | Formel (I-2) | Ar¹-192 | Ar¹-121 |
| 938 | Formel (I-1) | Ar¹-192 | Ar¹-151 | 1918 | Formel (I-2) | Ar¹-192 | Ar¹-151 |
| 939 | Formel (I-1) | Ar¹-192 | Ar¹-153 | 1919 | Formel (I-2) | Ar¹-192 | Ar¹-153 |
| 940 | Formel (I-1) | Ar¹-192 | Ar¹-162 | 1920 | Formel (I-2) | Ar¹-192 | Ar¹-162 |
| 941 | Formel (I-1) | Ar¹-234 | Ar¹-1 | 1921 | Formel (I-2) | Ar¹-234 | Ar¹-1 |
| 942 | Formel (I-1) | Ar¹-234 | Ar¹-2 | 1922 | Formel (I-2) | Ar¹-234 | Ar¹-2 |
| 943 | Formel (I-1) | Ar¹-234 | Ar¹-3 | 1923 | Formel (I-2) | Ar¹-234 | Ar¹-3 |
| 944 | Formel (I-1) | Ar¹-234 | Ar¹-4 | 1924 | Formel (I-2) | Ar¹-234 | Ar¹-4 |
| 945 | Formel (I-1) | Ar¹-234 | Ar¹-59 | 1925 | Formel (I-2) | Ar¹-234 | Ar¹-59 |
| 946 | Formel (I-1) | Ar¹-234 | Ar¹-60 | 1926 | Formel (I-2) | Ar¹-234 | Ar¹-60 |
| 947 | Formel (I-1) | Ar¹-234 | Ar¹-63 | 1927 | Formel (I-2) | Ar¹-234 | Ar¹-63 |
| 948 | Formel (I-1) | Ar¹-234 | Ar¹-65 | 1928 | Formel (I-2) | Ar¹-234 | Ar¹-65 |
| 949 | Formel (I-1) | Ar¹-234 | Ar¹-68 | 1929 | Formel (I-2) | Ar¹-234 | Ar¹-68 |
| 950 | Formel (I-1) | Ar¹-234 | Ar¹-75 | 1930 | Formel (I-2) | Ar¹-234 | Ar¹-75 |
| 951 | Formel (I-1) | Ar¹-234 | Ar¹-87 | 1931 | Formel (I-2) | Ar¹-234 | Ar¹-87 |
| 952 | Formel (I-1) | Ar¹-234 | Ar¹-91 | 1932 | Formel (I-2) | Ar¹-234 | Ar¹-91 |
| 953 | Formel (I-1) | Ar¹-234 | Ar¹-107 | 1933 | Formel (I-2) | Ar¹-234 | Ar¹-107 |
| 954 | Formel (I-1) | Ar¹-234 | Ar¹-109 | 1934 | Formel (I-2) | Ar¹-234 | Ar¹-109 |
| 955 | Formel (I-1) | Ar¹-234 | Ar¹-111 | 1935 | Formel (I-2) | Ar¹-234 | Ar¹-111 |
| 956 | Formel (I-1) | Ar¹-234 | Ar¹-114 | 1936 | Formel (I-2) | Ar¹-234 | Ar¹-114 |
| 957 | Formel (I-1) | Ar¹-234 | Ar¹-121 | 1937 | Formel (I-2) | Ar¹-234 | Ar¹-121 |
| 958 | Formel (I-1) | Ar¹-234 | Ar¹-151 | 1938 | Formel (I-2) | Ar¹-234 | Ar¹-151 |
| 959 | Formel (I-1) | Ar¹-234 | Ar¹-153 | 1939 | Formel (I-2) | Ar¹-234 | Ar¹-153 |
| 960 | Formel (I-1) | Ar¹-234 | Ar¹-162 | 1940 | Formel (I-2) | Ar¹-234 | Ar¹-162 |
| 961 | Formel (I-1) | Ar¹-237 | Ar¹-1 | 1941 | Formel (I-2) | Ar¹-237 | Ar¹-1 |
| 962 | Formel (I-1) | Ar¹-237 | Ar¹-2 | 1942 | Formel (I-2) | Ar¹-237 | Ar¹-2 |
| 963 | Formel (I-1) | Ar¹-237 | Ar¹-3 | 1943 | Formel (I-2) | Ar¹-237 | Ar¹-3 |
| 964 | Formel (I-1) | Ar¹-237 | Ar¹-4 | 1944 | Formel (I-2) | Ar¹-237 | Ar¹-4 |
| 965 | Formel (I-1) | Ar¹-237 | Ar¹-59 | 1945 | Formel (I-2) | Ar¹-237 | Ar¹-59 |
| 966 | Formel (I-1) | Ar¹-237 | Ar¹-60 | 1946 | Formel (I-2) | Ar¹-237 | Ar¹-60 |
| 967 | Formel (I-1) | Ar¹-237 | Ar¹-63 | 1947 | Formel (I-2) | Ar¹-237 | Ar¹-63 |
| 968 | Formel (I-1) | Ar¹-237 | Ar¹-65 | 1948 | Formel (I-2) | Ar¹-237 | Ar¹-65 |
| 969 | Formel (I-1) | Ar¹-237 | Ar¹-68 | 1949 | Formel (I-2) | Ar¹-237 | Ar¹-68 |
| 970 | Formel (I-1) | Ar¹-237 | Ar¹-75 | 1950 | Formel (I-2) | Ar¹-237 | Ar¹-75 |
| 971 | Formel (I-1) | Ar¹-237 | Ar¹-87 | 1951 | Formel (I-2) | Ar¹-237 | Ar¹-87 |
| 972 | Formel (I-1) | Ar¹-237 | Ar¹-91 | 1952 | Formel (I-2) | Ar¹-237 | Ar¹-91 |
| 973 | Formel (I-1) | Ar¹-237 | Ar¹-107 | 1953 | Formel (I-2) | Ar¹-237 | Ar¹-107 |
| 974 | Formel (I-1) | Ar¹-237 | Ar¹-109 | 1954 | Formel (I-2) | Ar¹-237 | Ar¹-109 |
| 975 | Formel (I-1) | Ar¹-237 | Ar¹-111 | 1955 | Formel (I-2) | Ar¹-237 | Ar¹-111 |
| 976 | Formel (I-1) | Ar¹-237 | Ar¹-114 | 1956 | Formel (I-2) | Ar¹-237 | Ar¹-114 |
| 977 | Formel (I-1) | Ar¹-237 | Ar¹-121 | 1957 | Formel (I-2) | Ar¹-237 | Ar¹-121 |
| 978 | Formel (I-1) | Ar¹-237 | Ar¹-151 | 1958 | Formel (I-2) | Ar¹-237 | Ar¹-151 |
| 979 | Formel (I-1) | Ar¹-237 | Ar¹-153 | 1959 | Formel (I-2) | Ar¹-237 | Ar¹-153 |
| 980 | Formel (I-1) | Ar¹-237 | Ar¹-162 | 1960 | Formel (I-2) | Ar¹-237 | Ar¹-162 |

Weitere bevorzugte Verbindungen entsprechen den oben gezeigten Verbindungen 1 bis 1960, wobei anstelle der Formel (I-1) bzw. Formel (I-2) entsprechend eine Formel (I-3) steht.

Nochmals weitere bevorzugte Verbindungen entsprechen den oben gezeigten Verbindungen 1 bis 1960, wobei anstelle der Formel (I-1) bzw. Formel (I-2) entsprechend eine Formel (I-4) steht.

Weitere Beispiele für Verbindungen gemäß Formel (I) sind im Folgenden abgebildet:

| | | |
|---|---|---|
| 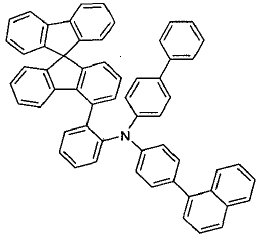 | 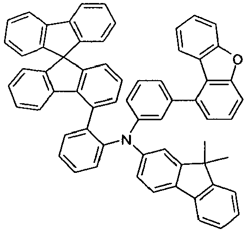 | 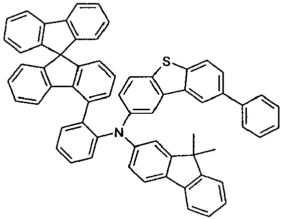 |
| 1 | 2 | 3 |
| 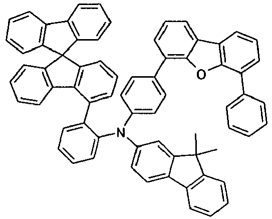 | 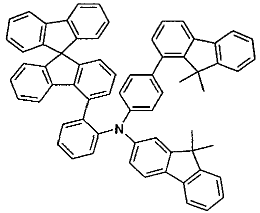 | 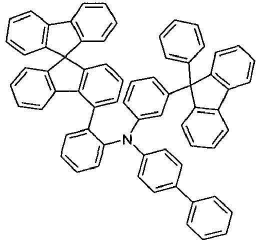 |
| 4 | 5 | 6 |
| 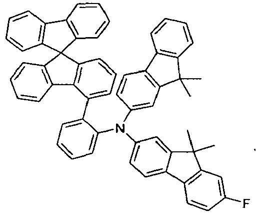 | 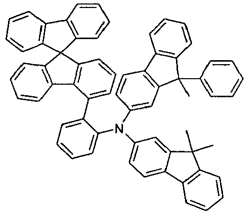 | 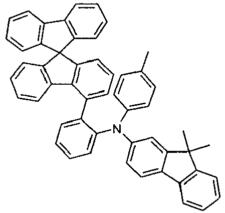 |
| 7 | 8 | 9 |
| 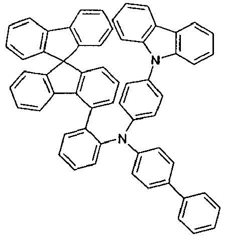 | 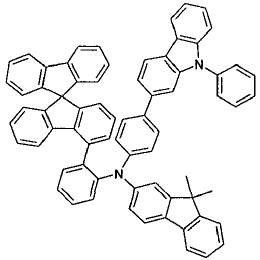 | 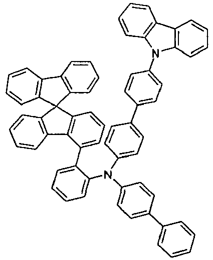 |
| 10 | 11 | 12 |
| 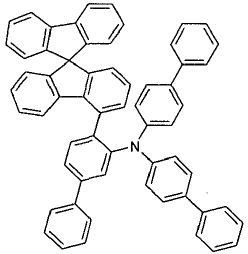 | 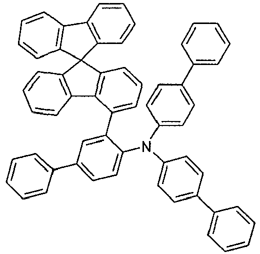 | 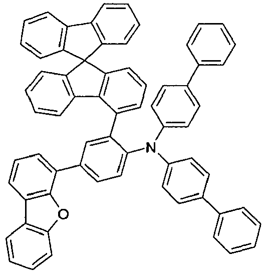 |
| 13 | 14 | 15 |
| 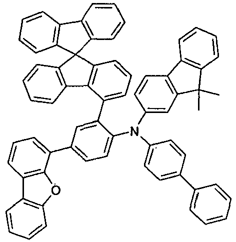 | 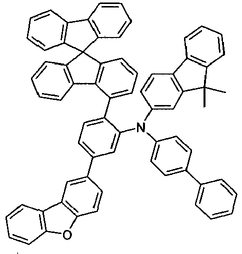 | 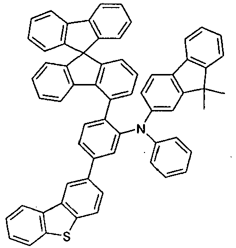 |
| 16 | 17 | 18 |
| 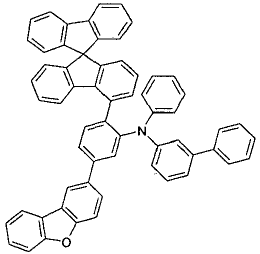 | 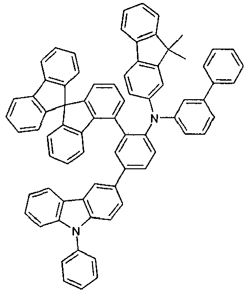 | 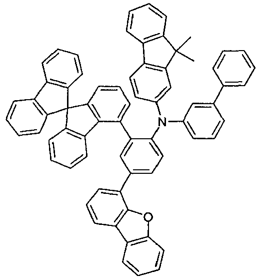 |
| 19 | 20 | 21 |
| 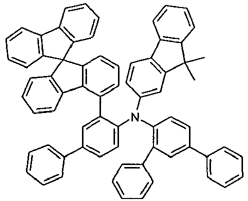 | 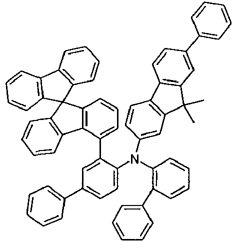 | 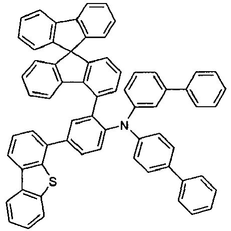 |
| 22 | 23 | 24 |
| 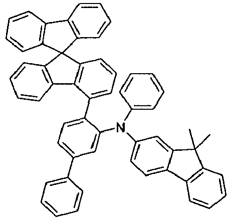 | 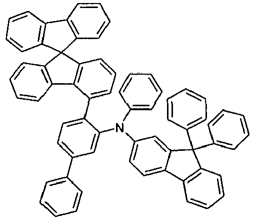 | 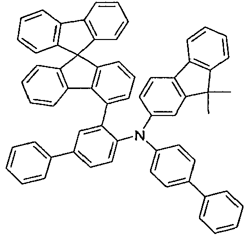 |
| 25 | 26 | 27 |
| 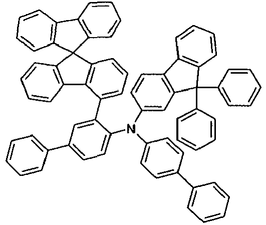 | 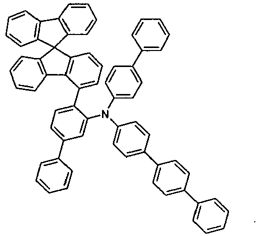 | 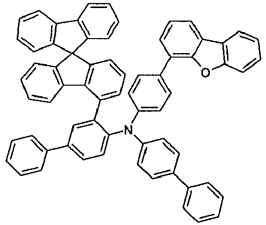 |
| 28 | 29 | 30 |
| 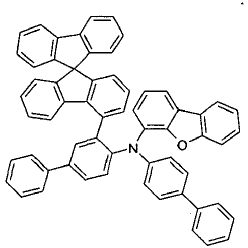 | 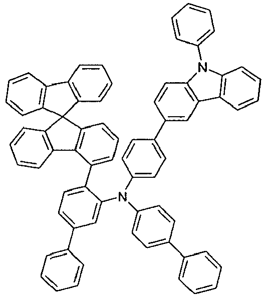 | 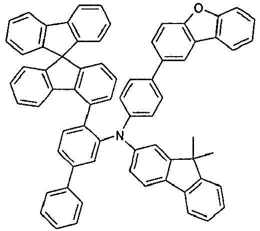 |
| 31 | 32 | 33 |
| 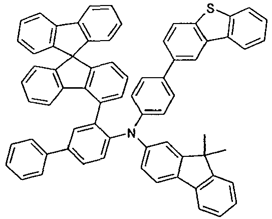 | 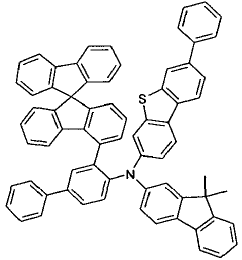 | 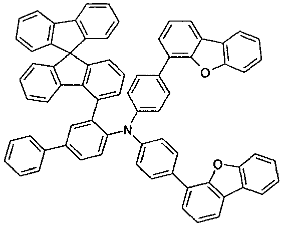 |
| 34 | 35 | 36 |
| 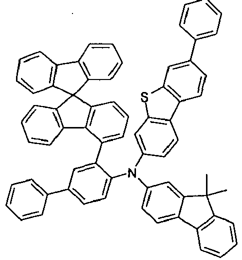 | 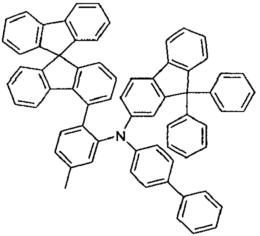 | 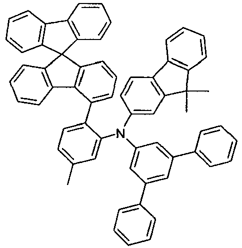 |
| 37 | 38 | 39 |
| 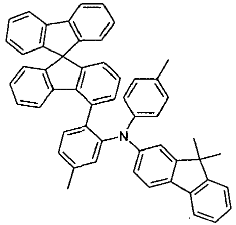 | 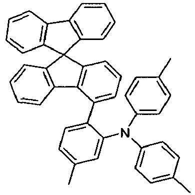 | 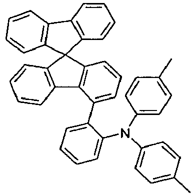 |
| 40 | 41 | 42 |
| 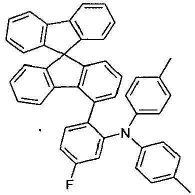 | 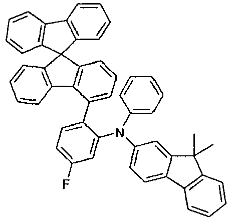 | 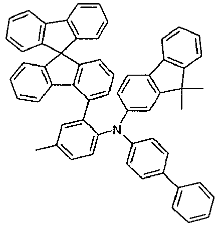 |
| 43 | 44 | 45 |
| 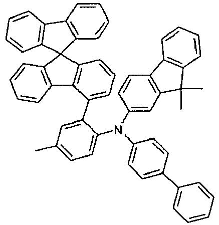 | 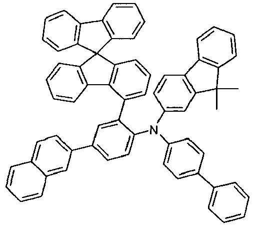 | 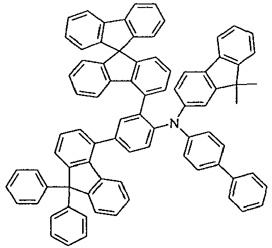 |
| 46 | 47 | 48 |
| 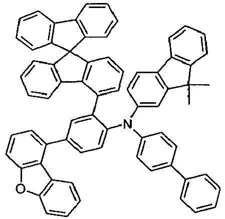 | 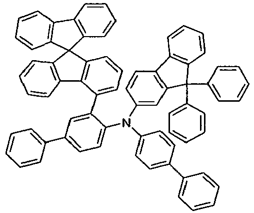 | 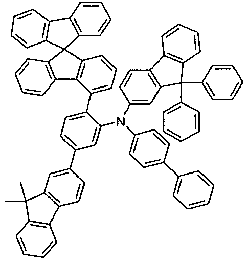 |
| 49 | 50 | 51 |
| 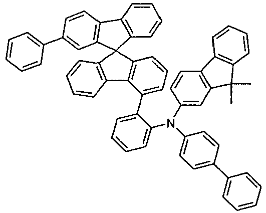 | 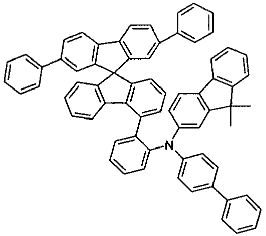 | 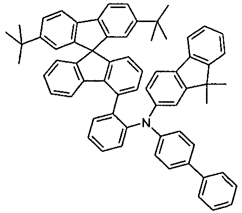 |
| 52 | 53 | 54 |
| 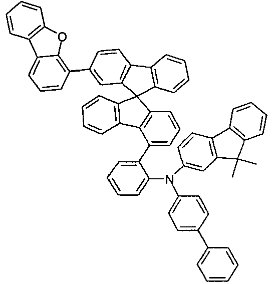 | 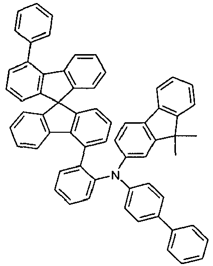 | 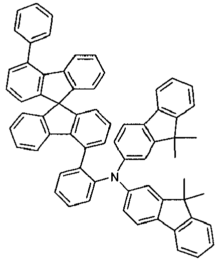 |
| 55 | 56 | 57 |
| 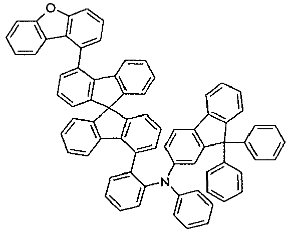 | 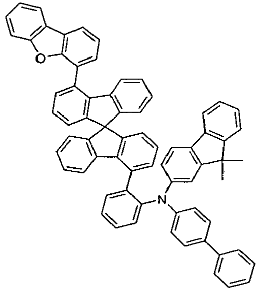 | 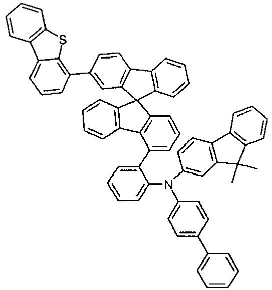 |
| 58 | 59 | 60 |
| 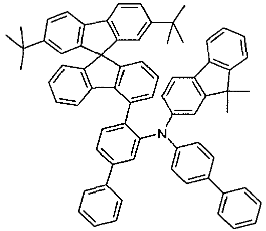 | 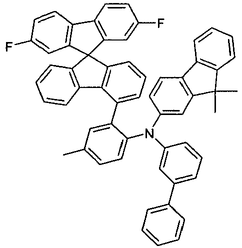 | 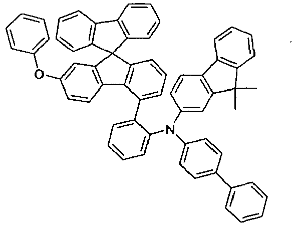 |
| 61 | 62 | 63 |
| 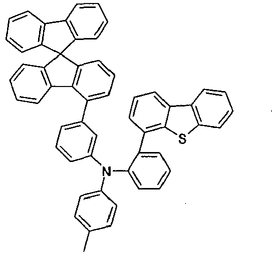 | 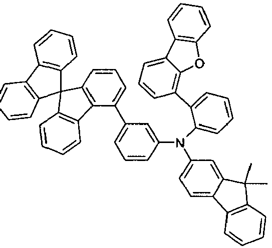 | 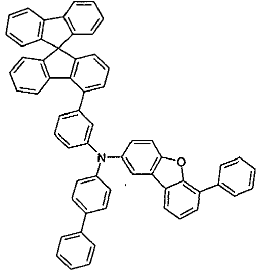 |
| 64 | 65 | 66 |
| 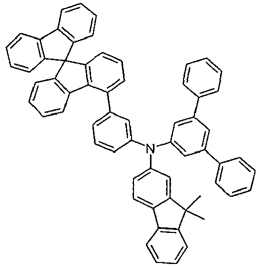 | 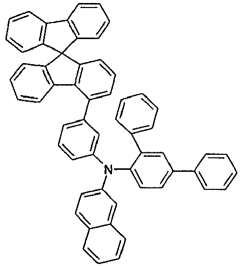 | 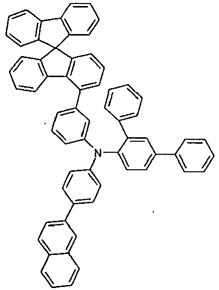 |
| 67 | 68 | 69 |
| 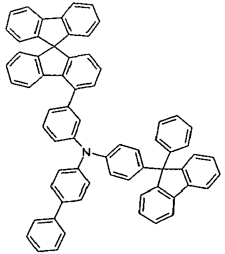 | 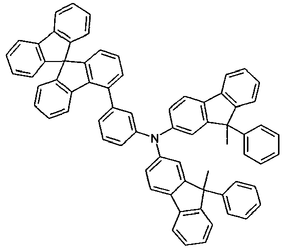 | 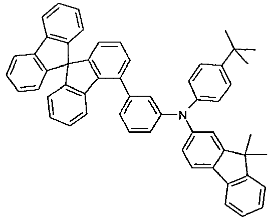 |
| 70 | 71 | 72 |
| 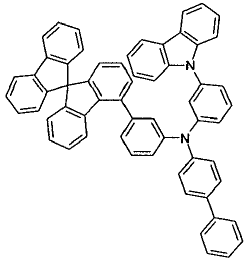 | 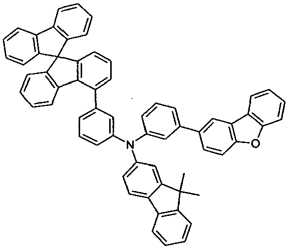 | 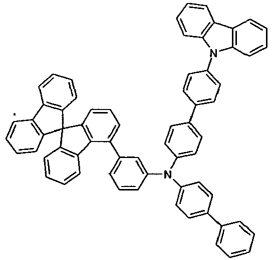 |
| 73 | 74 | 75 |
| 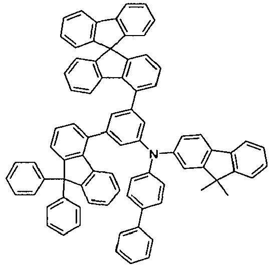 | 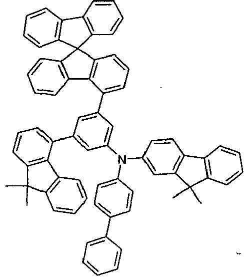 | 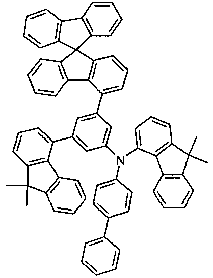 |
| 76 | 77 | 78 |
| 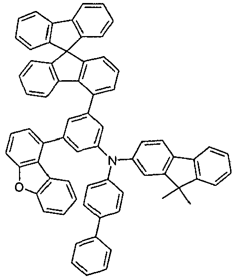 | 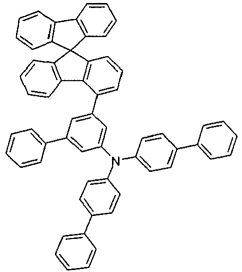 | 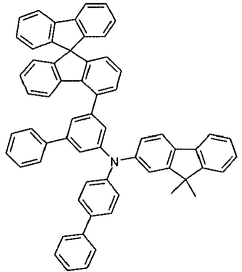 |
| 79 | 80 | 81 |
| 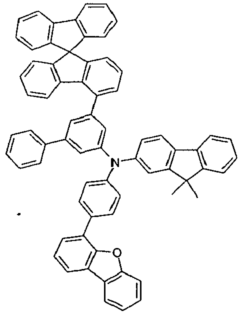 | 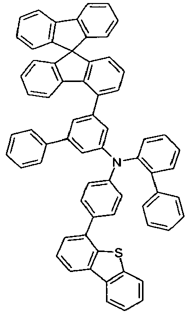 | 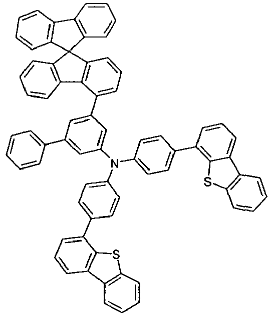 |
| 82 | 83 | 84 |
| 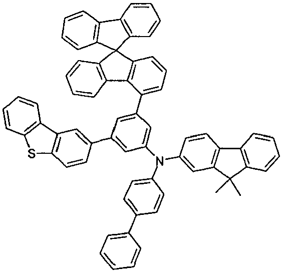 | 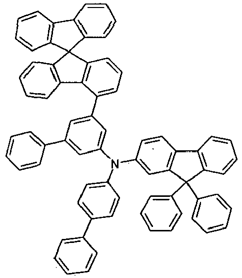 | 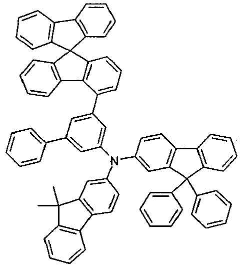 |
| 85 | 86 | 87 |
| 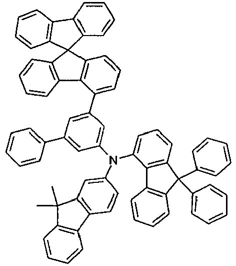 | 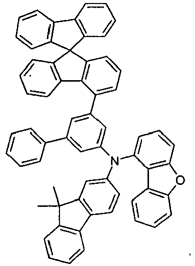 | 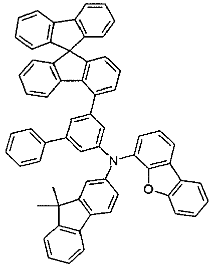 |
| 88 | 89 | 90 |
| 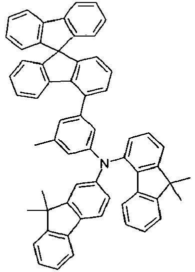 | 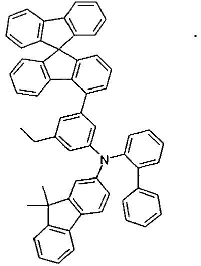 | 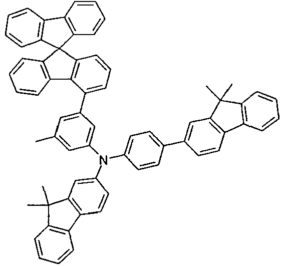 |
| 91 | 92 | 93 |
| 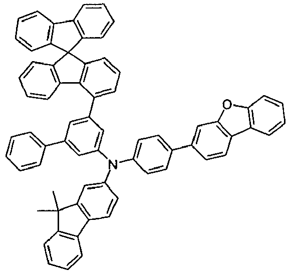 | 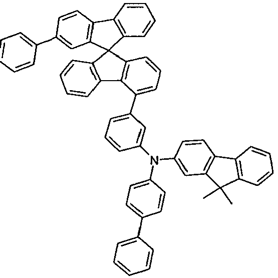 | 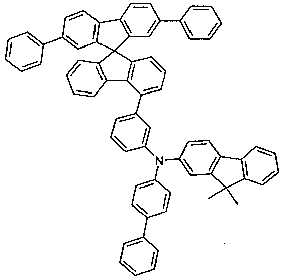 |
| 94 | 95 | 96 |
| 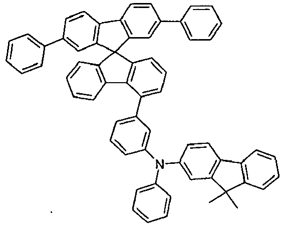 | 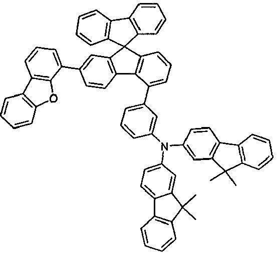 | 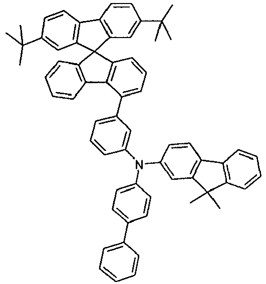 |
| 97 | 98 | 99 |
| 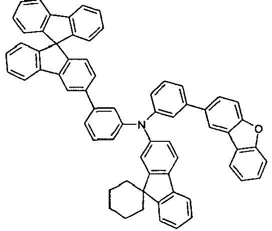 | 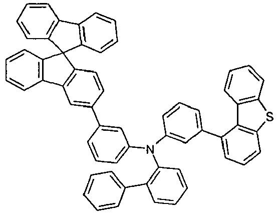 | 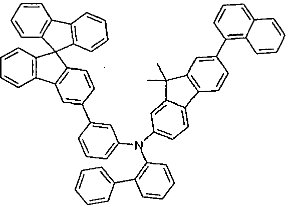 |
| 100 | 101 | 102 |
| 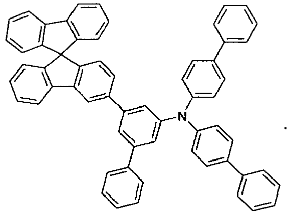 | 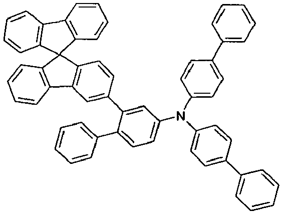 | 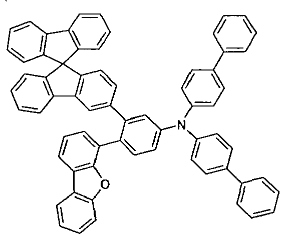 |
| 103 | 104 | 105 |
| 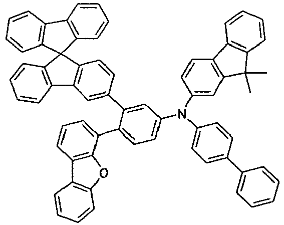 | 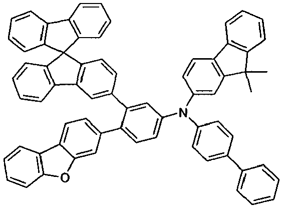 | 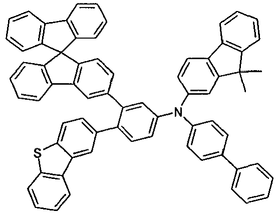 |
| 106 | 107 | 108 |
| 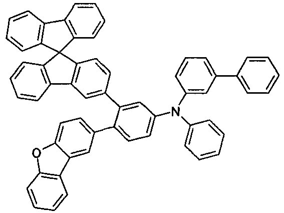 | 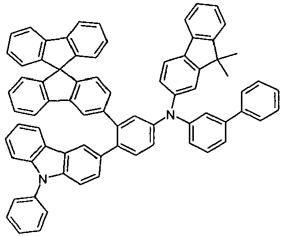 | 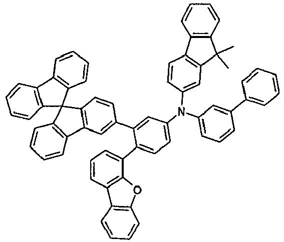 |
| 109 | 110 | 111 |
| 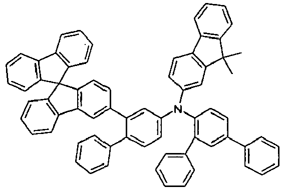 | 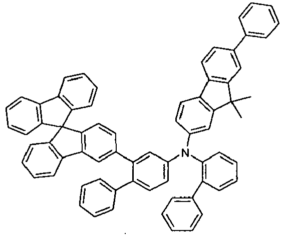 | 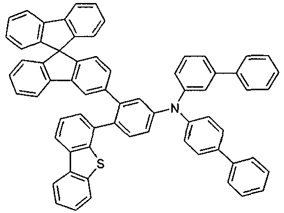 |
| 112 | 113 | 114 |
| 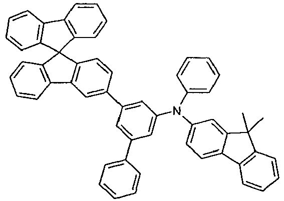 | 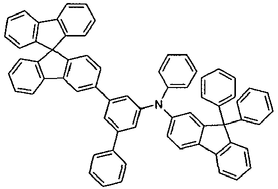 | 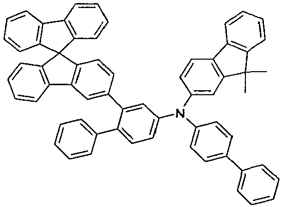 |
| 115 | 116 | 117 |
| 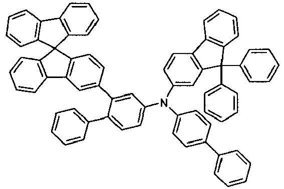 | 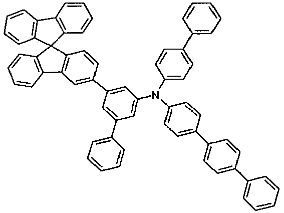 | 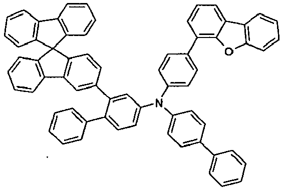 |
| 118 | 119 | 120 |
| 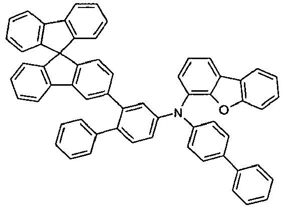 | 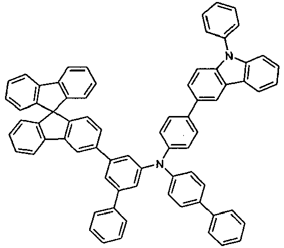 | 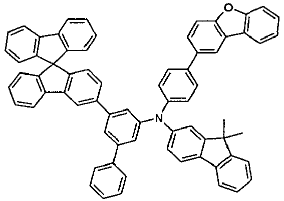 |
| 121 | 122 | 123 |
| 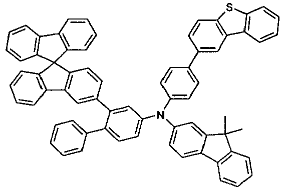 | 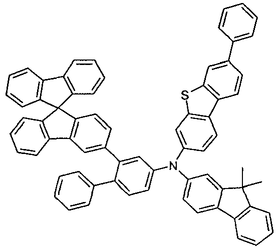 | 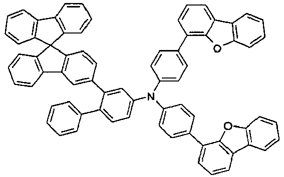 |
| 124 | 125 | 126 |
| 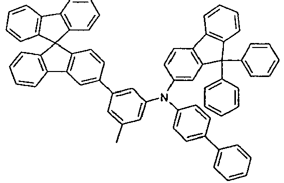 | 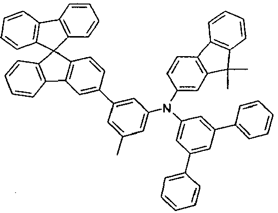 | 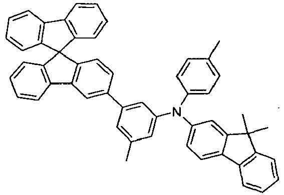 |
| 127 | 128 | 129 |
| 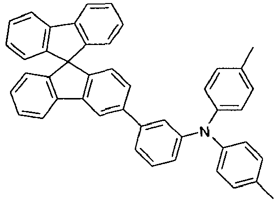 | 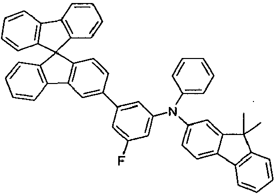 | 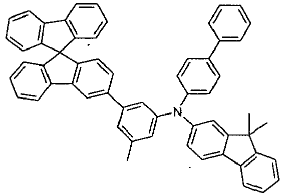 |
| 130 | 131 | 132 |
| 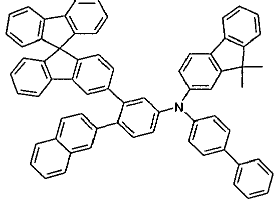 | 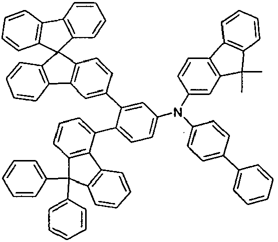 | 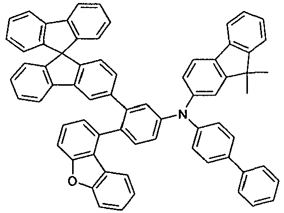 |
| 133 | 134 | 135 |
| 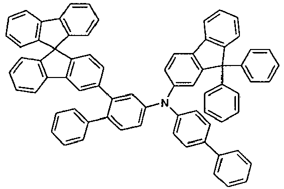 | 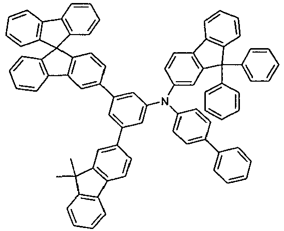 | 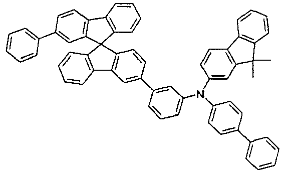 |
| 136 | 137 | 138 |
| 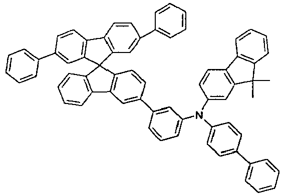 | 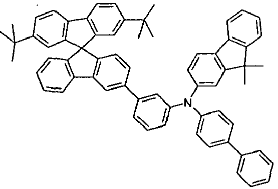 | 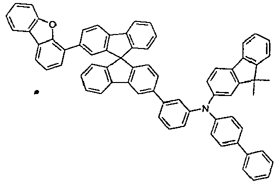 |
| 139 | 140 | 141 |
| 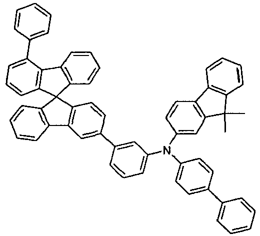 | 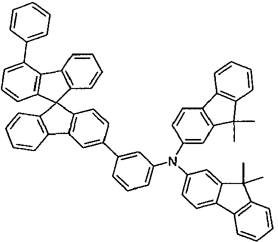 | 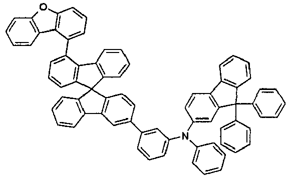 |
| 142 | 143 | 144 |
| 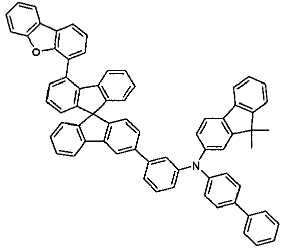 | 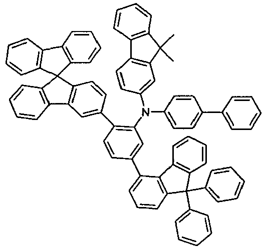 | 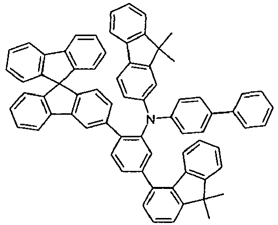 |
| 145 | 146 | 147 |
| 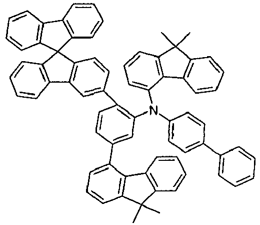 | 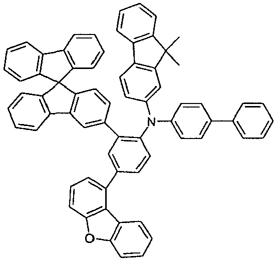 | 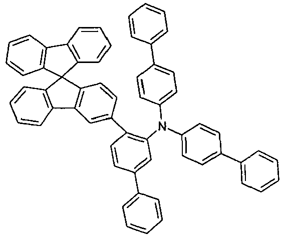 |
| 148 | 149 | 150 |
| 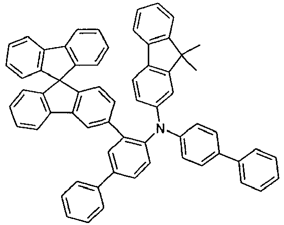 | 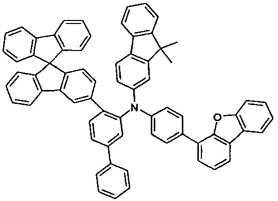 | 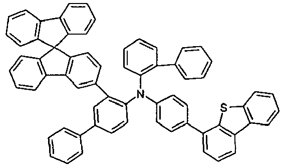 |
| 151 | 152 | 153 |
| 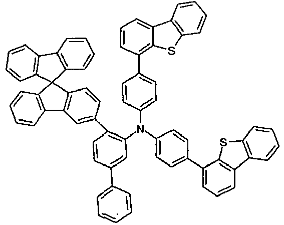 | 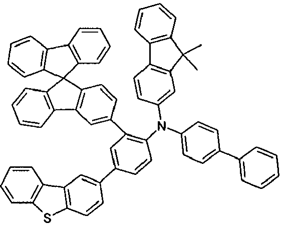 | 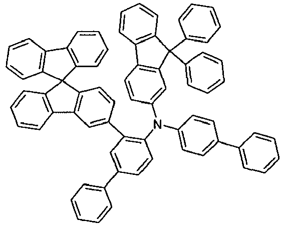 |
| 154 | 155 | 156 |
| 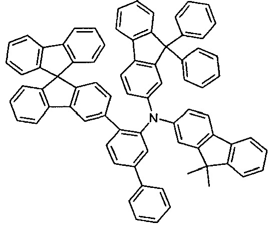 | 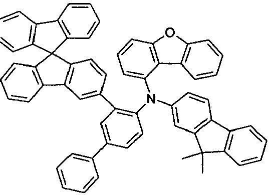 | 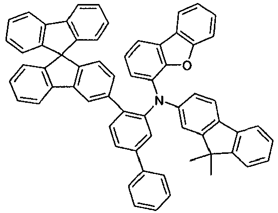 |
| 157 | 158 | 159 |
| 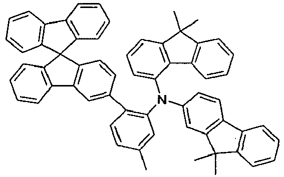 | 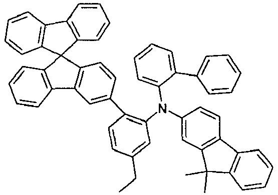 | 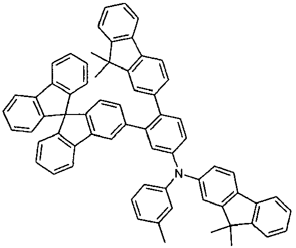 |
| 160 | 161 | 162 |
| 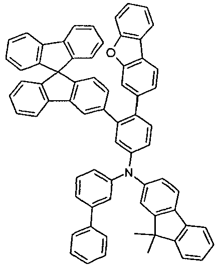 | 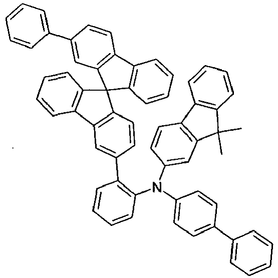 | 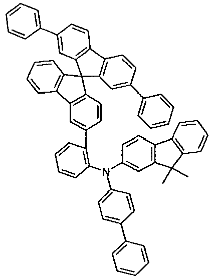 |
| 163 | 164 | 165 |
| 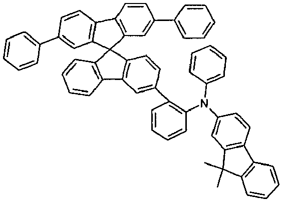 | 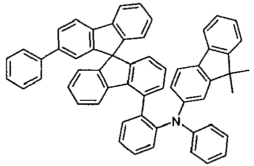 | 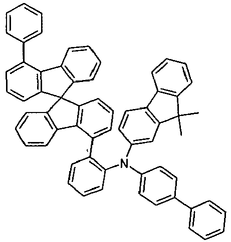 |
| 166 | 167 | 168 |
| 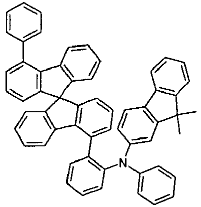 | 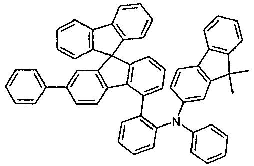 | 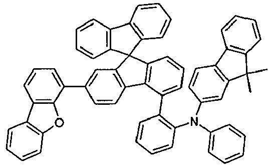 |
| 169 | 170 | 171 |
| 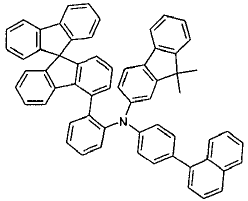 | 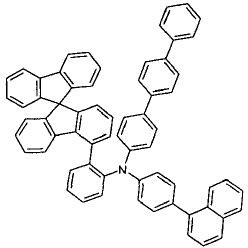 | 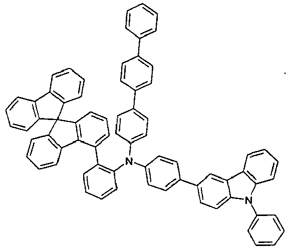 |
| 172 | 173 | 174 |
| 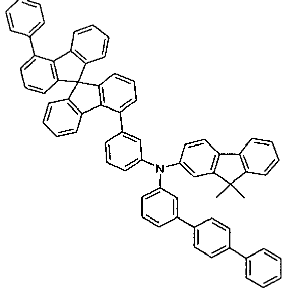 | 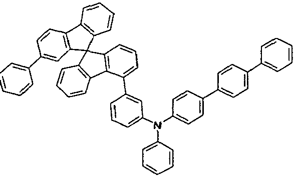 | 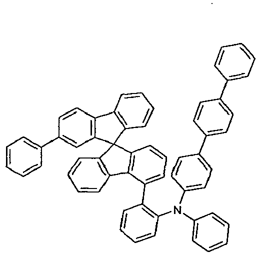 |
| 175 | 176 | 177 |

Die Verbindungen können gemäß dem Fachmann bekannten Verfahren der organischen Synthese hergestellt werden. Dazu zählen insbesondere Halogenierungsreaktionen und metallkatalysierte Kupplungsreaktionen, darunter insbesondere Buchwald-Kupplungsreaktionen und Suzuki-Kupplungsreaktionen.

Gemäß einem bevorzugten Verfahren zur Herstellung der erfindungsgemäßen Verbindungen (s. folgendes Schema 1) wird an eine Spirobifluoreneinheit, die in entsprechender Position 3 oder 4 mit einer reaktiven Gruppe, bevorzugt einem Brom- oder lodatom substitutiert ist, über eine Suzuki-Kupplung ein Benzolring angebunden. Bevorzugt ist dieser bereits mit einer weiteren reaktiven Gruppe substituiert. Alternativ kann diese weitere reaktive Gruppe in einer Funktionalisierungsreaktion eingeführt werden. Im Anschluss wird an dieser weiteren reaktiven Gruppe eine Buchwald-Kupplung mit einem entsprechenden Amin durchgeführt. Dadurch kann die erfindungsgemäße Verbindung erhalten werden. An die Kupplungsreaktion können sich optional weitere Funktionalisierungsschritte anschließen.

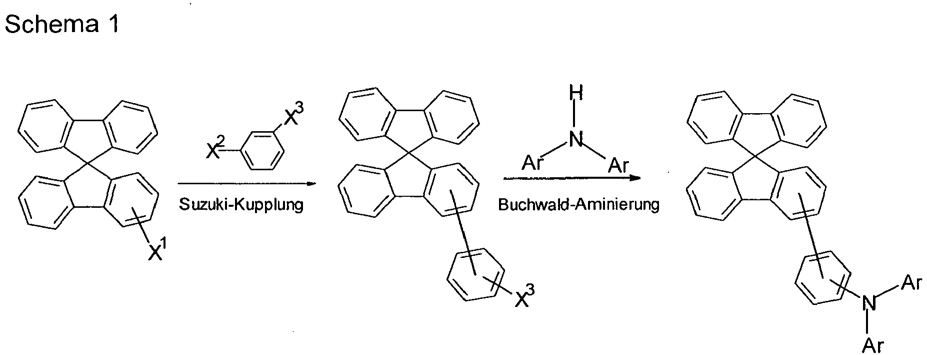

Die unsubstituiert gezeichneten Positionen in Schema 1 können jeweils mit einem organischen Rest substituiert sein. Die variablen Gruppen in Schema 1 sind wie folgt definiert:
X¹ = reaktive Gruppe, bevorzugt Br oder I
X² = reaktive Gruppe, bevorzugt -B(OR)₂
X³ = reaktive Gruppe, bevorzugt Cl
Ar = aromatisches oder heteroaromatisches Ringsystem

Alternativ können die erfindungsgemäßen Verbindungen hergestellt werden, indem in einem ersten Schritt eine zweifach mit reaktiven Gruppen substituierte Benzolverbindung zunächst an der einen reaktiven Gruppe in einer Buchwald-Reaktion zu einer Benzolverbindung, die eine reaktive Gruppe und eine Aminogruppe trägt, umgesetzt wird. In einem zweiten Schritt wird dann in einer Suzuki-Reaktion eine Spirobifluoren-Einheit an die verbleibende reaktive Gruppe der Benzolverbindung gekoppelt (Schema 2).

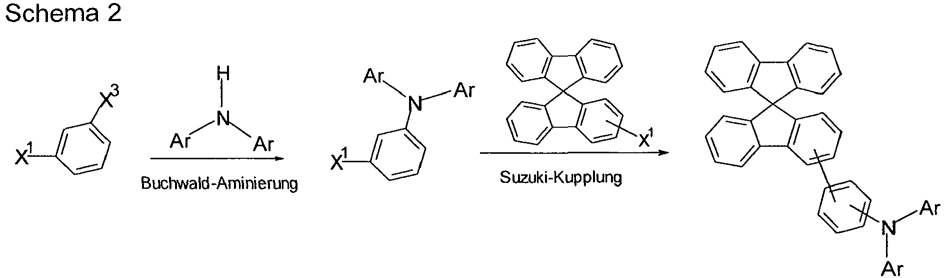

Die unsubstituiert gezeichneten Positionen in Schema 2 können jeweils mit einem organischen Rest substituiert sein. Die variablen Gruppen in Schema 2 sind definiert wie für Schema 1.

Gegenstand der vorliegenden Erfindung sind ist damit ein Verfahren zur Herstellung einer Verbindung gemäß Formel (I), dadurch gekennzeichnet, dass zunächst ein Spirobifluoren, welches eine reaktive Gruppe trägt, in einer ersten metallkatalysierten Kupplungsreaktion mit einem Benzolderivat umgesetzt wird, welches zwei reaktive Gruppen enthält, von denen eine in dieser Kupplungsreaktion umgesetzt wird, und wobei schließlich eine Aminogruppe über eine zweite metallorganische Kupplungsreaktion an der anderen der zwei reaktiven Gruppen in die Verbindung eingeführt wird.

Die erste metallkatalysierte Kupplungsreaktion ist bevorzugt eine Suzuki-Kupplungsreaktion. Die zweite metallkatalysierte Kupplungsreaktion ist bevorzugt eine Buchwald-Kupplungsreaktion. Weiterhin ist die reaktive Gruppe am Spirobifluoren bevorzugt ein Halogenatom, besonders bevorzugt ein Br-Atom. Nochmals weiterhin sind die reaktiven Gruppen am Benzolderivat bevorzugt gewählt aus Halogenatomen, besonders bevorzugt Chlor, und aus Boronsäurederivaten.

Weiterer Gegenstand der Erfindung ist ein alternatives Verfahren zur Herstellung einer Verbindung gemäß Formel (I), dadurch gekennzeichnet, dass eine Benzolverbindung, welche zwei reaktive Gruppen trägt, in einer ersten metallkatalysierten Kupplungsreaktion an der einen der zwei reaktiven Gruppen mit einer Aminoverbindung umgesetzt wird, und in einer zweiten metallkatalysierten Kupplungsreaktion an der anderen der zwei reaktiven Gruppen mit einem Spirobifluoren-Derivat umgesetzt wird.

Die erste metallkatalysierte Kupplungsreaktion ist bevorzugt eine Buchwald-Kupplungsreaktion. Die zweite metallkatalysierte Kupplungsreaktion ist bevorzugt eine Suzuki-Kupplungsreaktion. Weiterhin ist die reaktive Gruppe am Spirobifluoren bevorzugt ein Halogenatom, besonders bevorzugt ein Br-Atom. Nochmals weiterhin sind die reaktiven Gruppen am Benzolderivat bevorzugt gewählt aus Halogenatomen, besonders bevorzugt Chlor, und aus Boronsäurederivaten.

Die oben beschriebenen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere, enthaltend eine oder mehrere Verbindungen gemäß Formel (I), wobei die Bindung(en) zum Polymer, Oligomer oder Dendrimer an beliebigen, in Formel (I) mit R¹, R² oder R³ substituierten Positionen lokalisiert sein können. Je nach Verknüpfung der Verbindung gemäß Formel (I) ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nicht-konjugiert sein. Die erfindungsgemäßen Oligomere oder Polymere können linear, verzweigt oder dendritisch sein. In den linear verknüpften Strukturen können die Einheiten gemäß Formel (I) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formel (I) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (I) in Oligomeren, Dendrimeren und Polymeren gelten dieselben Bevorzugungen wie oben für Verbindungen gemäß Formel (I) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 2000/22026), Spirobifluorenen (z. B. gemäß EP 707020, EP 894107 oder WO 2006/061181), Paraphenylenen (z. B. gemäß WO 1992/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (I) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind die Suzuki-Polymerisation, die Yamamoto-Polymerisation; die Stille-Polymerisation; und die Hartwig-Buchwald-Polymerisation.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Gegenstand der Erfindung ist daher weiterhin eine Formulierung, insbesondere eine Lösung, Dispersion oder Emulsion, enthaltend mindestens eine Verbindung gemäß Formel (I) sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Die erfindungsgemäßen Verbindungen eignen sich für den Einsatz in elektronischen Vorrichtungen, insbesondere in organischen Elektrolumineszenzvorrichtungen (OLEDs). Abhängig von der Substitution werden die Verbindungen in unterschiedlichen Funktionen und Schichten eingesetzt.

Weiterer Gegenstand der Erfindung ist daher die Verwendung der Verbindung gemäß Formel (I) in einer elektronischen Vorrichtung. Dabei ist die elektronische Vorrichtung bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen Elektrolumineszenzvorrichtungen (OLEDs).

Weiterer Gegenstand der Erfindung ist, wie bereits oben ausgeführt, eine elektronische Vorrichtung, enthaltend mindestens eine Verbindung gemäß Formel (I). Dabei ist die elektronische Vorrichtung bevorzugt ausgewählt aus den oben genannten Vorrichtungen.

Besonders bevorzugt ist sie eine organische Elektrolumineszenzvorrichtung (OLED), enthaltend Anode, Kathode und mindestens eine emittierende Schicht, dadurch gekennzeichnet, dass mindestens eine organische Schicht, die eine emittierende Schicht, eine Lochtransportschicht oder eine andere Schicht sein kann, mindestens eine Verbindung gemäß Formel (I) enthält.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen.

Die Abfolge der Schichten der organischen Elektrolumineszenzvorrichtung enthaltend die Verbindung der Formel (I) ist bevorzugt die folgende: Anode-Lochinjektionsschicht-Lochtransportschicht-wahlweise weitere Lochtransportschicht(en)-wahlweise Elektronenblockierschicht-emittierende Schicht-wahlweise Lochblockierschicht-Elektronentransportschicht-Elektroneninjektionsschicht-Kathode. Es können zusätzlich weitere Schichten in der OLED vorhanden sein.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues, grünes, gelbes, orangefarbenes oder rotes Licht emittieren. Insbesondere bevorzugt sind Dreischichtsysteme, also Systeme mit drei emittierenden Schichten, wobei die drei Schichten blaue, grüne und orangefarbene oder rote Emission zeigen (für den prinzipiellen Aufbau siehe z. B. WO 2005/011013). Die erfindungsgemäßen Verbindungen sind dabei bevorzugt in der Lochtransportschicht, Lochinjektionsschicht oder der Elektronenblockierschicht vorhanden.

Es ist erfindungsgemäß bevorzugt, wenn die Verbindung gemäß Formel (I) in einer elektronischen Vorrichtung enthaltend einen oder mehrere phosphoreszierende emittierende Verbindungen eingesetzt wird. Dabei kann die Verbindung in unterschiedlichen Schichten, bevorzugt in einer Lochtransportschicht, einer Elektronenblockierschicht, einer Lochinjektionsschicht oder in einer emittierenden Schicht, enthalten sein.

Vom Begriff phosphoreszierende emittierende Verbindungen sind typischerweise Verbindungen umfasst, bei denen die Lichtemission durch einen spin-verbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende emittierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende emittierende Verbindungen Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten. Dabei werden im Sinne der vorliegenden Erfindung alle lumineszierenden Iridium-, Platin- oder Kupferkomplexe als phosphoreszierende emittierende Verbindungen angesehen.

Beispiele für die oben beschriebenen emittierenden Verbindungen können den Anmeldungen WO 00/70655, WO 01/41512, WO 02/02714, WO 02/15645, EP 1191613, EP 1191612, EP 1191614, WO 05/033244, WO 05/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind. Auch kann der Fachmann ohne erfinderisches Zutun weitere phosphoreszierende Komplexe in Kombination mit den Verbindungen gemäß Formel (I) in organischen Elektrolumineszenzvorrichtungen einsetzen. Weitere Beispiele sind in einer folgenden Tabelle aufgeführt.

Die Verbindung gemäß Formel (I) kann aber auch erfindungsgemäß in einer elektronischen Vorrichtung enthaltend eine oder mehrere fluoreszierende emittierende Verbindungen eingesetzt werden.

In einer bevorzugten Ausführungsform der Erfindung werden die Verbindungen gemäß Formel (I) als lochtransportierendes Material eingesetzt. Die Verbindungen liegen dann bevorzugt in einer Lochtransportschicht, einer Elektronenblockierschicht oder einer Lochinjektionsschicht vor. Besonders bevorzugt ist die Verwendung in einer Elektronenblockierschicht.

Eine Lochtransportschicht gemäß der vorliegenden Anmeldung ist eine Schicht mit lochtransportierender Funktion, welche sich zwischen Anode und emittierender Schicht befindet.

Lochinjektionsschichten und Elektronenblockierschichten werden im Sinne der vorliegenden Anmeldung als spezielle Ausführungsformen von Lochtransportschichten verstanden. Eine Lochinjektionsschicht ist dabei im Fall von mehreren Lochtransportschichten zwischen Anode und emittierender Schicht eine Lochtransportschicht, welche sich direkt an die Anode anschließt oder nur durch eine einzelne Beschichtung der Anode von ihr getrennt ist. Eine Elektronenblockierschicht ist im Fall von mehreren Lochtransportschichten zwischen Anode und emittierender Schicht diejenige Lochtransportschicht, welche sich direkt anodenseitig an die emittierende Schicht anschließt. Bevorzugt enthält die erfindungsgemäße OLED zwei, drei oder vier lochtransportierende Schichten zwischen Anode und emittierender Schicht, von denen bevorzugt mindestens eine eine Verbindung gemäß Formel (I) enthält, besonders bevorzugt genau eine oder zwei eine Verbindung gemäß Formel (I) enthalten.

Wird die Verbindung gemäß Formel (I) als Lochtransportmaterial in einer Lochtransportschicht, einer Lochinjektionsschicht oder einer Elektronenblockierschicht eingesetzt, so kann die Verbindung als Reinmaterial, d.h. in einem Anteil von 100 %, in der Lochtransportschicht eingesetzt werden, oder sie kann in Kombination mit einer oder mehreren weiteren Verbindungen eingesetzt werden. Gemäß einer bevorzugten Ausführungsform enthält die organische Schicht enthaltend die Verbindung der Formel (I) dann zusätzlich einen oder mehrere p-Dotanden. Als p-Dotanden werden gemäß der vorliegenden Erfindung bevorzugt solche organischen Elektronenakzeptorverbindungen eingesetzt, die eine oder mehrere der anderen Verbindungen der Mischung oxidieren können.

Besonders bevorzugte Ausführungsformen von p-Dotanden sind die in WO 2011/073149, EP 1968131, EP 2276085, EP 2213662, EP 1722602, EP 2045848, DE 102007031220, US 8044390, US 8057712, WO 2009/003455, WO 2010/094378, WO 2011/120709, US 2010/0096600, WO 2012/095143 und DE 102012209523 offenbarten Verbindungen.

Besonders bevorzugt als p-Dotanden sind Chinodimethanverbindungen, Azaindenofluorendione, Azaphenalene, Azatriphenylene, I₂, Metallhalogenide, bevorzugt Übergangsmetallhalogenide, Metalloxide, bevorzugt Metalloxide enthaltend mindestens ein Übergangsmetall oder ein Metall der 3. Hauptgruppe, und Übergangsmetallkomplexe, bevorzugt Komplexe von Cu, Co, Ni, Pd und Pt mit Liganden enthaltend mindestens ein Sauerstoffatom als Bindungsstelle. Bevorzugt sind weiterhin Übergangsmetalloxide als Dotanden, bevorzugt Oxide von Rhenium, Molybdän und Wolfram, besonders bevorzugt Re₂O₇. MoO₃, WO₃ und ReO₃.
Die p-Dotanden liegen bevorzugt weitgehend gleichmäßig verteilt in den p-dotierten Schichten vor. Dies kann beispielsweise durch Co-Verdampfung des p-Dotanden und der Lochtransportmaterial-Matrix erreicht werden.

Bevorzugt sind als p-Dotanden insbesondere die folgenden Verbindungen:

| | | |
|---|---|---|
| 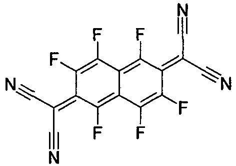 | 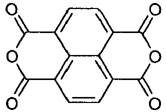 | 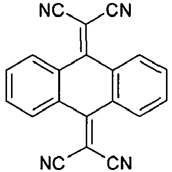 |
| (D-1) | (D-2) | (D-3) |
| 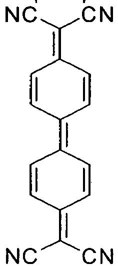 | 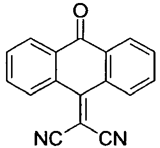 | 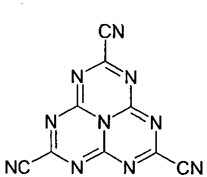 |
| (D-4) | (D-5) | (D-6) |
| 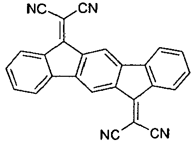 | 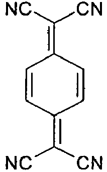 | 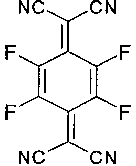 |
| (D-7) | (D-8) | (D-9) |
| 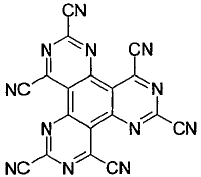 | 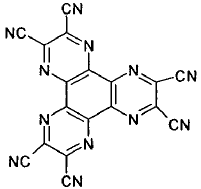 | 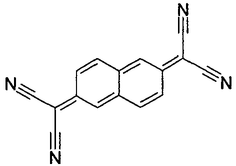 |
| (D-10) | (D-11) | (D-12) |
| 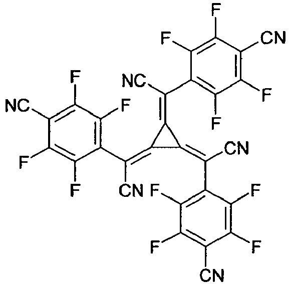 | | |
| (D-13) | | |

In einer weiteren bevorzugten Ausführungsform der Erfindung wird die Verbindung gemäß Formel (I) als Lochtransportmaterial in Kombination mit einem Hexaazatriphenylenderivat, wie in US 2007/0092755 beschrieben, verwendet. Besonders bevorzugt wird das Hexaazatriphenylenderivat dabei in einer separaten Schicht eingesetzt.

In einer weiteren Ausführungsform der vorliegenden Erfindung wird die Verbindung der Formel (I) in einer emittierenden Schicht als Matrixmaterial in Kombination mit einer oder mehreren emittierenden Verbindungen, vorzugsweise phosphoreszierenden emittierenden Verbindungen, eingesetzt.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil der emittierenden Verbindung zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere emittierende Verbindungen enthalten. Auch in diesem Fall sind die emittierenden Verbindungen im Allgemeinen diejenigen Verbindungen, deren Anteil im System der kleinere ist und die Matrixmaterialien sind diejenigen Verbindungen, deren Anteil im System der größere ist. In Einzelfällen kann jedoch der Anteil eines einzelnen Matrixmaterials im System kleiner sein als der Anteil einer einzelnen emittierenden Verbindung.

Es ist bevorzugt, dass die Verbindungen gemäß Formel (I) als eine Komponente von Mixed-Matrix-Systemen verwendet werden. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die Verbindung der Formel (I) stellt dabei bevorzugt das Matrixmaterial mit lochtransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Die Mixed-Matrix-Systeme können einen oder mehrere emittierende Verbindungen umfassen, bevorzugt eine oder mehrere phosphoreszierende emittierende Verbindungen. Allgemein werden Mixed-Matrix-Systeme bevorzugt in phosphoreszierenden organischen Elektrolumineszenzvorrichtungen eingesetzt.

Besonders geeignete Matrixmaterialien, welche in Kombination mit den erfindungsgemäßen Verbindungen als Matrixkomponenten eines Mixed-Matrix-Systems verwendet werden können, sind ausgewählt aus den unten angegebenen bevorzugten Matrixmaterialien für phosphoreszierende emittierende Verbindungen oder den bevorzugten Matrixmaterialien für fluoreszierende emittierende Verbindungen, je nachdem welche Art von emittierender Verbindung im mixed-Matrix-System eingesetzt wird.

Bevorzugte phosphoreszierende emittierende Verbindungen zur Verwendung in Mixed-Matrix-Systemen sind dieselben, wie sie weiter oben als allgemein bevorzugte phosphoreszierende Emittermaterialien aufgeführt wurden.

Im Folgenden werden bevorzugte Ausführungsformen für die verschiedenen Funktionsmaterialien der elektronischen Vorrichtung aufgeführt.

Bevorzugte phosphoreszierende emittierende Verbindungen sind die oben genannten.

Bevorzugte fluoreszierende emittierende Verbindungen sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte emittierende Verbindungen sind Indenofluorenamine bzw. -diamine, beispielsweise gemäß WO 2006/108497 oder WO 2006/122630, Benzoindenofluorenamine bzw. -diamine, beispielsweise gemäß WO 2008/006449, und Dibenzoindenofluorenamine bzw. -diamine, beispielsweise gemäß WO 2007/140847, sowie die in WO 2010/012328 offenbarten Indenofluorenderivate mit kondensierten Arylgruppen. Ebenfalls bevorzugt sind die in WO 2012/048780 und die in WO 2013/185871 offenbarten Pyren-Arylamine. Ebenfalls bevorzugt sind die in WO 2014/037077 offenbarten Benzoindenofluoren-Amine, die in WO 2014/106522 offenbarten Benzofluoren-Amine, die in WO 2014/111269 und in der noch nicht offengelegten Anmeldung EP 15182993.4 offenbarten erweiterten Benzoindenofluorene, die in den noch nicht offengelegten Anmeldungen EP 15181178.3 und EP 15181177.5 offenbarten Phenoxazine, und die in der noch nicht offengelegten Anmeldung EP 15000876.1 offenbarten Fluoren-Derivate, die mit Furan-Einheiten oder mit Thiophen-Einheiten verbunden sind.

Als Matrixmaterialien, bevorzugt für fluoreszierende emittierende Verbindungen, kommen Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind. Bevorzugt sind weiterhin die in WO 2006/097208, WO 2006/131192, WO 2007/065550, WO 2007/110129, WO 2007/065678, WO 2008/145239, WO 2009/100925, WO 2011/054442, und EP 1553154 offenbarten Anthracenderivate, die in EP 1749809, EP 1905754 und US 2012/0187826 offenbarten Pyren-Verbindungen, die in WO 2015/158409 offenbarten Benzanthracenyl-Anthracen-Verbindungen, die in der noch nicht offengelegten Anmeldung EP 15180777.3 offenbarten Indeno-Benzofurane, und die in der noch nicht offengelegten Anmeldung EP 15182962.9 offenbarten Phenanthryl-Anthracene.

Bevorzugte Matrixmaterialien für phosphoreszierende emittierende Verbindungen sind neben den Verbindungen der Formel (I) aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Bis-carbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109, WO 2011/000455 oder WO 2013/041176, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte CarbazolDerivate, z. B. gemäß US 2009/0136779, WO 2010/050778, WO 2011/042107, WO 2011/088877 oder WO 2012/143080, Triphenylenderivaten, z. B. gemäß WO 2012/048781, oder Lactame, z. B. gemäß WO 2011/116865 oder WO 2011/137951.

Geeignete Ladungstransportmaterialien, wie sie in der Lochinjektions- bzw. Lochtransportschicht bzw. Elektronenblockierschicht oder in der Elektronentransportschicht der erfindungsgemäßen elektronischen Vorrichtung verwendet werden können, sind neben den Verbindungen der Formel (I) beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Bevorzugt umfasst die erfindungsgemäße OLED zwei oder mehr unterschiedliche lochtransportierende Schichten. Die Verbindung der Formel (I) kann dabei in einer oder in mehreren oder in allen lochtransportierenden Schichten eingesetzt werden. Gemäß einer bevorzugten Ausführungsform wird die Verbindung der Formel (I) in genau einer oder genau zwei lochtransportierenden Schichten eingesetzt, und in den weiteren vorhandenen lochtransportierenden Schichten werden andere Verbindungen eingesetzt, bevorzugt aromatische Aminverbindungen. Weitere Verbindungen, die neben den Verbindungen der Formel (I) bevorzugt in lochtransportierenden Schichten der erfindungsgemäßen OLEDs eingesetzt werden, sind insbesondere Indenofluorenamin-Derivate (z. B. gemäß WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 07/140847), Spirobifluoren-Amine (z. B. gemäß WO 2012/034627 oder WO 2013/120577), Fluoren-Amine (z. B. gemäß WO 2014/015937, WO 2014/015938, WO 2014/015935 und WO 2015/082056), Spiro-Dibenzopyran-Amine (z. B. gemäß WO 2013/083216), Dihydroacridin-Derivate (z. B. gemäß WO 2012/150001), Spirodibenzofurane und Spirodibenzothiophene, z.B. gemäß WO 2015/022051 und den noch nicht offen gelegten Anmeldungen PCT/EP2015/002475 und PCT/EP2016/000084, Phenanthren-Diarylamine, z.B. gemäß WO 2015/131976, Spiro-Tribenzotropolone, z.B. gemäß der noch nicht offen gelegten Anmeldung PCT/EP2015/002225, Spirobifluorene mit meta-Phenyldiamingruppen, z.B. gemäß der noch nicht offen gelegten Anmeldung PCT/EP2015/002112, Spiro-Bisacridine, zB. gemäß WO 2015/158411, Xanthen-Diarylamine, z.B. gemäß WO 2014/072017, und 9,10-Dihydroanthracen-Spiroverbindungen mit Diarylaminogruppen gemäß WO 2015/086108.

Ganz besonders bevorzugt ist die Verwendung von in 4-Position mit Diarylaminogruppen substituierten Spirobifluorenen als lochtransportierende Verbindungen, insbesondere die Verwendung derjenigen Verbindungen, die in WO 2013/120577 beansprucht und offenbart sind, und die Verwendung von in 2-Position mit Diarylaminogruppen substituierten Spirobifluorenen als lochtransportierende Verbindungen, insbesondere insbesondere die Verwendung derjenigen Verbindungen, die in WO 2012/034627 beansprucht und offenbart sind.

Als Materialien für die Elektronentransportschicht können alle Materialien verwendet werden, wie sie gemäß dem Stand der Technik als Elektronentransportmaterialien in der Elektronentransportschicht verwendet werden. Insbesondere eignen sich Aluminiumkomplexe, beispielsweise Alq₃, Zirkoniumkomplexe, beispielsweise Zrq₄, Lithiumkomplexe, beispielsweise Liq, Benzimidazolderivate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Weiterhin geeignete Materialien sind Derivate der oben genannten Verbindungen, wie sie in JP 2000/053957, WO 2003/060956, WO 2004/028217, WO 2004/080975 und WO 2010/072300 offenbart werden.

Als Kathode der elektronischen Vorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, AI, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Die Vorrichtung wird entsprechend (je nach Anwendung) strukturiert, kontaktiert und schließlich versiegelt, um schädigende Effekte von Wasser und Luft auszuschließen.

In einer bevorzugten Ausführungsform ist die elektronische Vorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine elektronische Vorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine elektronische Vorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I) nötig. Hohe Löslichkeit lässt sich durch geeignete Substitution der Verbindungen erreichen.

Weiterhin bevorzugt ist es, dass zur Herstellung einer erfindungsgemäßen elektronischen Vorrichtung eine oder mehrere Schichten aus Lösung und eine oder mehrere Schichten durch ein Sublimationsverfahren aufgetragen werden.

Erfindungsgemäß können die elektronischen Vorrichtungen enthaltend eine oder mehrere Verbindungen gemäß Formel (I) in Displays, als Lichtquellen in Beleuchtungsanwendungen sowie als Lichtquellen in medizinischen und/oder kosmetischen Anwendungen (z.B. Lichttherapie) eingesetzt werden.

### Beispiele

### A) Synthesebeispiele

### A1) Beispiel 1

Synthese der Verbindung Bis-biphenyl-3-yl-[4-(9,9-spirobifluoren-4-yl)-phenyl]-amin (1-1) sowie der Verbindungen (1-2) bis (1-17)

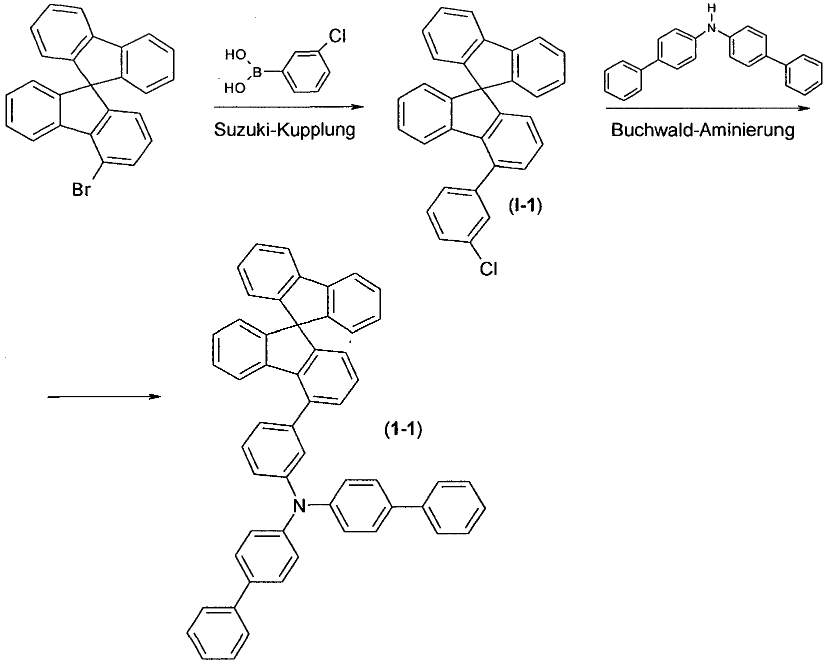

### Synthese der Zwischenstufe I-1: 4-(3-Chloro-phenyl)-9,9-spirobifluoren

21,7 g (139 mmol) 4-Chlor-benzolboronsäure, 50 g (126 mmol) 4-Brom-9,9-spirobifluoren und 208 mL einer wässrigen 2 M K₂CO₃-Lösung (416 mmol) werden in 300 mL Tetrahydrofuran suspendiert. Zu dieser Suspension werden 1,45 g (1,26 mmol) Tetrakis-(triphenyl)phosphin-palladium(0) gegeben. Die Reaktionsmischung wird 16 h unter Rückfluss erhitzt. Nach Erkalten wird die organische Phase abgetrennt, über Kieselgel filtriert, dreimal mit 300 mL Wasser gewaschen und anschließend zur Trockene eingeengt. Nach Filtration des Rohproduktes über Kieselgel mit Heptan/Essigsäureester (20:1) erhält man 48 g (89%) 4-(3-Chloro-phenyl)-9,9-spirobifluoren.

Analog dazu werden folgende Verbindungen hergestellt:

| | **Edukt 1** | **Edukt 2** | **Produkt** | **Ausbeute** |
|---|---|---|---|---|
| **I-1** | 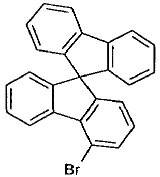 | 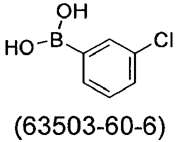 | 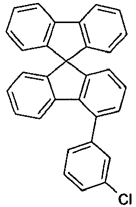 | 89% |
| **I-2** | 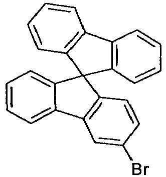 | 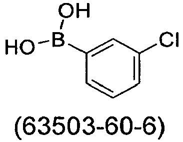 | 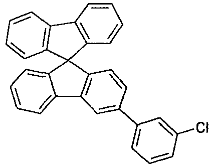 | 88% |
| **I-3** | 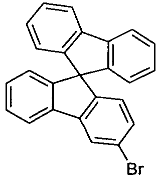 | 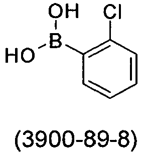 | 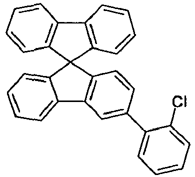 | 85% |
| **I-4** | 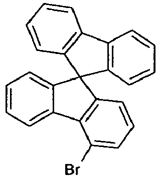 | 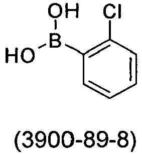 | 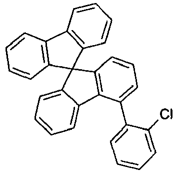 | 89% |
| **I-5** | 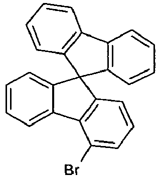 | 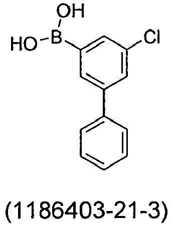 | 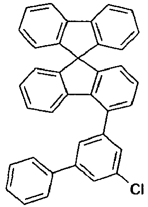 | 89% |
| **I-6** | 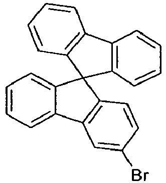 | 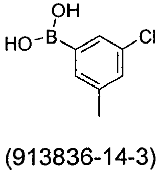 | 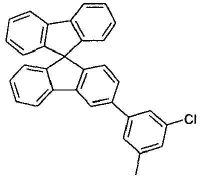 | 83% |
| **I-7** | 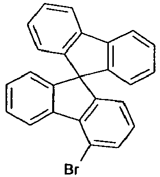 | 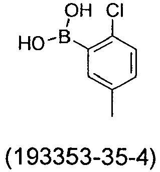 | 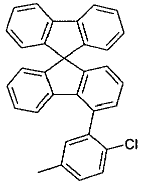 | 83% |

### Bis-biphenyl-3-yl-[4-(9,9-spirobifluoren-4-yl)-phenyl]-amin (1-1)

15,1 g Bis-biphenyl-4-yl-amin (46,8 mmol) und 20 g 4-(3-Chloro-phenyl)-9,9-spirobifluoren (46,8 mmol) werden in 300 mL Toluol gelöst: Die Lösung wird entgast und mit N₂ gesättigt. Danach wird sie mit 2,34 mL (2,34 mmol) einer 1 M Tri-tert-Butylphosphin-Lösung und 0,26 g (1.17 mmol) Palladium(II)acetat versetzt. Anschließend werden 11,2 g Natrium-tert-butylat (117 mmol) zugegeben. Die Reaktionsmischung wird 4 h unter Schutzatmosphäre zum Sieden erhitzt. Das Gemisch wird im Anschluss zwischen Toluol und Wasser verteilt, die organische Phase dreimal mit Wasser gewaschen, über Na₂SO₄ getrocknet und einrotiert. Nach Filtration des Rohproduktes über Kieselgel mit Toluol wird der verbleibende Rückstand aus Heptan/Toluol umkristallisiert und abschließend im Hochvakuum sublimiert. Die Reinheit beträgt 99.9%. Die Ausbeute beträgt 23 g (71% der Theorie).

Analog dazu werden folgende Verbindungen hergestellt:

| | **Edukt 1** | **Edukt 2** | **Produkt** | **Ausbeute** |
|---|---|---|---|---|
| **1-1** | 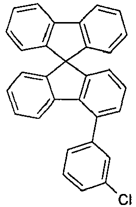 | 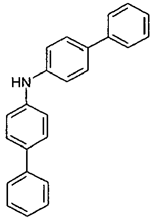 | 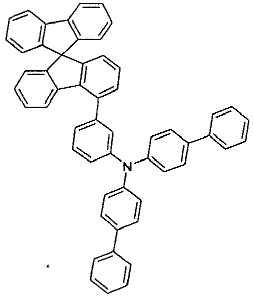 | 71% |
| **1-2** | 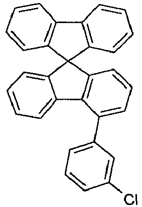 | 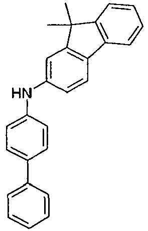 | 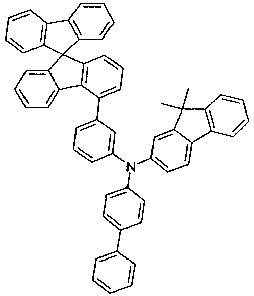 | 68% |
| **1-3** | 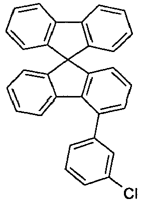 | 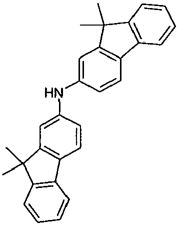 | 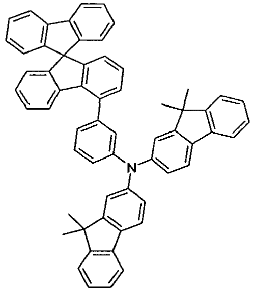 | 80% |
| **1-4** | 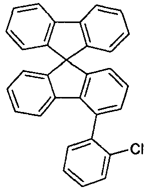 | 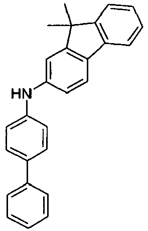 | 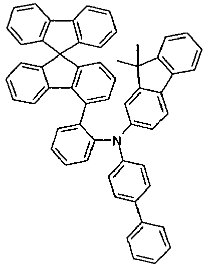 | 70% |
| **1-5** | 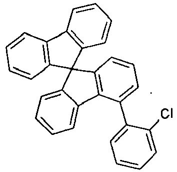 | 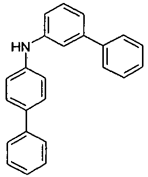 | 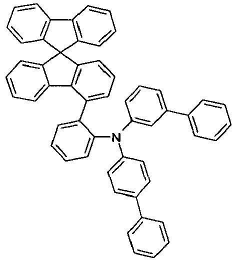 | 75% |
| **1-6** | 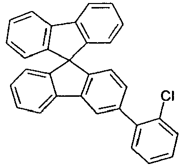 | 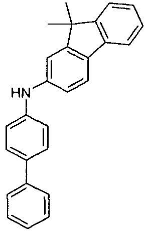 | 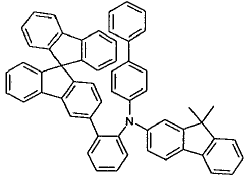 | 70% |
| **1-8** | 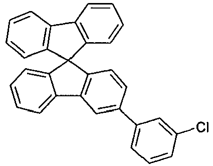 | 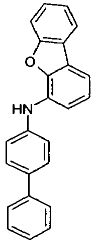 | 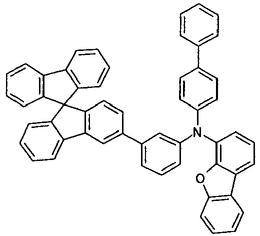 | 70% |
| **1-9** | 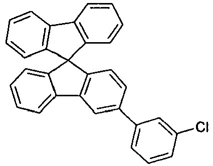 | 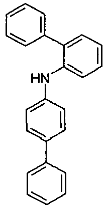 | 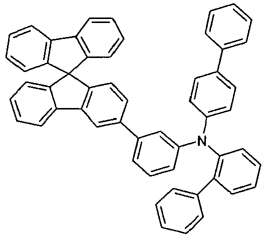 | 73% |
| **1-10** | 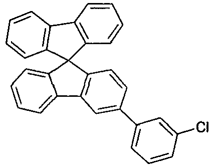 | 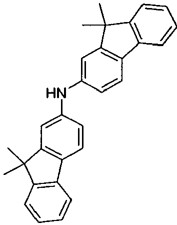 | 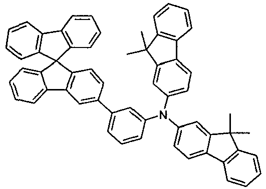 | 73% |
| **1-11** | 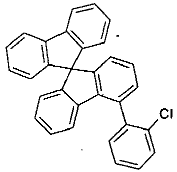 | 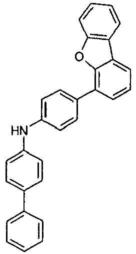 | 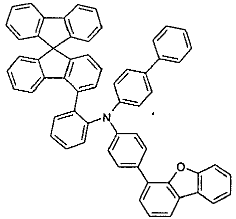 | 65% |
| **1-12** | 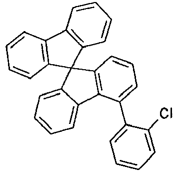 | 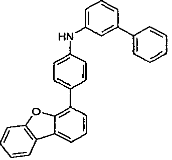 | 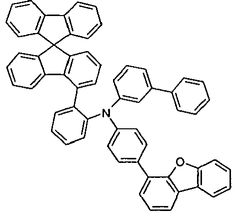 | 71% |
| **1-13** | 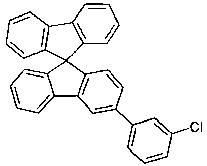 | 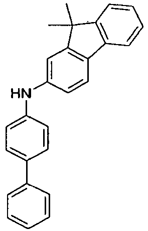 | 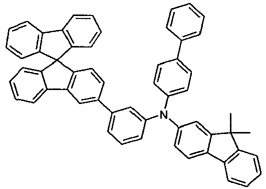 | 68% |
| **1-14** | 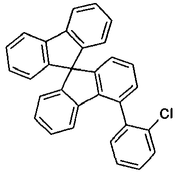 | 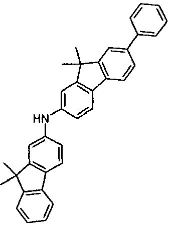 | 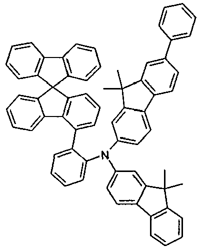 | 63% |
| **1-15** | 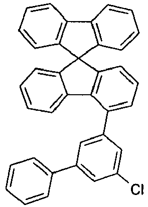 | 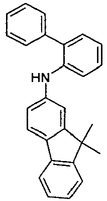 | 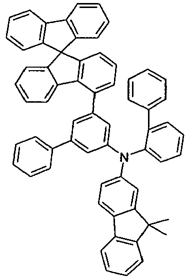 | 58% |
| **1-16** | 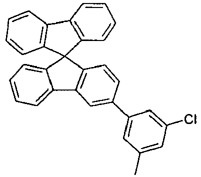 | 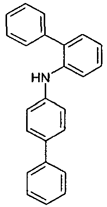 | 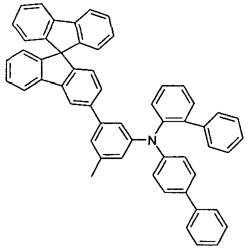 | 50% |
| **1-17** | 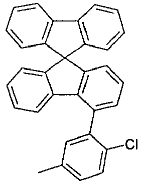 | 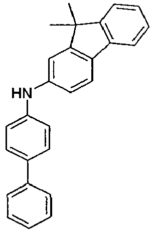 | 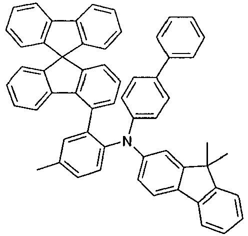 | 50% |
| **1-18** | 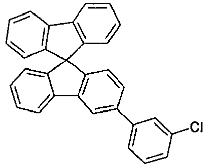 | 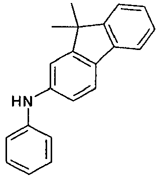 | 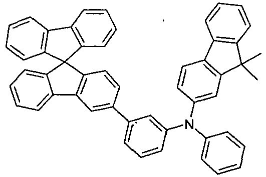 | 53% |
| **1-19** | 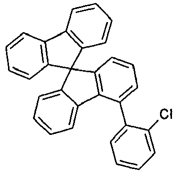 | 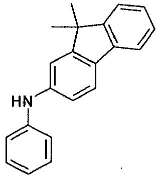 | 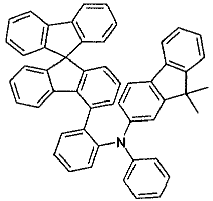 | 45% |

### B) Device-Beispiele

Die Herstellung von erfindungsgemäßen OLEDs sowie OLEDs nach dem Stand der Technik erfolgt nach einem allgemeinen Verfahren gemäß WO 04/058911, das auf die hier beschriebenen Gegebenheiten (z.B. Materialien, Schichtdicken) angepasst wird.

In den folgenden erfindungsgemäßen Beispielen werden die Daten verschiedener OLEDs vorgestellt (siehe Tabellen 1 bis 4). Als Substrate werden Glasplättchen verwendet, die mit strukturiertem ITO (Indium Zinn Oxid) der Dicke 50nm beschichtet sind. Die OLEDs haben folgenden allgemeinen Schichtaufbau: Substrat / Lochinjektionsschicht (HIL) / Lochtransportschicht (HTL) / optionale zweite Lochtransportschicht (HTL2) / Elektronenblockierschicht (EBL) / Emissionsschicht (EML) / Elektronentransportschicht (ETL) / Elektroneninjektionsschicht (EIL) und abschließend eine Kathode. Die Kathode wird durch eine 100nm dicke Aluminiumschicht gebildet. Die genaue Struktur der OLEDs ist in Tabelle 1 gezeigt. Die zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie H1:SEB(5%) bedeutet hierbei, dass das Material H1 in einem Volumenanteil von 95% und SEB in einem Volumenanteil von 5% in der Schicht vorliegt. Analog können auch andere Schichten aus einer Mischung von zwei oder mehr Materialien bestehen. Einzelheiten hierzu sind in Tabellen 1 und 4 angegeben.

Die OLEDs werden standardmäßig charakterisiert. Hierfür wird die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Angabe EQE @ 10mA/cm² bezeichnet die externe Quanteneffizienz bei einer Stromdichte von 10mA/cm².
LD80 @ 60mA/cm² ist die Lebensdauer, bis zu der die OLED von ihrer Anfangshelligkeit von 5000cd/m² auf 80% ihrer Anfangshelligkeit, d.h. 4000cd/m² ohne einen Beschleunigungsfaktor abgefallen ist. Die erhaltenen Werte für die erfindungsgemäßen OLEDs und die Vergleichs-OLEDs sind in Tabellen 2a und 2b zusammengefasst.

| **Tabelle 1: Aufbau der OLEDs** | | | | | | | |
|---|---|---|---|---|---|---|---|
| ***Ex.*** | ***HIL*** | ***HTL*** | ***HTL2*** | ***EBL*** | ***EML*** | ***ETL*** | ***EIL*** |
| | Dicke / nm | Dicke / nm | Dicke / nm | Dicke / nm | Dicke / nm | Dicke / nm | Dicke / nm |
| V1 | HIM: F4TCNQ(5%) 20 nm | HIM 180 nm | -- | HTMV1 10 nm | H1:SEB(5%) 20 nm | ETM:LiQ(50%) 30 nm | LiQ 1 nm |
| V2 | HIM: F4TCNQ(5%) 20 nm | HIM 180 nm | -- | HTMV2 10 nm | H1:SEB(5%) 20 nm | ETM:LiQ(50%) 30 nm | LiQ 1 nm |
| E1 | HIM: F4TCNQ(5%) 20 nm | HIM 180 nm | -- | HTM1 10 nm | H1:SEB(5%) 20 nm | ETM:LiQ(50%) 30 nm | LiQ 1 nm |
| E3 | HIM: F4TCNQ(5%) 20 nm | HIM 180 nm | -- | HTM2 10 nm | H1:SEB(5%) 20 nm | ETM:LiQ(50%) 30 nm | LiQ 1 nm |
| E8 | HIM: F4TCNQ(5%) 20 nm | HIM 180 nm | -- | HTM6 10 nm | H1:SEB(5%) 20 nm | ETM:LiQ(50%) 30 nm | LiQ 1 nm |
| E10 | HIM: F4TCNQ(5%) 20 nm | HIM 180 nm | -- | HTM7 10 nm | H1:SEB(5%) 20 nm | ETM:LiQ(50%) 30 nm | LiQ 1 nm |
| V3 | HTMV3: F4TCNQ(5%) 20 nm | HTMV3 180 nm | -- | EBM 10 nm | H1:SEB(5%) 20 nm | ETM:LiQ(50%) 30 nm | LiQ 1 nm |
| E2 | HTM3: F4TCNQ(5%) 20 nm | HTM3 180 nm | -- | EBM 10 nm | H1:SEB(5%) 20 nm | ETM:LiQ(50%) 30 nm | LiQ 1 nm |
| E4 | HTM2: F4TCNQ(5%) 20 nm | HTM2 180 nm | -- | EBM 10 nm | H1:SEB(5%) 20 nm | ETM:LiQ(50%) 30 nm | LiQ 1 nm |
| E9 | HTM6: F4TCNQ(5%) 20 nm | HTM6 180 nm | -- | EBM 10 nm | H1:SEB(5%) 20 nm | ETM:LiQ(50%) 30 nm | LiQ 1 nm |
| V4 | HIM: F4TCNQ(5%) 20 nm | HIM 210 nm | HTMV1$: F4TCNQ(5%) 20 nm | HTMV1 20 nm | TMM:TEG(10%) 30 nm | ETM:LiQ(50%) 40 nm | LiQ 1 nm |
| V5 | HIM: F4TCNQ(5%) 20 nm | HIM 210 nm | HTMV2: F4TCNQ(5%) 20 nm | HTMV2 20 nm | TMM:TEG(10%) 30 nm | ETM:LiQ(50%) 40 nm | LiQ 1 nm |
| E7 | HIM: F4TCNQ(5%) 20 nm | HIM 210 nm | HTM2: F4TCNQ(5%) 20 nm | HTM2 20 nm | TMM:TEG(10%) 30 nm | ETM:LiQ(50%) 40 nm | LiQ 1 nm |

| **Tabelle 2a: Erhaltene Daten für die OLEDs mit Singulett-Blau-EML** | | |
|---|---|---|
| | U | EQE @ 10mA/cm² |
| | [V] | [%] |
| V1 | 3.8 | 8.0 |
| V2 | 4.1 | 8.0 |
| E1 | 3.8 | 8.7 |
| E3 | 4.0 | 8.6 |
| E8 | 3.6 | 8.1 |
| E10 | 3.8 | 8.6 |
| V3 | 7.9 | 5.5 |
| E2 | 3.9 | 8.9 |
| E4 | 4.0 | 8.1 |
| E9 | 3.8 | 8.7 |

| **Tabelle 2b: Erhaltene Daten für die OLEDs mit Triplett-Grün-EML** | | |
|---|---|---|
| | U | LD80 @ 60 mA/cm² |
| V4 | 4.1 | 130 |
| V5 | 3.9 | 70 |
| E7 | 4.1 | 165 |

| **Tabelle 3 - Strukturen der verwendeten Verbindungen** | | |
|---|---|---|
| 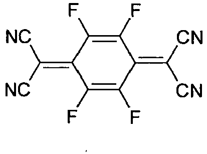 | 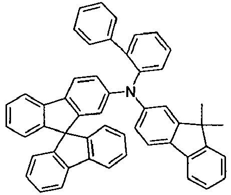 | 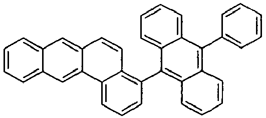 |
| F4TCNQ | HIM | H1 |
| 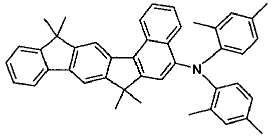 | 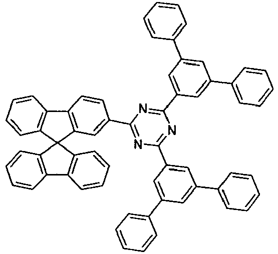 | 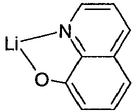 |
| SEB | ETM | LiQ |
| 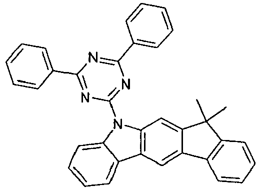 | 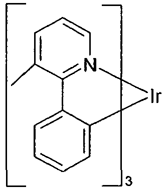 | |
| TMM | TEG | |
| 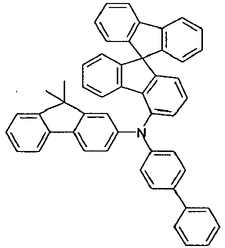 | 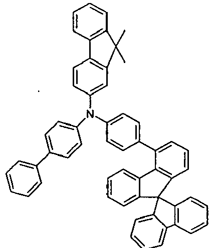 | 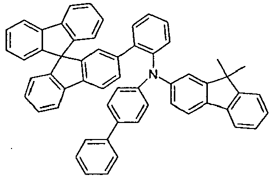 |
| EBM | HTMV1 | HTMV2 |
| 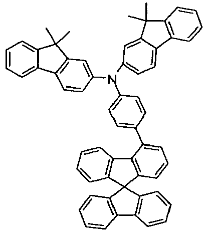 | 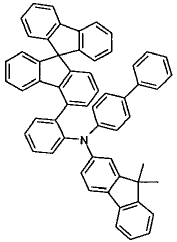 | 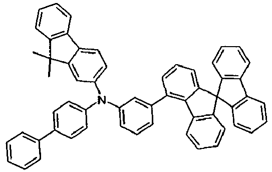 |
| HTMV3 | HTM1 | HTM2 |
| 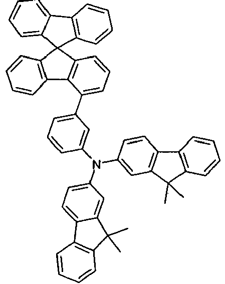 | 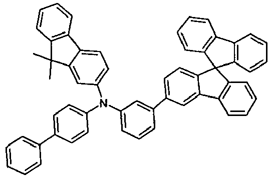 | 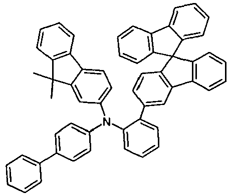 |
| HTM3 | HTM4 | HTM5 |
| 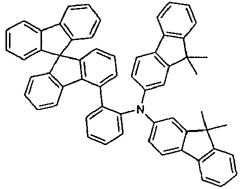 | 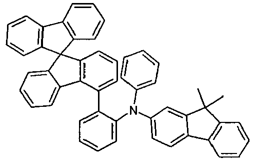 | |
| HTM6 | HTM7 | |

Alle getesteten OLEDs, welche erfindungsgemäße Verbindungen enthalten, zeigen sehr gute Werte für die gemessenen Parameter Lebensdauer und Effizienz, sowohl für Singulett-blau-Devices (E1 bis E4 und E8 bis E10) als auch für Triplett-Grün-Devices (E7). Die erfindungsgemäßen Verbindungen werden in den hergestellten Vorrichtungen jeweils in Lochinjektionsschichten und/oder Lochtransportschichten und/oder Elektronenblockierschichten eingesetzt.

Aus den erhaltenen Ergebnissen sind die folgenden Vergleiche hervorzuheben:
Ein Vergleich der ansonsten identisch aufgebauten Devices V1, E1 und E3 zeigt, dass Devices enthaltend die erfindungsgemäßen Verbindungen HTM1 (E1) und HTM2 (E3) deutlich bessere Werte für die Effizienz zeigen als das Vergleichs-Device enthaltend die Vergleichsverbindung HTMV1 (V1). Dies zeigt die Verbesserung, die durch das Einführen einer ortho- bzw. meta-Phenylen-Gruppe erzielt wird, verglichen mit einer para-Phenylen-Gruppe.

Ein Vergleich der ansonsten identisch aufgebauten Devices V2 und E1 zeigt, dass ein Device enthaltend die erfindungsgemäße Verbindung HTM1 (E1) deutlich bessere Werte für die Effizienz zeigt als das Vergleichs-Device enthaltend die Vergleichsverbindung HTMV2 (V2). Dies zeigt die Verbesserung, die durch 4-substituierte Spiro-Verbindungen gezeigt wird, verglichen mit 2-substituierten Spiro-Verbindungen.

Ein Vergleich der ansonsten identisch aufgebauten Devices V3 und E2 und E9 zeigt, dass ein Device enthaltend die erfindungsgemäße Verbindung HTM3 (E2) und ein Device enthaltend die erfindungsgemäße Verbindung HTM6 (E9) deutlich bessere Werte für die Effizienz zeigen als das Vergleichs-Device enthaltend die Vergleichsverbindung HTMV3 (V3). Dies zeigt erneut für zwei andere Beispiele die Verbesserung, die durch das Einführen einer meta-Phenylen-Gruppe erzielt wird, verglichen mit einer para-Phenylen-Gruppe.

Ein weiterer Vergleich zwischen den beiden ansonsten identisch aufgebauten erfindungsgemäßen OLEDs E2 enthaltend HTM3 und E4 enthaltend HTM2 zeigt die überraschende deutliche Verbesserung der Effizienz, die durch die Verwendung von zwei Fluorenylgruppen anstelle von einer Fluorenylgruppe und einer Biphenylgruppe am Amin erhalten wird.

Ein Vergleich der ansonsten identisch aufgebauten Devices V4 und E7 zeigt, dass ein Device enthaltend die erfindungsgemäße Verbindung HTM2 (E7) deutlich bessere Werte für die Lebensdauer zeigt als das Vergleichs-Device enthaltend die Vergleichsverbindung HTMV1 (V4). Dies zeigt erneut für ein anderes Beispiel die Verbesserung, die durch das Einführen einer meta-Phenylen-Gruppe erzielt wird, verglichen mit einer para-Phenylen-Gruppe Zusätzlich werden die folgenden beiden Device-Aufbauten erstellt:

| **Tabelle 4: Aufbau der OLEDs** | | | | | | | |
|---|---|---|---|---|---|---|---|
| Ex. | HIL | HTL | HIL2 | EBL | EML | ETL | EIL |
| | Dicke / nm | Dicke / nm | Dicke / nm | Dicke / nm | Dicke / nm | Dicke / nm | Dicke / nm |
| E5 | HIM: F4TCNQ(5%) 20 nm | HIM 180 nm | -- | HTM4 10 nm | H1:SEB(5%) 20 nm | ETM:LiQ(50%) 30 nm | LiQ 1 nm |
| E6 | HIM: F4TCNQ(5%) 20 nm | HIM 180 nm | -- | HTM5 10 nm | H1:SEB(5%) 20 nm | ETM:LiQ(50%) 30 nm | LiQ 1 nm |

In diesen beiden Beispielen werden die Materialien HTM4 und HTM5 eingesetzt, die in 3-Position substituierte Spirobifluorene gemäß der vorliegenden Erfindung sind. Auch mit diesen beiden Materialien werden gute Ergebnisse in OLEDs erzielt.

## Patentansprüche

1. Verbindung gemäß Formel (I) 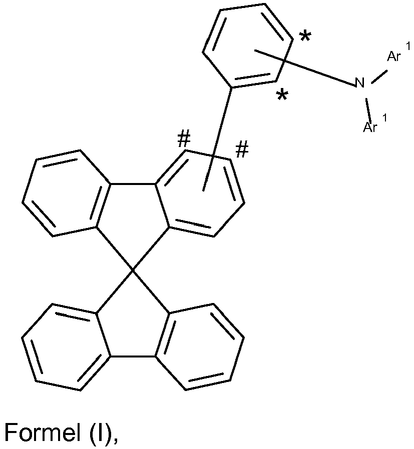 wobei die Verbindung an jeder der freien Positionen an der Spirobifluoren-Einheit jeweils mit einem Rest R¹ substituiert sein kann, und an jeder der freien Positionen an der Phenylen-Einheit jeweils mit einem Rest R² substituiert sein kann, und
wobei für die auftretenden Variablen gilt:
R¹ ist bei jedem Auftreten gleich oder verschieden gewählt aus H, D, F, C(=O)R⁴, CN, Si(R⁴)₃, N(R⁴)₂, P(=O)(R⁴)₂, OR⁴, S(=O)R⁴, S(=O)₂R⁴, geradkettigen Alkyl- oder Alkoxygruppen mit 1 bis 20 C-Atomen, verzweigten oder cyclischen Alkyl- oder Alkoxy-gruppen mit 3 bis 20 C-Atomen, Alkenyl- oder Alkinylgruppen mit 2 bis 20 C-Atomen, aromatischen Ringsystemen mit 6 bis 40 aromatischen Ringatomen, wobei der Begriff "aromatisches Ringsystem" nicht Triarylamin umfasst, und heteroaromatischen Ringsystemen mit 5 bis 40 aromatischen Ringatomen; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁴ substituiert sein können; und wobei eine oder mehrere CH₂-Gruppen in den genannten Alkyl-, Alkoxy-, Alkenyl- und Alkinylgruppen durch -R⁴C=CR⁴⁻, -C=C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können;
R² ist bei jedem Auftreten gleich oder verschieden gewählt aus H, D, F, C(=O)R⁴, CN, Si(R⁴)₃, P(=O)(R⁴)₂, OR⁴, S(=O)R⁴, S(=O)₂R⁴, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R² miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁴ substituiert sein können; und wobei eine oder mehrere CH₂-Gruppen in den genannten Alkyl-, Alkoxy-, Alkenyl- und Alkinylgruppen durch -R⁴C=CR⁴⁻, -C=C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können;
Ar¹ ist bei jedem Auftreten gleich oder verschieden gewählt aus aromatischen Ringsystemen mit 6 bis 20 aromatischen Ringatomen, die optional mit einem oder mehreren Resten R³ substituiert sind, und aus heteroaromatischen Ringsystemen mit 5 bis 30 aromatischen Ringatomen, die optional mit einem oder mehreren Resten R³ substituiert sind;
R³ ist bei jedem Auftreten gleich oder verschieden gewählt aus H, D, F, C(=O)R⁴, CN, Si(R⁴)₃, N(R⁴)₂, P(=O)(R⁴)₂, OR⁴, S(=O)R⁴, S(=O)₂R⁴, geradkettigen Alkyl- oder Alkoxygruppen mit 1 bis 20 C-Atomen, verzweigten oder cyclischen Alkyl- oder Alkoxy-gruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁴ substituiert sein können; und wobei eine oder mehrere CH₂-Gruppen in den genannten Alkyl-, Alkoxy-, Alkenyl- und Alkinylgruppen durch -R⁴C=CR⁴⁻, -C=C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können;
R⁴ ist bei jedem Auftreten gleich oder verschieden gewählt aus H, D, F, C(=O)R⁵, CN, Si(R5)₃, P(=O)(R⁵)₂, OR⁵, S(=O)R⁵, S(=O)₂R⁵, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁵ substituiert sein können; und wobei eine oder mehrere CH₂-Gruppen in den genannten Alkyl-, Alkoxy-, Alkenyl- und Alkinylgruppen durch -R⁵C=CR⁵-, -C≡C-, Si(R⁵)₂, C=O, C=NR⁵, -C(=O)O-, -C(=O)NR⁵-, NR⁵, P(=O)(R⁵),-O-, -S-, SO oder SO₂ ersetzt sein können;
R⁵ ist bei jedem Auftreten gleich oder verschieden gewählt aus H, D, F, CN, Alkyl- oder Alkoxygruppen mit 1 bis 20 C-Atomen, Alkenyl- oder Alkinylgruppen mit 2 bis 20 C-Atomen, aromatischen Ringsystemen mit 6 bis 40 aromatischen Ringatomen und heteroaromatischen Ringsystemen mit 5 bis 40 aromatischen Ringatomen; und wobei die genannten Alkyl-, Alkoxy-, Alkenyl- und Alkinylgruppen, aromatischen Ringsysteme und heteroaromatischen Ringsysteme mit F oder CN substituiert sein können;
wobei der Benzolring an einer der mit dem Zeichen # markierten Positionen an die Spirobifluorengruppe gebunden ist; und
wobei die Gruppe N(Ar¹)₂ an einer der mit dem Zeichen * markierten Positionen an den Benzolring gebunden ist.

2. Verbindung nach Anspruch 1, **dadurch gekennzeichnet, dass** am Spirobifluoren-Grundgerüst kein Rest R¹ gebunden ist, genau ein Rest R¹ gebunden ist, der ungleich H ist, oder genau zwei Reste R¹ gebunden sind, die ungleich H sind.

3. Verbindung nach Anspruch 1 oder 2, **dadurch gekennzeichnet, dass** R² bei jedem Auftreten gleich oder verschieden gewählt ist aus H, F, Methyl, Ethyl, Propyl, Butyl, tert-Butyl, Phenyl, Biphenyl, Terphenyl, Naphthyl, Carbazolyl, Benzofuranyl, Benzothiophenyl, Dibenzofuranyl, Dibenzothiophenyl, 9,9'-Dimethylfluorenyl und 9,9'-Diphenylfluorenyl.

4. Verbindung nach einem oder mehreren der Ansprüche 1 bis 3, **dadurch gekennzeichnet, dass** an die Phenylengruppe in Formel (I) kein Rest R² oder genau ein Rest R² gebunden ist, der ungleich H ist.

5. Verbindung nach einem oder mehreren der Ansprüche 1 bis 4, **dadurch gekennzeichnet, dass** Ar¹, wenn es ein heteroaromatisches Ringsystem ist, bei jedem Auftreten gleich oder verschieden gewählt ist aus jeweils optional mit einem oder mehreren Resten R³ substituiertem Dibenzofuranyl, Dibenzothiophenyl, Benzofuranyl, Benzothiophenyl, benzo-annelliertem Dibenzofuranyl, benzo-annelliertem Dibenzothiophenyl, Indolyl, Chinolinyl, Pyridyl, Pyrimidyl, Pyrazinyl, Pyridazinyl, Triazinyl, Triazol, Oxazol, Oxadiazol, Benzoxazol, Benzothiazol, Phenanthrolyl und Azacarbazolyl; und/oder dass Ar¹, wenn es ein aromatisches Ringsystem ist, bei jedem Auftreten gleich oder verschieden gewählt aus aromatischen Ringsystemen mit 6 bis 12 aromatischen Ringatomen, die optional mit einem oder mehreren Resten R³ substituiert sind.

6. Verbindung nach einem oder mehreren der Ansprüche 1 bis 5, **dadurch gekennzeichnet, dass** Ar¹ bei jedem Auftreten gleich oder verschieden gewählt sind aus jeweils optional mit einem oder mehreren Resten R³ substituiertem Phenyl, Biphenyl, Terphenyl, Quaterphenyl, Naphthyl, Fluorenyl, insbesondere 9,9'-Dimethylfluorenyl und 9,9'-Diphenylfluorenyl, Monobenzofluorenyl, Dibenzofluorenyl, Indenofluorenyl, Dibenzofuranyl, Dibenzothiophenyl, Benzofuranyl, Benzothiophenyl, benzo-annelliertem Dibenzofuranyl, benzo-annelliertem Dibenzothiophenyl, Indolyl, Chinolinyl, Pyridyl, Pyrimidyl, Pyrazinyl, Pyridazinyl und Triazinyl.

7. Verbindung nach einem oder mehreren der Ansprüche 1 bis 6, **dadurch gekennzeichnet, dass** R³ gleich H ist.

8. Verbindung nach einem oder mehreren der Ansprüche 1 bis 7, **dadurch gekennzeichnet, dass** die Verbindung der Formel (I) eine Verbindung gemäß einer der Formeln (I-1) bis (I-4) ist 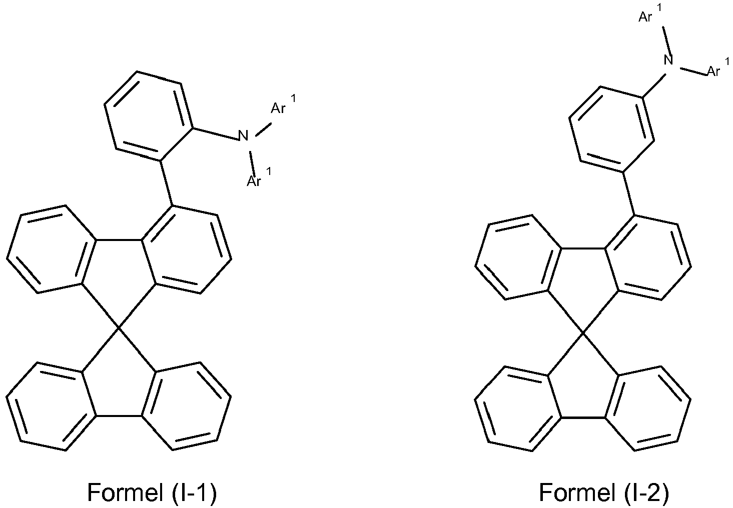 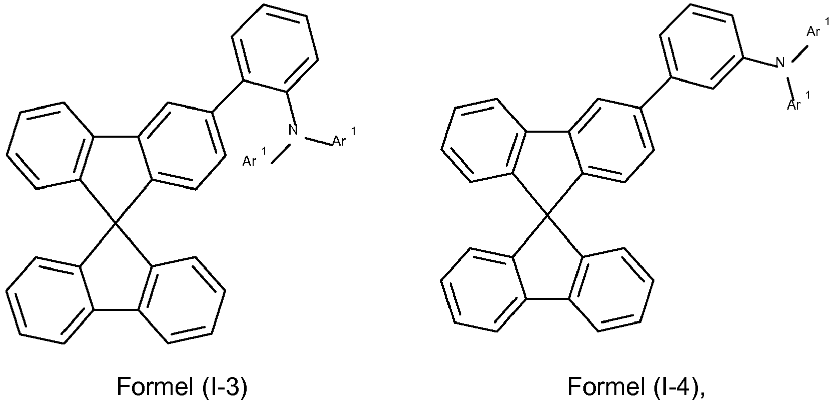 wobei die Formel an jeder der freien Positionen an der Spirobifluoren-Einheit jeweils mit einem Rest R¹ substituiert sein kann, und an jeder der freien Positionen an der Phenylen-Einheit jeweils mit einem Rest R² substituiert sein kann, und
wobei die auftretenden Gruppen wie in Anspruch 1 oder in einem der Ansprüche 2 bis 7 definiert sind.

9. Verbindung nach einem oder mehreren der Ansprüche 1 bis 8, **dadurch gekennzeichnet, dass** R¹ bei jedem Auftreten gleich oder verschieden gewählt ist aus H, F, Methyl, Ethyl, Propyl, Butyl, tert-Butyl, Phenyl, Biphenyl, Terphenyl, Naphthyl, Carbazolyl, Benzofuranyl, Benzothiophenyl, Dibenzofuranyl, Dibenzothiophenyl, 9,9'-Dimethylfluorenyl und 9,9'-Diphenylfluorenyl.

10. Verbindung nach einem oder mehreren der Ansprüche 1 bis 9, **dadurch gekennzeichnet, dass** R¹ gleich H ist.

11. Verfahren zur Herstellung einer Verbindung nach einem oder mehreren der Ansprüche 1 bis 10, **dadurch gekennzeichnet, dass** zunächst ein Spirobifluoren, welches eine reaktive Gruppe trägt, in einer ersten metallkatalysierten Kupplungsreaktion mit einem Benzolderivat umgesetzt wird, welches zwei reaktive Gruppen enthält, von denen eine in dieser Kupplungsreaktion umgesetzt wird, und wobei schließlich eine Aminogruppe über eine zweite metallorganische Kupplungsreaktion an der anderen der zwei reaktiven Gruppen in die Verbindung eingeführt wird; oder **dadurch gekennzeichnet, dass** eine Benzolverbindung, welche zwei reaktive Gruppen trägt, in einer ersten metallkatalysierten Kupplungsreaktion an der einen der zwei reaktiven Gruppen mit einer Aminoverbindung umgesetzt wird, und in einer zweiten metallkatalysierten Kupplungsreaktion an der anderen der zwei reaktiven Gruppen mit einem Spirobifluoren-Derivat umgesetzt wird.

12. Oligomer, Polymer oder Dendrimer, enthaltend eine oder mehrere Verbindungen gemäß Formel (I) nach einem oder mehreren der Ansprüche 1 bis 10, wobei die Bindung(en) zum Polymer, Oligomer oder Dendrimer an beliebigen, in Formel (I) mit R¹ oder R² oder R³ substituierten Positionen lokalisiert sein können.

13. Formulierung, enthaltend mindestens eine Verbindung nach einem oder mehreren der Ansprüche 1 bis 10 oder ein Polymer, Oligomer oder Dendrimer nach Anspruch 12, sowie mindestens ein Lösungsmittel.

14. Elektronische Vorrichtung, enthaltend mindestens eine Verbindung nach einem oder mehreren der Ansprüche 1 bis 10, oder ein Polymer, Oligomer oder Dendrimer nach Anspruch 12.

15. Elektronische Vorrichtung nach Anspruch 14, **dadurch gekennzeichnet, dass** sie eine organische Elektrolumineszenzvorrichtung ist, enthaltend Anode, Kathode und mindestens eine emittierende Schicht, wobei mindestens eine organische Schicht der Vorrichtung, die eine emittierende Schicht oder eine lochtransportierende Schicht, insbesondere eine Lochtransportschicht, eine Lochinjektionsschicht oder eine elektronenblockierende Schicht sein kann, die mindestens eine Verbindung enthält.

16. Elektronische Vorrichtung nach Anspruch 14, **dadurch gekennzeichnet, dass** sie eine organische Elektrolumineszenzvorrichtung ist, enthaltend Anode, Kathode, mindestens eine emittierende Schicht, und genau zwei drei oder vier lochtransportierende Schichten zwischen Anode und emittierender Schicht, wobei mindestens eine der lochtransportierenden Schichten die mindestens eine Verbindung enthält.

17. Elektronische Vorrichtung nach Anspruch 14, **dadurch gekennzeichnet, dass** sie eine organische Elektrolumineszenzvorrichtung ist, enthaltend Anode, Kathode, mindestens eine emittierende Schicht und mindestens eine lochtransportierende Schicht, welche die mindestens eine Verbindung sowie mindestens eine weitere Verbindung gewählt aus p-Dotanden enthält.

18. Verwendung einer Verbindung nach einem oder mehreren der Ansprüche 1 bis 10 in einer elektronischen Vorrichtung.

## Claims

1. Compound of the formula (I) 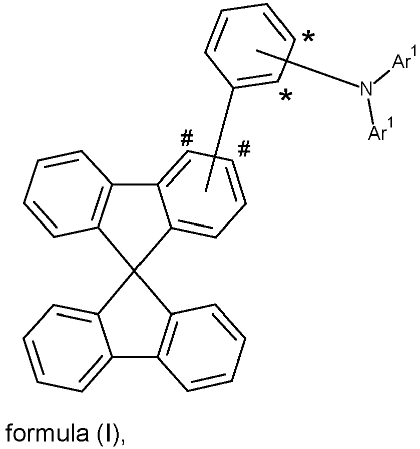 where the compound may be substituted in each case by a radical R¹ at each of the free positions on the spirobifluorene unit, and may be substituted in each case by a radical R² at each of the free positions on the phenylene unit, and
where the following applies to the variables occurring:
R¹ is selected on each occurrence, identically or differently, from H, D, F, C(=O)R⁴, CN, Si(R⁴)₃, N(R⁴)₂, P(=O)(R⁴)₂, OR⁴, S(=O)R⁴, S(=O)₂R⁴, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, where the term "aromatic ring system" does not include triarylamine, and heteroaromatic ring systems having 5 to 40 aromatic ring atoms; where the said alkyl, alkoxy, alkenyl and alkynyl groups and the said aromatic ring systems and heteroaromatic ring systems may in each case be substituted by one or more radicals R⁴; and where one or more CH₂ groups in the said alkyl, alkoxy, alkenyl and alkynyl groups may be replaced by -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO or SO₂;
R² is selected on each occurrence, identically or differently, from H, D, F, C(=O)R⁴, CN, Si(R⁴)₃, P(=O)(R⁴)2, OR⁴, S(=O)R⁴, S(=O)₂R⁴, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R² may be linked to one another and may form a ring; where the said alkyl, alkoxy, alkenyl and alkynyl groups and the said aromatic ring systems and heteroaromatic ring systems may be substituted in each case by one or more radicals R⁴; and where one or more CH₂ groups in the said alkyl, alkoxy, alkenyl and alkynyl groups may be replaced by -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO or SO₂;
Ar¹ is selected on each occurrence, identically or differently, from aromatic ring systems having 6 to 20 aromatic ring atoms, which are optionally substituted by one or more radicals R³, and from heteroaromatic ring systems having 5 to 30 aromatic ring atoms, which are optionally substituted by one or more radicals R³;
R³ is selected on each occurrence, identically or differently, from H, D, F, C(=O)R⁴, CN, Si(R⁴)₃, N(R⁴)₂, P(=O)(R⁴)2, OR⁴, S(=O)R⁴, S(=O)₂R⁴, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where the said alkyl, alkoxy, alkenyl and alkynyl groups and the said aromatic ring systems and heteroaromatic ring systems may be substituted in each case by one or more radicals R⁴; and where one or more CH₂ groups in the said alkyl, alkoxy, alkenyl and alkynyl groups may be replaced by -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO or SO₂;
R⁴ is selected on each occurrence, identically or differently, from H, D, F, C(=O)R⁵, CN, Si(R⁵)₃, P(=O)(R⁵)₂, OR⁵, S(=O)R⁵, S(=O)₂R⁵, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where the said alkyl, alkoxy, alkenyl and alkynyl groups and the said aromatic ring systems and heteroaromatic ring systems may be substituted in each case by one or more radicals R⁵; and where one or more CH₂ groups in the said alkyl, alkoxy, alkenyl and alkynyl groups may be replaced by -R⁵C=CR⁵-, -C=C-, Si(R⁵)₂, C=O, C=NR⁵, -C(=O)O-, -C(=O)NR⁵-, NR⁵, P(=O)(R⁵), -O-, -S-, SO or SO₂;
R⁵ is selected on each occurrence, identically or differently, from H, D, F, CN, alkyl or alkoxy groups having 1 to 20 C atoms, alkenyl or alkynyl groups having 2 to 20 C atoms, aromatic ring systems having 6 to 40 aromatic ring atoms and heteroaromatic ring systems having 5 to 40 aromatic ring atoms; and where the said alkyl, alkoxy, alkenyl and alkynyl groups, aromatic ring systems and heteroaromatic ring systems may be substituted by F or CN;
where the benzene ring is bonded to the spirobifluorene group at one of the positions marked with the symbol #; and
where the group N(Ar¹)₂ is bonded to the benzene ring at one of the positions marked with the symbol *.

2. Compound according to Claim 1, **characterised in that** no radical R¹, precisely one radical R¹ that is not equal to H, or precisely two radicals R¹ that are not equal to H are bonded to the spirobifluorene basic structure.

3. Compound according to Claim 1 or 2, **characterised in that** R² is selected on each occurrence, identically or differently, from H, F, methyl, ethyl, propyl, butyl, tert-butyl, phenyl, biphenyl, terphenyl, naphthyl, carbazolyl, benzofuranyl, benzothiophenyl, dibenzofuranyl, dibenzothiophenyl, 9,9'-dimethylfluorenyl and 9,9'-diphenylfluorenyl.

4. Compound according to one or more of Claims 1 to 3, **characterised in that** no radical R² or precisely one radical R² that is not equal to H is bonded to the phenylene group in formula (I).

5. Compound according to one or more of Claims 1 to 4, **characterised in that** Ar¹, if it is a heteroaromatic ring system, is selected on each occurrence, identically or differently, from dibenzofuranyl, dibenzothiophenyl, benzofuranyl, benzothiophenyl, benzo-fused dibenzofuranyl, benzo-fused dibenzothiophenyl, indolyl, quinolinyl, pyridyl, pyrimidyl, pyrazinyl, pyridazinyl, triazinyl, triazole, oxazole, oxadiazole, benzoxazole, benzothiazole, phenanthrolyl and azacarbazolyl, in each case optionally substituted by one or more radicals R³; and/or that Ar¹, if it is an aromatic ring system, is selected on each occurrence, identically or differently, from aromatic ring systems having 6 to 12 aromatic ring atoms, which are optionally substituted by one or more radicals R³.

6. Compound according to one or more of Claims 1 to 5, **characterised in that** Ar¹ is selected on each occurrence, identically or differently, from phenyl, biphenyl, terphenyl, quaterphenyl, naphthyl, fluorenyl, in particular 9,9'-dimethylfluorenyl and 9,9'-diphenylfluorenyl, monobenzofluorenyl, dibenzofluorenyl, indenofluorenyl, dibenzofuranyl, dibenzothiophenyl, benzofuranyl, benzothiophenyl, benzo-fused dibenzofuranyl, benzo-fused dibenzothiophenyl, indolyl, quinolinyl, pyridyl, pyrimidyl, pyrazinyl, pyridazinyl and triazinyl, in each case optionally substituted by one or more radicals R³.

7. Compound according to one or more of Claims 1 to 6, **characterised in that** R³ is equal to H.

8. Compound according to one or more of Claims 1 to 7, **characterised in that** the compound of the formula (I) is a compound of one of the formulae (I-1) to (I-4) 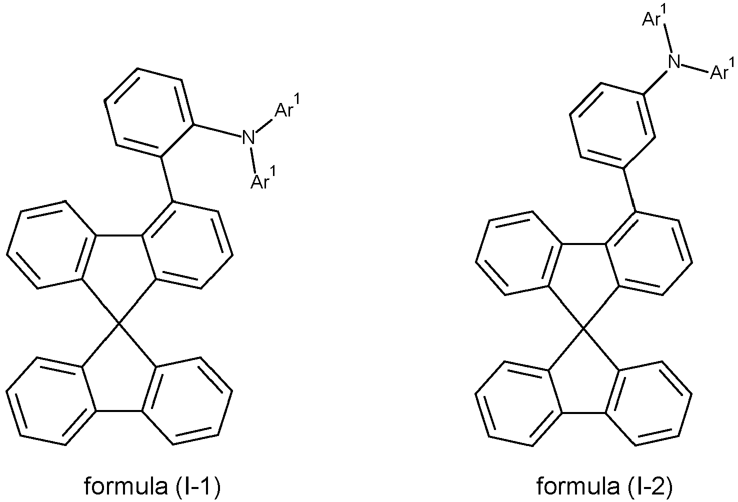 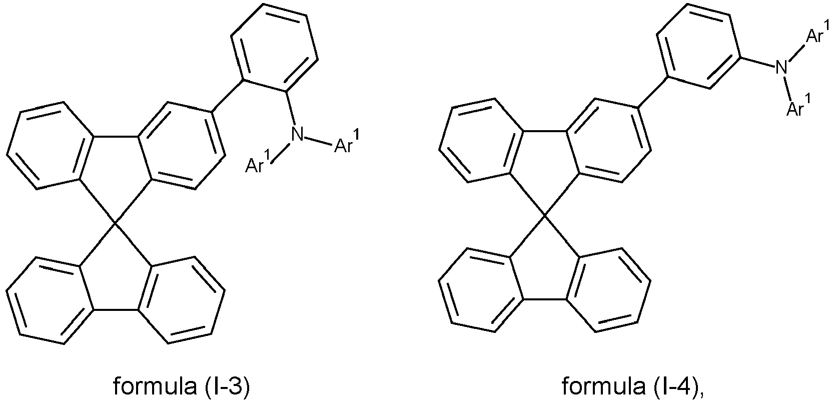 where the formulae may be substituted in each case by a radical R¹ at each of the free positions on the spirobifluorene unit, and may be substituted in each case by a radical R² at each of the free positions on the phenylene unit, and
where the groups occurring are defined as in Claim 1 or in one of Claims 2 to 7.

9. Compound according to one or more of Claims 1 to 8, **characterised in that** R¹ is selected on each occurrence, identically or differently, from H, F, methyl, ethyl, propyl, butyl, tert-butyl, phenyl, biphenyl, terphenyl, naphthyl, carbazolyl, benzofuranyl, benzothiophenyl, dibenzofuranyl, dibenzothiophenyl, 9,9'-dimethylfluorenyl and 9,9'-diphenylfluorenyl.

10. Compound according to one or more of Claims 1 to 9, **characterised in that** R¹ is equal to H.

11. Process for the preparation of a compound according to one or more of Claims 1 to 10, **characterised in that** firstly a spirobifluorene carrying a reactive group is reacted in a first metal-catalysed coupling reaction with a benzene derivative containing two reactive groups, one of which is reacted in this coupling reaction, and where finally an amino group is introduced into the compound via a second organometallic coupling reaction on the other of the two reactive groups; or **characterised in that** a benzene compound carrying two reactive groups is reacted with an amino compound in a first metal-catalysed coupling reaction on one of the two reactive groups, and is reacted with a spirobifluorene derivative in a second metal-catalysed coupling reaction on the other of the two reactive groups.

12. Oligomer, polymer or dendrimer containing one or more compounds of the formula (I) according to one or more of Claims 1 to 10, where the bond(s) to the polymer, oligomer or dendrimer may be localised at any desired positions in formula (I) that are substituted by R¹ or R² or R³.

13. Formulation comprising at least one compound according to one or more of Claims 1 to 10 or a polymer, oligomer or dendrimer according to Claim 12, and at least one solvent.

14. Electronic device containing at least one compound according to one or more of Claims 1 to 10, or a polymer, oligomer or dendrimer according to Claim 12.

15. Electronic device according to Claim 14, **characterised in that** it is an organic electroluminescent device comprising anode, cathode and at least one emitting layer, where at least one organic layer of the device, which can be an emitting layer or a hole-transporting layer, in particular a hole-transport layer, a hole-injection layer or an electron-blocking layer which comprises at least one compound.

16. Electronic device according to Claim 14, **characterised in that** it is an organic electroluminescent device comprising anode, cathode, at least one emitting layer, and precisely two, three or four hole-transporting layers between anode and emitting layer, where at least one of the hole-transporting layers comprises the at least one compound.

17. Electronic device according to Claim 14, **characterised in that** it is an organic electroluminescent device comprising anode, cathode, at least one emitting layer and at least one hole-transporting layer which comprises the at least one compound and at least one further compound selected from p-dopants.

18. Use of a compound according to one or more of Claims 1 to 10 in an electronic device.

## Revendications

1. Composé de la formule (I) 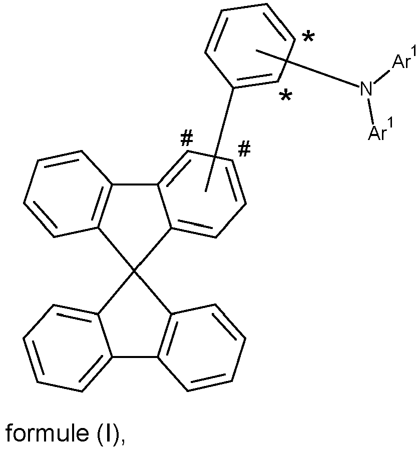 où le composé peut être substitué dans chaque cas par un radical R¹ au niveau de chacune des positions libres sur l'unité spirobifluorène, et peut être substitué dans chaque cas par un radical R² au niveau de chacune des positions libres sur l'unité phénylène, et
où ce qui suit s'applique aux variables présentes :
R¹ est sélectionné pour chaque occurrence, de manière identique ou différente, parmi H, D, F, C(=O)R⁴, CN, Si(R⁴)₃, N(R⁴)₂, P(=O)(R⁴)2, OR⁴, S(=O)R⁴, S(=O)₂R⁴, les groupes alkyle ou alcoxy en chaîne droite qui comportent de 1 à 20 atome(s) de C, les groupes alkyle ou alcoxy ramifiés ou cycliques qui comportent de 3 à 20 atomes de C, les groupes alkényle ou alkynyle qui comportent de 2 à 20 atomes de C, les systèmes de cycle aromatique qui comportent de 6 à 40 atomes de cycle aromatique, où l'expression "système de cycle aromatique" n'inclut pas la triarylamine, et les systèmes de cycle hétéroaromatique qui comportent de 5 à 40 atomes de cycle aromatique ; où lesdits groupes alkyle, alcoxy, alkényle et alkynyle et lesdits systèmes de cycle aromatique et lesdits systèmes de cycle hétéroaromatique peuvent dans chaque cas être substitués par un radical ou par plusieurs radicaux R⁴ ; et où un ou plusieurs groupe(s) CH₂ dans lesdits groupes alkyle, alcoxy, alkényle et alkynyle peut/peuvent être remplacé(s) par -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO ou SO₂;
R² est sélectionné pour chaque occurrence, de manière identique ou différente, parmi H, D, F, C(=O)R⁴, CN, Si(R⁴)₃, P(=O)(R⁴)₂, OR⁴, S(=O)R⁴, S(=O)₂R⁴, les groupes alkyle ou alcoxy en chaîne droite qui comportent de 1 à 20 atome(s) de C, les groupes alkyle ou alcoxy ramifiés ou cycliques qui comportent de 3 à 20 atomes de C, les groupes alkényle ou alkynyle qui comportent de 2 à 20 atomes de C, les systèmes de cycle aromatique qui comportent de 6 à 40 atomes de cycle aromatique, et les systèmes de cycle hétéroaromatique qui comportent de 5 à 40 atomes de cycle aromatique ; où deux radicaux R² ou plus peuvent être liés l'un à l'autre ou les uns aux autres et peuvent former un cycle ; où lesdits groupes alkyle, alcoxy, alkényle et alkynyle et lesdits systèmes de cycle aromatique et lesdits systèmes de cycle hétéroaromatique peuvent être substitués dans chaque cas par un radical ou par plusieurs radicaux R⁴ ; et où un ou plusieurs groupe(s) CH₂ dans lesdits groupes alkyle, alcoxy, alkényle et alkynyle peut/peuvent être remplacé(s) par -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO ou SO₂;
Ar¹ est sélectionné pour chaque occurrence, de manière identique ou différente, parmi les systèmes de cycle aromatique qui comportent de 6 à 20 atomes de cycle aromatique, lesquels sont en option substitués par un radical ou par plusieurs radicaux R³, et parmi les systèmes de cycle hétéroaromatique qui comportent de 5 à 30 atomes de cycle aromatique, lesquels sont en option substitués par un radical ou par plusieurs radicaux R³ ;
R³ est sélectionné pour chaque occurrence, de manière identique ou différente, parmi H, D, F, C(=O)R⁴, CN, Si(R⁴)₃, N(R⁴)₂, P(=O)(R⁴)2, OR⁴, S(=O)R⁴, S(=O)₂R⁴, les groupes alkyle ou alcoxy en chaîne droite qui comportent de 1 à 20 atome(s) de C, les groupes alkyle ou alcoxy ramifiés ou cycliques qui comportent de 3 à 20 atomes de C, les groupes alkényle ou alkynyle qui comportent de 2 à 20 atomes de C, les systèmes de cycle aromatique qui comportent de 6 à 40 atomes de cycle aromatique, et les systèmes de cycle hétéroaromatique qui comportent de 5 à 40 atomes de cycle aromatique ; où lesdits groupes alkyle, alcoxy, alkényle et alkynyle et lesdits systèmes de cycle aromatique et lesdits systèmes de cycle hétéroaromatique peuvent être substitués dans chaque cas par un radical ou par plusieurs radicaux R⁴ ; et où un ou plusieurs groupe(s) CH₂ dans lesdits groupes alkyle, alcoxy, alkényle et alkynyle peut/peuvent être remplacé(s) par -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO ou SO₂;
R⁴ est sélectionné pour chaque occurrence, de manière identique ou différente, parmi H, D, F, C(=O)R⁵, CN, Si(R⁵)₃, P(=O)(R⁵)₂, OR⁵, S(=O)R⁵, S(=O)₂R⁵, les groupes alkyle ou alcoxy en chaîne droite qui comportent de 1 à 20 atome(s) de C, les groupes alkyle ou alcoxy ramifiés ou cycliques qui comportent de 3 à 20 atomes de C, les groupes alkényle ou alkynyle qui comportent de 2 à 20 atomes de C, les systèmes de cycle aromatique qui comportent de 6 à 40 atomes de cycle aromatique, et les systèmes de cycle hétéroaromatique qui comportent de 5 à 40 atomes de cycle aromatique ; où lesdits groupes alkyle, alcoxy, alkényle et alkynyle et lesdits systèmes de cycle aromatique et lesdits systèmes de cycle hétéroaromatique peuvent être substitués dans chaque cas par un radical ou par plusieurs radicaux R⁵ ; et où un ou plusieurs groupe(s) CH₂ dans lesdits groupes alkyle, alcoxy, alkényle et alkynyle peut/peuvent être remplacé(s) par -R⁵C=CR⁵-, -C=C-, Si(R⁵)₂, C=O, C=NR⁵, -C(=O)O-, -C(=O)NR⁵-, NR⁵, P(=O)(R⁵), -O-, -S-, SO ou SO₂;
R⁵ est sélectionné pour chaque occurrence, de manière identique ou différente, parmi H, D, F, CN, les groupes alkyle ou alcoxy qui comportent de 1 à 20 atome(s) de C, les groupes alkényle ou alkynyle qui comportent de 2 à 20 atomes de C, les systèmes de cycle aromatique qui comportent de 6 à 40 atomes de cycle aromatique et les systèmes de cycle hétéroaromatique qui comportent de 5 à 40 atomes de cycle aromatique ; et où lesdits groupes alkyle, alcoxy, alkényle et alkynyle, lesdits systèmes de cycle aromatique et lesdits systèmes de cycle hétéroaromatique peuvent être substitués par F ou CN ;
où le cycle benzène est lié au groupe spirobifluorène au niveau de l'une des positions qui sont indiquées au moyen du symbole # ; et
où le groupe N(Ar¹)₂ est lié au cycle benzène au niveau de l'une des positions qui sont indiquées au moyen du symbole *.

2. Composé selon la revendication 1, **caractérisé en ce qu'**aucun radical R¹, n'est lié à la structure de base spirobifluorène, ou de façon précise un seul radical R¹ qui n'est pas égal à H, ou de façon précise deux radicaux R¹ qui ne sont pas égaux à H est/sont lié(s) à la structure de base spirobifluorène.

3. Composé selon la revendication 1 ou 2, **caractérisé en ce que** R² est sélectionné pour chaque occurrence, de manière identique ou différente, parmi H, F, méthyle, éthyle, propyle, butyle, tert-butyle, phényle, biphényle, terphényle, naphtyle, carbazolyle, benzofuranyle, benzothiophényle, dibenzofuranyle, dibenzothiophényle, 9,9'-diméthylfluorényle et 9,9'-diphénylfluorényle.

4. Composé selon une ou plusieurs des revendications 1 à 3, **caractérisé en ce qu'**aucun radical R² n'est lié au groupe phénylène dans la formule (I) ou de façon précise un seul radical R² qui n'est pas égal à H est lié au groupe phénylène dans la formule (I).

5. Composé selon une ou plusieurs des revendications 1 à 4, **caractérisé en ce que** Ar¹, s'il s'agit d'un système de cycle hétéroaromatique, est sélectionné pour chaque occurrence, de manière identique ou différente, parmi dibenzofuranyle, dibenzothiophényle, benzofuranyle, benzothiophényle, dibenzofuranyle benzo-fusionné, dibenzothiophényle benzo-fusionné, indolyle, quinolinyle, pyridyle, pyrimidyle, pyrazinyle, pyridazinyle, triazinyle, triazole, oxazole, oxadiazole, benzoxazole, benzothiazole, phénanthrolyle et azacarbazolyle, et est dans chaque cas en option substitué par un radical ou par plusieurs radicaux R³; et/ou **en ce que** Ar¹, s'il s'agit d'un système de cycle aromatique, est sélectionné pour chaque occurrence, de manière identique ou différente, parmi les systèmes de cycle aromatique qui comportent de 6 à 12 atomes de cycle aromatique, lesquels sont en option substitués par un radical ou par plusieurs radicaux R³.

6. Composé selon une ou plusieurs des revendications 1 à 5, **caractérisé en ce que** Ar¹ est sélectionné pour chaque occurrence, de manière identique ou différente, parmi phényle, biphényle, terphényle, quaterphényle, naphtyle, fluorényle, en particulier 9,9'-diméthylfluorényle et 9,9'-diphénylfluorényle, monobenzofluorényle, dibenzofluorényle, indénofluorényle, dibenzofuranyle, dibenzothiophényle, benzofuranyle, benzothiophényle, dibenzofuranyle benzo-fusionné, dibenzothiophényle benzo-fusionné, indolyle, quinolinyle, pyridyle, pyrimidyle, pyrazinyle, pyridazinyle et triazinyle, dans chaque cas en option il est substitué par un radical ou par plusieurs radicaux R³.

7. Composé selon une ou plusieurs des revendications 1 à 6, **caractérisé en ce que** R³ est égal à H.

8. Composé selon une ou plusieurs des revendications 1 à 7, **caractérisé en ce que** le composé de la formule (I) est un composé de l'une des formules (I-1) à (I-4) 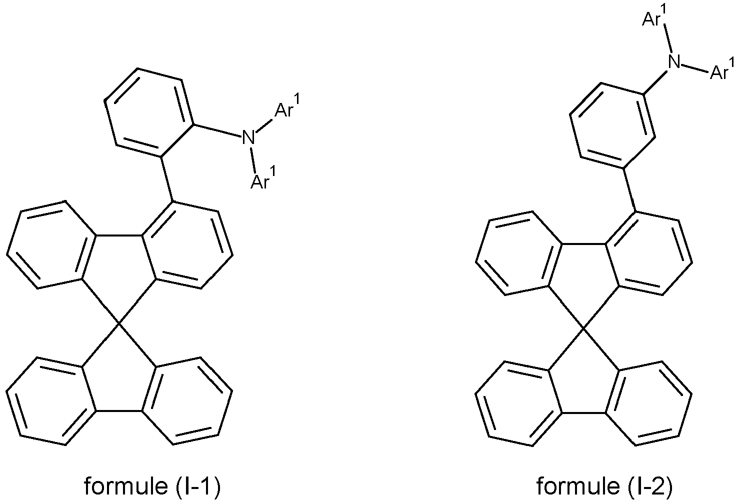 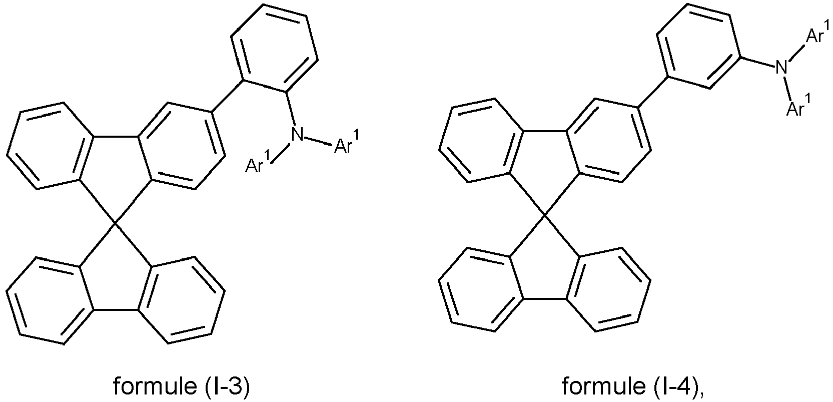 où les formules peuvent être substituées dans chaque cas par un radical R¹ au niveau de chacune des positions libres sur l'unité spirobifluorène, et peuvent être substituées dans chaque cas par un radical R² au niveau de chacune des positions libres sur l'unité phénylène, et
où les groupes qui sont présents sont définis tel que selon la revendication 1 ou que selon l'une des revendications 2 à 7.

9. Composé selon une ou plusieurs des revendications 1 à 8, **caractérisé en ce que** R¹ est sélectionné pour chaque occurrence, de manière identique ou différente, parmi H, F, méthyle, éthyle, propyle, butyle, tert-butyle, phényle, biphényle, terphényle, naphtyle, carbazolyle, benzofuranyle, benzothiophényle, dibenzofuranyle, dibenzothiophényle, 9,9'-diméthylfluorényle et 9,9'-diphénylfluorényle.

10. Composé selon une ou plusieurs des revendications 1 à 9, **caractérisé en ce que** R¹ est égal à H.

11. Procédé pour la préparation d'un composé selon une ou plusieurs des revendications 1 à 10, **caractérisé en ce qu'**en premier lieu, un spirobifluorène qui est porteur d'un groupe réactif est amené à réagir selon une première réaction de couplage catalysée par métal avec un dérivé de benzène qui contient deux groupes réactifs, dont l'un est amené à réagir au niveau de cette réaction de couplage, et où, pour finir, un groupe amino est introduit à l'intérieur du composé via une seconde réaction de couplage organométallique sur l'autre des deux groupes réactifs ; ou **caractérisé en ce qu'**un composé benzène qui est porteur de deux groupes réactifs est amené à réagir avec un composé amino selon une première réaction de couplage catalysée par métal sur l'un des deux groupes réactifs, et est amené à réagir avec un dérivé de spirobifluorène selon une seconde réaction de couplage catalysée par métal sur l'autre des deux groupes réactifs.

12. Oligomère, polymère ou dendrimère contenant un ou plusieurs composé(s) de la formule (I) selon une ou plusieurs des revendications 1 à 10, où la/les liaison(s) sur le polymère, l'oligomère ou le dendrimère peut/ peuvent être localisée(s) au niveau de n'importe quelles positions souhaitées dans la formule (I) qui sont substituées par R¹ ou R² ou R³.

13. Formulation comprenant au moins un composé selon une ou plusieurs des revendications 1 à 10 ou polymère, oligomère ou dendrimère selon la revendication 12, et au moins un solvant.

14. Dispositif électronique contenant au moins un composé selon une ou plusieurs des revendications 1 à 10, ou polymère, oligomère ou dendrimère selon la revendication 12.

15. Dispositif électronique selon la revendivation14, **caractérisé en ce qu'**il s'agit d'un dispositif électroluminescent organique qui comprend une anode, une cathode et au moins une couche d'émission, où au moins une couche organique du dispositif, laquelle peut être une couche d'émission ou une couche de transport de trous, en particulier une couche de transport de trous, une couche d'injection de trous ou une couche de blocage d'électrons comprend au moins un composé.

16. Dispositif électronique selon la revendivation14, **caractérisé en ce qu'**il s'agit d'un dispositif électroluminescent organique qui comprend une anode, une cathode, au moins une couche d'émission, et de façon précise deux, trois ou quatre couches de transport de trous entre l'anode et la couche d'émission, où au moins l'une des couches de transport de trous comprend l'au moins un composé.

17. Dispositif électronique selon la revendivation14, **caractérisé en ce qu'**il s'agit d'un dispositif électroluminescent organique qui comprend une anode, une cathode, au moins une couche d'émission et au moins une couche de transport de trous qui comprend l'au moins un composé et au moins un autre composé qui est sélectionné les dopants p.

18. Utilisation d'un composé selon une ou plusieurs des revendications 1 à 10 dans un dispositif électronique.
